(19) **Europäisches Patentamt**
**European Patent Office**
**Office européen des brevets**

(11) **EP 4 501 965 A1**

(12) **EUROPEAN PATENT APPLICATION**
published in accordance with Art. 153(4) EPC

(43) Date of publication:
05.02.2025 Bulletin 2025/06

(21) Application number: 23778322.0

(22) Date of filing: 29.03.2023

(51) International Patent Classification (IPC):
$C07K\ 16/28^{(2006.01)}$  $C07K\ 19/00^{(2006.01)}$
$C07K\ 14/705^{(2006.01)}$  $G01N\ 33/68^{(2006.01)}$
$G01N\ 33/564^{(2006.01)}$  $A61K\ 39/395^{(2006.01)}$

(52) Cooperative Patent Classification (CPC):
A61K 38/17; A61K 39/00; A61K 39/395;
A61K 47/68; A61P 13/12; B01J 20/24; C07K 1/22;
C07K 14/705; C07K 16/00; C07K 16/28;
C07K 19/00; C12N 5/10; C12N 15/62;
C12N 15/867; G01N 33/564;                    (Cont.)

(86) International application number:
PCT/CN2023/084857

(87) International publication number:
WO 2023/185957 (05.10.2023 Gazette 2023/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.03.2022 CN 202210323725

(71) Applicant: Chengdu Di'ao Pharmaceutical Group
Co., Ltd.
Chengdu, Sichuan 610041 (CN)

(72) Inventors:
• LAI, Bing
Chengdu, Sichuan 610041 (CN)

• QIU, Yong
Chengdu, Sichuan 610041 (CN)
• ZHANG, Zhiming
Chengdu, Sichuan 610041 (CN)
• GONG, Shilin
Chengdu, Sichuan 610041 (CN)
• HE, Ling
Chengdu, Sichuan 610041 (CN)

(74) Representative: Dehns Germany Partnerschaft
mbB
Theresienstraße 6-8
80333 München (DE)

Remarks:
The complete document including Reference
Table(s) and the Sequence Listing(s) can be
downloaded from the EPO website

(54) **ANTIBODY, FUSION PROTEIN AND USE THEREOF**

(57)    The present application relates to an antibody, a fusion protein, and the uses thereof. The antibody includes a CDR sequence selected from at least one of: light chain variable region CDR sequences: SEQ ID NO: 1 to SEQ ID NO: 3, SEQ ID NO: 7 to SEQ ID NO: 9, and SEQ ID NO: 83 to SEQ ID NO: 85; and heavy chain variable region CDR sequences: SEQ ID NO: 4 to SEQ ID NO: 6, SEQ ID NO: 10 to SEQ ID NO: 12, SEQ ID NO: 86 to SEQ ID NO: 88, and SEQ ID NO: 89 to SEQ ID NO: 91. The fusion protein includes a neutral antibody. The neutral antibody includes an antibody FC segment or the above mentioned antibody, and one or more PLA2R antigen epitope peptides. The PLA2R antigen epitope peptide is linked to the neutral antibody. The antibody of the present application has a low antigen-binding activity and a high expression level.

**EP 4 501 965 A1**

(52) Cooperative Patent Classification (CPC): (Cont.)
**G01N 33/68;** Y02A 50/30

**Description**

**FIELD**

The present disclosure relates to the fields of molecular biology and medicine. Specifically, the present disclosure relates to an antibody, a fusion protein and the use thereof, and more specifically, the present disclosure relates to an antibody, a nucleic acid molecule thereof, an expression vector, and a recombinant cell, as well as a fusion protein, a nucleic acid molecule thereof, an expression vector, a recombinant cell, a pharmaceutical composition and use thereof.

**BACKGROUND**

Membranous nephropathy (MN), i.e., membranous glomerulonephritis, specifically refers to nephrotic syndrome caused by excessive deposition of immune complexes in glomerular capillaries. MN is one of the most common pathological types of nephrotic syndrome in adults, with a global incidence of about 12/1 million (Mc Grogan A, Franssen CF, de Vries CS. Nephrol Dial Transplant. 2011; 26(2): 414-430). In recent years, the incidence of MN has annually increased at a rate of 13% (Lwezaula BF, Ameh OI, Ekrikpo UE, et al. BMC Nephrol. 2021; 22(1): 15), and MN has become the second leading cause of chronic glomerulonephritis. Clinically, MN is mainly characterized by massive proteinuria, hypoproteinemia, edema and hyperlipidemia. The diagnosis depends on pathological examination of renal biopsy tissue, and its pathological diagnosis features include thickened glomerular capillary wall, deposition of IgG and complement C3 along the capillary wall observed with immunofluorescence microscopy, and formation of electronic dense deposits under the glomerular epithelial cells under electron microscope.

According to different causes, MN can be divided into idiopathic membranous nephropathy (IMN) and secondary membranous nephropathy (SMN). IMN, also known as primary membranous nephropathy (PMN), is the dominant type of MN, accounting for about 70% to 80% of MN cases, and the causes thereof are still unclear. At present, the pathogenesis of PMN is not very clear. However, most researcher believe that PMN is an antibody-mediated autoimmune glomerular disease. The target antigens located in podocytes are recognized by and bind to autoantibodies to form immune complexes, which are then deposited on the podocytes in the basement membrane. Thus, the complement system is activated to cause damage and shedding of the podocytes, resulting in an increase in a permeability of the basement membrane and the appearance of mass proteinuria.

Beck *et al.* first reported in 2009 that phospholipase A2 receptor (PLA2R) is one of the main antigens causing PMN, and phospholipase A2 receptor antibody (PLA2R-Ab) can be detected in blood and renal tissue eluates of 70% of PMN patients (Beck LH Jr, Bonegio RG, Lambeau G, et al. N Engl J Med. 2009; 361(1): 11-21), which attracted attention to PLA2R from the medical field. PLA2R-Ab can specifically bind to the target antigens (i.e., PLA2R) on podocytes in renal tissue, and can be detected in the serum of 70% to 80% of PMN patients (Bobart SA, De Vriese AS, Pawar AS, et al. Kidney Int. 2019; 95(2): 429-438). However, among healthy people or patients suffering from SMN or other glomerular diseases, the detection rate of PLA2R-Ab antibody in the serum is extremely low, and thus, the PLA2R-Ab can be highly specific for diagnosing MN. The latest Meta-analysis (Lian Mingjian, Lu Rong, Zhang Jiaqin, et al., Journal of Clinical Laboratory, 2017, 35 (7): 545-549) revealed that the total sensitivity and specificity of serum PLA2R-Ab for the diagnosis of PMN were 69% and 97%, respectively, further indicating that serum PLA2R-Ab can serve as a specific biomarker for the diagnosis of PMN with high sensitivity and high specificity for PMN.

In addition, a large number of studies have indicated that the serum PLA2R-Ab titer is negatively correlated with the glomerular filtration rate, serum total protein and albumin and other indicators in MN patients, and is also significantly negatively correlated with the spontaneous remission rate of PMN patients. PMN can have natural remission for mild patients without PLA2R-Ab or with low level of PLA2R-Ab. During the treatment with immunosuppressants, those who are negative in the serum PLA2R-Ab can respond to the immunosuppressive treatment in a shorter time, while those who are positive in the serum PLA2R-Ab have lower sensitivity to immunosuppressants. After receiving immunosuppressive treatment, the serum level of PLA2R-Ab continuously decreases, suggesting that PLA2R-Ab plays an important role in the evaluation of efficacy. After entering the remission stage, if the serum PLA2R-Ab does not turn to be negative or the titer thereof increases, it indicates a possible recurrence of the disease (Xu X, Nie S, Ding H, et al., Nat Rev Nephrol. 2018; 14(5): 313-324). For MN patients who have received kidney transplantation, disease recurrence may likely occur in those who produce circulating PLA2R-Ab (Passerini P, Malvica S, Tripodi F, et al. Front Immunol. 2019; 10: 1326), suggesting that postoperative recurrence may also be related to PLA2R-Ab. In addition, the research also indicated that the negative conversion rate of PLA2R-Ab is significantly positively correlated with the total remission rate, and the sensitivity, specificity and accuracy of PLA2R-Ab in the prediction of clinical remission events among MN patients within 12 months are 69.51%, 76.35%, and 80.26%, respectively (Zheng Na, Wang Meihua, An Zhi. Journal of Clinical Military Medicine. 2021; 49(08): 942-944).

To sum up, PLA2R-Ab has important reference value for clinical diagnosis, condition judgment and prognosis evaluation of PMN patients. PLA2R-Ab involves in the disease progression of PMN. The patients with low titer of PLA2R-Ab have relatively milder conditions and have more significant effect after immunosuppressant treatment, and some of them may even experience spontaneous remission. This suggests that monitoring the level of PLA2R-Ab is helpful to guide clinicians in the dosage and duration of immunosuppressants for patients, thereby avoiding excessive immunosuppressive treatment, and it is also helpful to reduce the medical burden of patients. The higher level of serum PLA2R-Ab represents the stronger immune response of the body, the higher severity of the disease, the poorer effect of immunosuppression treatment and the worse clinical prognosis, further indicating that monitoring serum PLA2R-Ab is helpful to guide clinical treatment. For the patients admitted to the hospital due to the high level of PLA2R-Ab, effective intervention treatment should be carried out at an early stage.

At present, the main clinic therapeutic treatment for MN includes the low-fat, low-salt and low-protein diet, and the symptomatic support treatment to control proteinuria and hyperlipidemia, supplemented by immunosuppressive therapy and some non-immunosuppressive treatments. However, the definitive treatment method is not available yet. In addition, for patients suffering from membranous nephropathy, the therapeutic effect is relatively poor, only 40% of them can achieve spontaneous remission, and 30% of the patients have poor therapeutic effect and eventually progress to end-stage renal disease. They may only maintain their lives through dialysis or transplantation, which seriously increases the financial burden and affects the life quality of the patients. For kidney transplant patients, about 40% of them have poor therapeutic effect, and some of them may lose their transplanted kidneys. Based on the pathogenesis of PMN and the important value of serum PLA2R-Ab level in the evaluation of the therapeutic effect and clinical prognosis of PMN, it is urgent to develop a drug or technology capable of effectively treating MN patients who are positive in PLA2R-Ab.

## SUMMARY

The present disclosure aims at solving one of the technical problems existing in the related art at least to some extent. To this end, the present disclosure provides an antibody and a fusion protein of the antibody binding to an autoimmune antigen of primary membranous nephropathy. The antibody of the present disclosure has low antigen-binding activity and the fusion protein of the present disclosure can specifically bind to the PLA2R antibody.

The present disclosure has been accomplished based on the following findings of the inventors.

For the PLA2R antibody-positive membranous nephropathy, a possible therapeutic method is to specifically neutralize PLA2R-Ab in serum. The inventors have surprisingly found that the fusion protein of antibody and PLA2R-Ab developed in the present disclosure (the fusion protein of the antigen and antibody is also referred to as antigenic antibody in the present disclosure) can achieve the technical effect of specifically neutralizing PLA2R-Ab in serum.

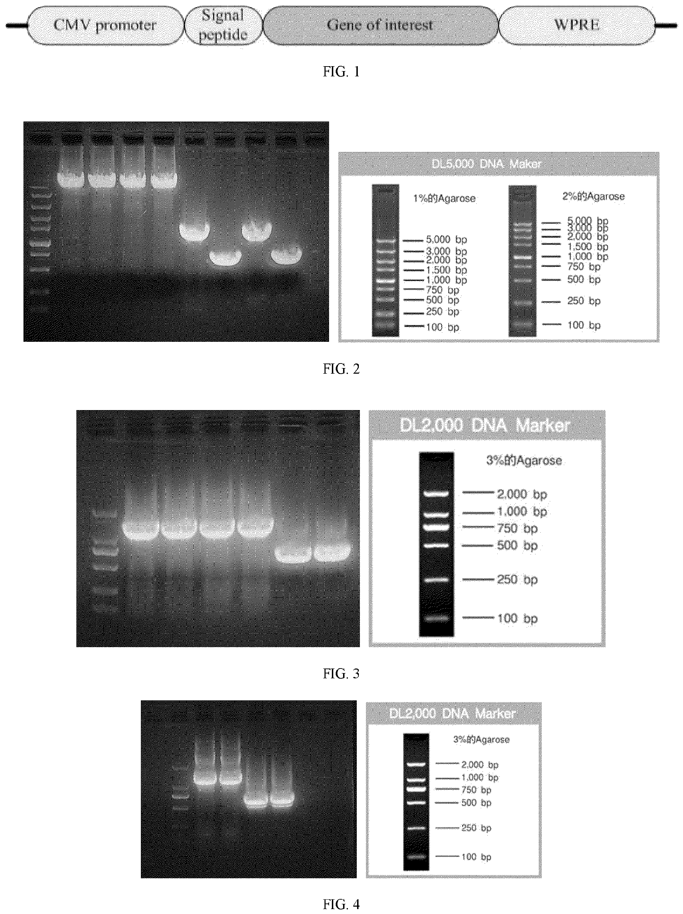

PLA2R is a membrane protein highly expressed on the surface of glomerular podocytes and contains the domain shown in FIG. 1. Meanwhile, a small amount of secreted PLA2R exists in the blood. In addition, according to the research of Liyo Kao *et al.* (Liyo Kao, Vinson Lam, et. al., J Am Soc Nephrol. 2015 Feb; 26(2):291-301), the antibodies in patients' plasma mainly recognize three domains of CysR, FNII, and CTLD1 in PLA2R.

Based on this, the inventors have found that the fusion protein obtained by fusing the antigen region of PLA2R with an antibody can maintain the binding ability of PLA2R to PLA2R-Ab. Moreover, during the research and development process, the inventors found that the antibody obtained by mutating the CDR region of the original antibody has extremely low antigen-binding activity, and thus the side effects caused by subsequent nonspecific binding can be avoided, and the antibody has higher expression level. Therefore, the fusion protein, which is obtained by fusing the mutated antibody with the antigen region of PLA2R, can specifically neutralize PLA2R-Ab without binding to other antibodies, thereby avoiding the side effects of nonspecific immunity. In addition, the fusion protein also has the advantage of long half-life, which can reduce the frequency of administration.

In a first aspect, the present disclosure provides an antibody. According to an embodiment of the present disclosure, the antibody includes a light chain variable region CDR and a heavy chain variable region CDR. A heavy chain variable region CDR1 sequence is as set forth in $GX_1X_2X_3X_4X_5X_6X_7X_8X_9$. A heavy chain variable region CDR2 sequence is as set forth in $X_{10}X_{11}X_{12}X_{13}X_{14}X_{15}GX_{16}X_{17}X_{18}X_{19}X_{20}X_{21}X_{22}X_{23}$. A heavy chain variable region CDR3 sequence is as set forth in $X_{24}GGX_{25}X_{26}X_{27}X_{28}X_{20}X_{30}X_{31}Y$. $X_1$ is G or F, $X_2$ is G or N, $X_3$ is G or I, $X_4$ is G, K or S, $X_5$ is G or D, $X_6$ is G or T, $X_7$ is G or Y, $X_8$ is G or I, $X_9$ is H or S, $X_{10}$ is R or G, $X_{11}$ is G or I, $X_{12}$ is G or Y, $X_{13}$ is G or P, $X_{14}$ is T or S, $X_{15}$ is G or N, $X_{16}$ is G or Y, $X_{17}$ is G or T, $X_{18}$ is G, S or R, $X_{19}$ is G or Y, $X_{20}$ is G or A, $X_{21}$ is G or D, $X_{22}$ is G or S, $X_{23}$ is S or V, $X_{24}$ is G or W, $X_{25}$ is G or D, $X_{26}$ is G or S, $X_{27}$ is G or F, $X_{28}$ is G or Y, $X_{29}$ is G or A, $X_{30}$ is G or M, and $X_{31}$ is S, G or D, with a provision that the followings do not occur at the same time: $X_1$ is F, $X_2$ is N, $X_3$ is I, $X_4$ is K, $X_5$ is D, $X_6$ is T, $X_7$ is Y, $X_8$ is I, $X_9$ is H, $X_{10}$ is R, $X_{11}$ is I, $X_{12}$ is Y, $X_{13}$ is P, $X_{14}$ is T, $X_{15}$ is N, $X_{16}$ is Y, $X_{17}$ is T, $X_{18}$ is R, $X_{19}$ is Y, $X_{20}$ is A, $X_{21}$ is D, $X_{22}$ is S, $X_{23}$ is V, $X_{24}$ is W, $X_{25}$ is D, $X_{26}$ is G, $X_{27}$ is F, $X_{28}$ is Y, $X_{29}$ is A, $X_{30}$ is M, and $X_{31}$ is D. A light chain variable region CDR1 sequence is as set forth in $X_{32}X_{33}X_{34}X_{35}X_{36}X_{37}X_{38}X_{39}X_{40}X_{41}A$. A light chain variable region CDR2 sequence is as set forth in $X_{42}X_{43}X_{44}X_{45}X_{46}X_{47}S$. A light chain variable region CDR3 sequence is as set forth in $X_{48}X_{49}X_{50}X_{51}X_{52}X_{53}X_{54}X_{55}X_{56}$. $X_{32}$ is G or R, $X_{33}$ is G or A, $X_{34}$ is G or S, $X_{35}$ is G or Q, $X_{36}$ is G, D or S, $X_{37}$ is G or V, $X_{38}$ is G or N, $X_{39}$ is G or T, $X_{40}$ is G or A, $X_{41}$ is S or V, $X_{42}$ is G or S, $X_{43}$ is G or A, $X_{44}$ is G or S, $X_{45}$ is G or F, $X_{46}$ is L or S, $X_{47}$ is G or Y, $X_{48}$ is G or Q, $X_{49}$ is G or Q, $X_{50}$ is G or H, $X_{51}$ is G or Y, $X_{52}$ is S or T, $X_{53}$ is G or T, $X_{54}$ is G or P, $X_{55}$ is G or P, and $X_{56}$ is G or T, with a provision that the followings do not occur at the same time: $X_{32}$ is R, $X_{33}$ is A, $X_{34}$ is S, $X_{35}$ is Q, $X_{36}$ is D, $X_{37}$ is V, $X_{38}$ is N, $X_{39}$ is T, $X_{40}$ is A, $X_{41}$ is V, $X_{42}$ is S, $X_{43}$ is A, $X_{44}$ is S, $X_{45}$ is F, $X_{46}$ is L, $X_{47}$ is Y, $X_{48}$ is Q, $X_{49}$ is Q, $X_{50}$ is H, $X_{51}$ is Y, $X_{52}$ is T, $X_{53}$ is T, $X_{54}$ is P, $X_{55}$ is P, and $X_{56}$ is T. Through a large number of experiments, the inventor found that: the above-mentioned antibody has low antigen-binding activity, and after forming an antigenic antibody, the nonspecific binding of the antigenic antibody can be effectively avoided; meanwhile, more importantly, the antibody has a high expression amount, which is suitable for the development of antigenic antibody drugs.

In a second aspect of the present disclosure, the present disclosure provides a first nucleic acid molecule. According to an embodiment of the present disclosure, the first nucleic acid molecule encodes the antibody described in the first aspect. The antibody encoded by the first nucleic acid molecule according to the embodiment of the present disclosure has low antigen-binding activity, and after forming an antigenic antibody, the nonspecific binding of the antigenic antibody can be effectively avoided. Meanwhile, the expression amount of the antibody is high, and the antibody is suitable for the development of antigenic antibody drugs.

In a third aspect of the present disclosure, the present disclosure provides a first expression vector. According to an embodiment of the present disclosure, the first expression vector carries the first nucleic acid molecule described in the second aspect. After the first expression vector according to the embodiment of the present disclosure is introduced into a suitable recipient cell, the antibody with low antigen-binding activity described in the first aspect can be effectively expressed under the mediation of a regulatory system, thereby obtaining the antibody in large quantities.

In a fourth aspect of the present disclosure, the present disclosure provides a first recombinant cell. According to an embodiment of the present disclosure, the first recombinant cell carries the first nucleic acid molecule described in the second aspect, or the first recombinant cell expresses the antibody described in the first aspect. The first recombinant cell according to the embodiment of the present disclosure can be used for *in vitro* expression and mass production of the antibody having low antigen-binding activity described in the first aspect.

In a fifth aspect of the present disclosure, the present disclosure provides use of the antibody described in the first aspect in the preparation of a fusion protein drug. As mentioned above, the above-mentioned antibody of the present disclosure has no antigen-binding activity, and thus it can be used as a neutral drug carrier to prepare a medicament for treating or preventing diseases. For example, the above-mentioned antibody can be fused with an antigen epitope peptide to prepare a fusion protein. Since the above-mentioned antibody has no antigen-binding activity, the prepared fusion protein can specifically bind to the antibody corresponding to the antigen epitope peptide without binding to other antibodies, thereby avoiding the side effects of non-specific binding of the fusion protein. The prepared fusion protein can be used to treat or prevent a PLA2R antibody-related disease corresponding to the antigen epitope peptide.

In a sixth aspect of the present disclosure, the present disclosure provides use of the above-mentioned antibody as a drug carrier or a negative antibody. As mentioned above, the above-mentioned antibody of the present disclosure has no antigen-binding activity, and thus it can be used as a neutral drug carrier to prepare drugs, or it can be directly used as a negative antibody reference without antigen-binding activity.

In a seventh aspect of the present disclosure, the present disclosure provides a fusion protein. According to an embodiment of the present disclosure, the fusion protein includes: a neutral antibody including an antibody FC segment or the antibody described in the first aspect; and one or more PLA2R antigen epitope peptides. The PLA2R antigen epitope peptide is linked to the neutral antibody. The inventors have found through experiments that the fusion protein obtained by fusing the PLA2R antigen epitope peptide with the above-mentioned neutral antibody can specifically bind to the PLA2R antibody without binding to other antibodies, thereby avoiding the side effects of non-specific binding. The fusion protein has the advantages of good expression and high yield.

In an eighth aspect of the present disclosure, the present disclosure provides a second nucleic acid molecule. According to an embodiment of the present disclosure, the nucleic acid molecule encodes the fusion protein described in the seventh aspect. The second nucleic acid molecule can be used to effectively express the above-mentioned fusion protein.

In a ninth aspect of the present disclosure, the present disclosure provides a second expression vector. According to an embodiment of the present disclosure, the second expression vector carries the second nucleic acid molecule described in the eighth aspect. The second expression vector can be used to effectively express the above-mentioned fusion protein.

In the tenth aspect of the present disclosure, the present disclosure provides a second recombinant cell. According to an embodiment of the present disclosure, the second recombinant cell carries the second nucleic acid molecule described in the eighth aspect; or the second recombinant cell expresses the fusion protein described in the seventh aspect. The recombinant cell according to the embodiment of the present disclosure can be used for *in vitro* expression and mass production of the fusion protein described in the fifth aspect.

In an eleventh aspect of the present disclosure, the present disclosure provides a pharmaceutical composition. According to an embodiment of the present disclosure, the pharmaceutical composition includes the fusion protein described in the seventh aspect. The inventors have found through a large number of experiments that the pharmaceutical composition can effectively prevent and treat a PLA2R antibody-related disease, for example, PLA2R antibody-positive membranous nephropathy.

In a twelfth aspect of the present disclosure, the present disclosure provides an immunosorbent. According to an embodiment of the present disclosure, the immunosorbent includes the fusion protein described in the seventh aspect. The inventors have found through a large number of experiments that the immunosorbent can adsorb PLA2R antibody. The immunosorbent can effectively treat a PLA2R antibody-related disease through immunoadsorption, for example, PLA2R antibody-positive membranous nephropathy.

In the thirteenth aspect of the present disclosure, the present disclosure provides a kit. According to an embodiment of the present disclosure, the kit includes the fusion protein described in the seventh aspect. The inventors have found through a large number of experiments that the kit can effectively detect the content of PLA2R antibody, and that the kit can also be used to diagnose or treat a PLA2R antibody-related disease, for example, PLA2R antibody-positive membranous nephropathy.

In the fourteenth aspect of the present disclosure, the present disclosure provides an adsorption product. According to the embodiment of the present disclosure, the adsorption product includes the above-described immunosorbent and a solid phase carrier. The immunosorbent is linked to the solid phase carrier. The adsorption product of the present disclosure can be used to adsorb the PLA2R antibody, for example, the PLA2R antibody in blood or plasma. By adsorbing the PLA2R antibody in blood or plasma, the adsorption product can be used to prevent or treat a PLA2R antibody-related disease.

In the fifteenth aspect of the present disclosure, the present disclosure provides use of the immunosorbent described in the twelfth aspect in the preparation of an adsorption product for adsorbing the PLA2R antibody.

In the sixteenth aspect of the present disclosure, the present disclosure provides use of the fusion protein as described in the seventh aspect, the second nucleic acid molecule as described in the eighth aspect, the second expression vector as described in the ninth aspect, the second recombinant cell as described in the tenth aspect, the pharmaceutical composition as described in the eleventh aspect, or the immunosorbent as described in the twelfth aspect in the preparation of a medicament for preventing or treating a PLA2R antibody-related disease.

In the seventeenth aspect of the present disclosure, the present disclosure provides use of the fusion protein described in the seventh aspect, the second nucleic acid molecule described in the eighth aspect, the second expression vector described in the ninth aspect, the second recombinant cell described in the tenth aspect, or the pharmaceutical composition described in the eleventh aspect in the preparation of an immunosorbent for adsorbing the PLA2R antibody.

In the eighteenth aspect of the present disclosure, the present disclosure provides use of the fusion protein described in the seventh aspect, the second nucleic acid molecule described in the eighth aspect, the second expression vector as described in the ninth aspect, the second recombinant cell described in the tenth aspect, the pharmaceutical composition described in the eleventh aspect or the immunosorbent described in the twelfth aspect in the preparation of a kit for detecting the PLA2R antibody.

In the nineteenth aspect of the present disclosure, the present disclosure provides a method for preventing or treating a PLA2R antibody-related disease. According to an embodiment of the present disclosure, the method includes: administering a pharmaceutically acceptable amount of the fusion protein described in the seventh aspect or the pharmaceutical composition described in the eleventh aspect to a subject. Therefore, the PLA2R antibody-related disease can be effectively treated.

In the twentieth aspect of the present disclosure, the present disclosure provides an immunoadsorption therapy method for preventing or treating a PLA2R antibody-related disease. According to an embodiment of the present disclosure, the method includes: treating a subject with the immunosorbent described in the twelfth aspect.

In the twenty-first aspect of the present disclosure, the present disclosure provides a method for diagnosing a PLA2R antibody-related disease. According to an embodiment of the present disclosure, the method includes: detecting the PLA2R antibody in a sample to be tested by using the fusion protein described in the seventh aspect, the second nucleic acid molecule described in the eighth aspect, the second expression vector described in the ninth aspect, the second recombinant cell described in the tenth aspect, the pharmaceutical composition described in the eleventh aspect, the immunosorbent described in the twelfth aspect, or the kit described in the thirteenth aspect; and determining a content of PLA2R antibody in the sample to be tested based on a detection result.

In the twenty-second aspect of the present disclosure, the present disclosure provides a method for evaluating a stage of a PLA2R antibody-related disease. According to an embodiment of the present disclosure, the method includes: detecting the PLA2R antibody in a sample to be detected by using the fusion protein described in the seventh aspect, the second nucleic acid molecule described in the eighth aspect, the second expression vector described in the ninth aspect, the second recombinant cell described in the tenth aspect, the pharmaceutical composition described in the eleventh aspect, the immunosorbent described in the twelfth aspect or the kit described in the thirteenth aspect; and determining a content of PLA2R antibody in the sample to be tested based on a detection result.

In the twenty-third aspect of the present disclosure, the present disclosure provides a method for evaluating a prognosis of a related disease caused by PLA2R antibody. According to the embodiment of the present disclosure, the method includes: detecting the PLA2R antibody in a sample to be tested by using the fusion protein described in the seventh aspect, the second nucleic acid molecule described in the eighth aspect, the second expression vector described in the ninth aspect, the second recombinant cell described in the tenth aspect, the pharmaceutical composition described in the eleventh aspect, the immunosorbent described in the twelfth aspect, or the kit described in the thirteenth aspect; and determining a content of PLA2R antibody in the sample to be tested based on a detection result.

Additional aspects and advantages of the present disclosure will be described in part in the specification below, and in part will be obvious from the specification below, or will be learned through practice of the present disclosure.

## BRIEF DESCRIPTION OF THE DRAWINGS

The above and/or additional aspects and advantages of the present disclosure will become apparent and easily understood from the description of the embodiments in conjunction with the following figures, in which:

FIG. 1 is a schematic structural diagram of an expression framework in Example 1 of the present disclosure;
FIG. 2 is a diagram of electrophoresis results of pcDNA3.4, trsH, trsL, RtxH and RtxL in Example 1 of the present disclosure;
FIG. 3 is a diagram of electrophoresis results of dTrs1H, dTrs2H and dTrs1L(dTrs2L) in Example 1 of the present disclosure;
FIG. 4 is a diagram of electrophoresis results of dTrs3H and dTrs3L in Example 1 of the present disclosure;
FIG. 5 is a diagram of electrophoresis results of dTrs4H and dTrs4L in Example 1 of the present disclosure;
FIG. 6 illustrates purification effect of Trs, dTrs1-4 and Rtx proteins detected by SDS-PAGE in Example 1 of the present disclosure;
FIG. 7 illustrates detection results of binding of Trs, dTrs1-4 and Rtx proteins to Her2 in Example 1 of the present disclosure;
FIG. 8 is a schematic structural diagram of RC1 to RC8 protein in Example 2 of the present disclosure;
FIG. 9 is a schematic structural diagram of PLA2R in Example 2 of the present disclosure;
FIG. 10 is a diagram of electrophoresis results of pcDNA3.4 in Example 2 of the present disclosure;
FIG. 11 is a diagram of electrophoresis results of pcDNA3.4, RC1H, RC2L, RC4H and RC5L in Example 2 of the present disclosure;
FIG. 12 is a diagram of electrophoresis results of RC3 and RC6 in Example 2 of the present disclosure;
FIG. 13 illustrates purification effect of RC1-8 protein detected by SDS-PAGE in Example 2 of the present disclosure;
FIG. 14 illustrates neutralization effect of RC1 to RC8 on PLA2R-Ab in plasma pMN-2 of membranous nephropathy-positive patient in Example 2 of the present disclosure;
FIG. 15 illustrates neutralization effect of RC1 to RC8 on PLA2R-Ab in plasma pMN-4 of membranous nephropathy-positive patient in Example 2 of the present disclosure;
FIG. 16 illustrates neutralization effect of RC1 to RC8 on PLA2R-Ab in plasma pMN-7 of membranous nephropathy-positive patient in Example 2 of the present disclosure;
FIG. 17 illustrates clearance rate of RC3 and RC6 on PLA2R-Ab adsorption in patient's plasma sample PMN-4 in Example 2 of the present disclosure;
FIG. 18 illustrates clearance rate of RC3 and RC6 on PLA2R-Ab adsorption in patient's plasma sample PMN-7 in Example 2 of the present disclosure;
FIG. 19 is a graph of weight trend of mice subcutaneously injected with RC1, RC2, RC4, RC5, RC7 and RC8 proteins in Example 2 of the present disclosure;
FIG. 20 is a graph of weight trend of mice subcutaneously injected with RC3 and RC6 proteins in Example 2 of the present disclosure;
FIG. 21 illustrates detection result of binding of Trs, dTrs1-4 and Rtx proteins to IgA in Example 1 of the present disclosure;
FIG. 22 illustrates detection result of binding of Trs, dTrs1-4 and Rtx proteins to IgM in Example 1 of the present disclosure;
FIG. 23 illustrates detection result of binding of Trs, dTrs1-4 and Rtx proteins to HSA in Example 1 of the present disclosure; and

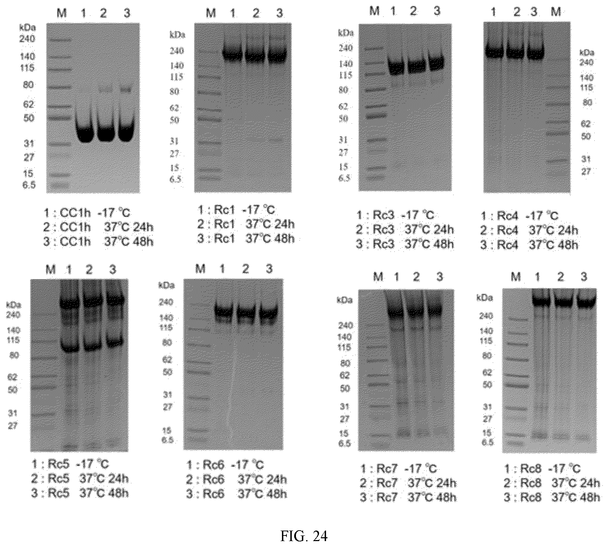

FIG. 24 is a diagram of electrophoresis results of stability investigation of RC1 to RC8 in Example 2 of the present disclosure.

## DETAILED DESCRIPTION

Embodiments of the present disclosure will be described in detail below. The embodiments described below are exemplary and are only used to explain the present disclosure, and they should not be construed as limiting the present disclosure.

It should be noted that the terms "first" and "second" are only used for descriptive purposes, rather than indicating or implying relative importance or implicitly indicating the number of indicated technical features. Therefore, the features defined with "first" and "second" may include one or more of these features explicitly or implicitly. Further, in the description of the present disclosure, unless otherwise specified, "plurality of" means two or more.

As used herein, the term "comprise" or "comprising" is an open expression, that is, including the contents indicated by the present disclosure, but not excluding other aspects.

As used herein, the terms "optionally" or "optional" generally mean that the event or situation described subsequently may happen but not necessarily happen, and the description includes the situation in which the event or situation happens and the situation in which the event or situation does not happen.

As used herein, the term "antibody" usually refers to an immunoglobulin molecule, including two light chains with a relatively light molecular weight and two heavy chains with a relatively heavy molecular weight. The heavy chains (H chains) and the light chains (L chains) are connected by disulfide bonds to form a tetrapeptide chain molecule. An amino acid sequence of an amino terminus (N terminus) of the peptide chain varies greatly, and thus it is referred to as variable region (V region). A carboxyl terminus (C terminus) is relatively stable and varies insignificantly, and thus it is called constant region (C region). The V regions of L chain and H chain are referred to as VL and VH, respectively. In the variable region, the composition and arrangement order of amino acids in some regions have a higher degree of variation, which is referred to as hypervariable region (HVR). The hypervariable region is a position where the antigen and antibody bind to each other, and thus it is also referred to as complementarity-determining region (CDR). The heavy chain variable region and the light chain variable region have each three CDRs thereon.

As used herein, the term "frame region" or "framework region" is abbreviated as FR, which refers to the non-CDR part of the variable region of the heavy chain or light chain of an antibody. About 110 amino acid sequences close to N-terminus of H chain and L chain of antibody vary greatly, while the amino acid sequences of the other parts are relatively constant. In this regard, the light chain and the heavy chain can be each divided into a variable region (V region) and a constant region (C region). The variable region contains a hypervariable region (HVR) or a complementarity-determining region (CDR) and frame region. The variability of FR is lower than that of CDR. There are four FR molecules, i.e., FRl, FR2, FR3, and FR4. When recognizing antibodies, four FR molecules may curl to allow CDR molecules to be close to each other.

As used herein, the term "neutral antibody" refers to a protein structure with low antigen-binding activity, such as Fc segment, or the antibody proposed in the first aspect of the present disclosure.

As used herein, the term "double-stranded antibody" refers to an antibody with a symmetrical structure, for example, an antibody consisting of only two Fc fragments, or a complete antibody consisting of two heavy chains and two light chains, for example, the antibody provided in the first aspect of the present disclosure.

As used herein, when the term "identity", "homology", or "similarity" is a percentage of the same amino acids or nucleotides between two amino acid sequences or nucleic acid sequences determined by a conventional method, for describing an amino acid sequence or a nucleic acid sequence relative to a reference sequence, for example, see Ausubel et al., ed. (1995), Current Protocols in Molecular Biology, Chapter 19 (Greene Publishing and Wiley-Interscience, New York); and ALIGN program (Dayhoff (1978), Atlas of Protein Sequence and Structure 5: Suppl.3 (National Biomedical Research Foundation, Washington, D.C.). Many algorithms are available for aligning sequences and determining sequence identity, including: the homology alignment algorithm (Needleman et al., (1970) J.Mol.Biol.48:443); local homology algorithm (Smith et al., (1981) Adv. Appl. Math.2: 482); similarity search method (Pearson et al., (1988) Proc.Natl.Acad.Sci.85: 2444); Smith-Waterman algorithm (Meth.Mol.Biol.70: 173-187 (1997); and BLASTP, BLASTN, and BLASTX algorithm (see Altschul et al., (1990) J.Mol.Biol.215: 403-410). The computer programs using these algorithms are also available, including but not limited to: ALIGN or Megalign (DNASTAR) software, or WU-BLAST-2 (Altschul et al., Meth. Enzym., 266: 460-480 (1996)); or GAP, BESTFIT, BLAST Altschul, etc., supra, FASTA and TFASTA, available from Genetics Computing Group (GCG) package, 8th edition, Madison, Wisconsin, USA; and CLUSTAL in PC/Gene program provided by Intelligenetics, Mountain View, California.

As used herein, the term "nucleotide" generally refers to ribonucleotide, deoxynucleotide or a modified form of any type of nucleotide, and combinations thereof.

As used herein, the term "at least 85% similarity" refers to at least 85% similarity with each reference sequence, which can be 85%, 86%, 87%, 88%, 89%, 90%, 91%, 92%, 93%, 94%, 95%, 96% and 97% similarity to each reference sequence.

As used herein, the term "expression vector" generally refers to a nucleic acid molecule that can be inserted into a suitable host for self-replication, to transfer the inserted nucleic acid molecule into and/or between host cells. The expression vector may include a vector mainly configured to insert DNA or RNA into cells, a vector mainly configured to replicate DNA or RNA, and an expression vector mainly configured to transcript and/or translate DNA or RNA. The expression vector further includes vectors with various functions as described above. The expression vector may be a polynucleotide that can be transcribed and translated into a polypeptide when introduced into a suitable host cell. Generally, the expression vector can produce a desired expression product by culturing the appropriate host cell containing the expression vector.

As used herein, the term "recombinant cell" usually refers to a cell with stably inherited unique traits that are obtained by modifying or recombining the genetic material of a host cell using genetic engineering technology or cell fusion technology. The term "host cell" refers to a prokaryotic cell or a eukaryotic cell, into which a recombinant expression vector can be introduced. As used herein, the term "transform" or "transfect" refers to the introduction of a nucleic acid (such as a vector) into a cell by various techniques known in the art. Suitable host cells can be transformed or transfected with the DNA sequence of the present disclosure, and can be used for the expression and/or secretion of the target proteins. Examples of suitable host cells that can be used in the present disclosure include immortalized hybridoma cells, NS/0 myeloma cells, 293 cells, Chinese hamster ovary (CHO) cells, HeLa cells, Cap cells (cells derived from human amniotic fluid), insect cells, PER.C6 cells, and CoS cells.

As used herein, the term "fusion protein" refers to a new type of protein formed by fusing at least two protein or polypeptides. The above-mentioned fusion operation can usually be performed by genetic engineering and other technologies, for example, the expression product of two genes recombined by DNA recombination technology. As used herein, fusion protein means that PLA2R and a neutral antibody in double-chain structure are in a fused state. It should be noted that, the PLA2R fusion protein does not mean that all parts of protein are formed by fusion. For example, the two Fc segments in the double-stranded structure can be linked together by chemical bonds such as disulfide bonds.

As used herein, the term "pharmaceutical composition" generally refers to a unit dosage form and can be prepared by any method well known in the pharmaceutical field. All the methods include the step of combining the active ingredient with a carrier, which constitutes one or more accessory ingredients. Generally, a composition is prepared by uniformly and sufficiently combining the active fusion protein with a liquid carrier, a finely divided solid carrier or both.

As used herein, the term "pharmaceutically acceptable" means that the substance or composition must be chemically and/or toxicologically compatible with the other ingredients containing the formulation and/or the mammal to be treated therewith. Preferably, the term "pharmaceutically acceptable" as mentioned in the present disclosure refers to those approved by the federal regulatory agency or the national government or listed in the United States Pharmacopoeia or other approved Pharmacopoeia for use in animals, especially humans.

As used herein, the term "pharmaceutically acceptable excipient" or "pharmaceutically acceptable carrier" may include any solvent, solid excipient, diluent or other liquid excipient, etc., which is suitable for the specific target dosage form. Except to the extent that any conventional excipients are incompatible with the fusion protein of the present disclosure, for example, producing any adverse biological effects or harmful interactions with any other components of a pharmaceutically acceptable composition, their uses are also contemplated by the present disclosure.

For other pharmaceutically acceptable excipients or carriers mentioned herein and their processes, reference can be made to a large number of literatures on this subject, specifically, Handbook of Pharmaceutical Excipients, 3-rd edition, edited by Arthur H. Kibbe, American Pharmaceutical Association, Washington, USA and Pharmaceutical Press, London; and Lexikon der Hilfsstoffe für Pharmazie, Kosmetik and angrenzende Gebiete, edited by H.P.Fiedler, 4-th edition, edited by Cantor, Aulendorf and earlier editions.

As used herein, the term "administrating" refers to introducing a predetermined amount of substance into a patient by a certain suitable route. The fusion protein or the pharmaceutical composition of the present disclosure can be administered by any common route as long as it can reach the intended tissue. Various administration routes can be contemplated, including peritoneal injection, intravenous injection, intramuscular injection, subcutaneous injection, etc. However, the present disclosure is not limited to these exemplified administration routes. Preferably, the composition of the present disclosure is administered by intravenous injection or subcutaneous injection.

As used herein, the term "PLA2R antibody-related disease" generally refers to a disease caused by abnormal PLA2R antibody, such as a related disease caused by increased PLA2R antibody, including but not limited to: PLA2R antibody-positive membranous nephropathy. PLA2R is a membrane protein expressed in glomerular podocytes, and it becomes an antigen for unknown reasons, causing the body to produce the corresponding autoimmune antibody (PLA2R antibody). PLA2R antibody binds to PLA2R and deposits under glomerular basement membrane, triggering downstream effects such as C3 deposition, thereby leading to PLA2R antibody-positive membranous nephropathy.

As used herein, the term "treatment" or "treat" refers to obtaining a desired pharmacological and/or physiological effect. The effect may be preventive in terms of completely or partially preventing the disease or its symptoms, and/or therapeutic in terms of partially or completely curing the disease and/or adverse effects caused by the disease. As used herein, the term "treatment" or "treat" is intend to aim at diseases in mammals, especially humans, including: (a) preventing the occurrence of diseases or disorders in individuals who are susceptible to diseases but have not been diagnosed with the diseases; (b) inhibiting the diseases, for example, retarding the progression of the disease; or (c) alleviating the diseases, such as alleviating the symptoms associated with the diseases. As used herein, the term "treatment" or "treat" includes treating, curing, alleviating, improving, mitigating or inhibiting the disease in an individual by administrating any of a fusion protein, a pharmaceutical composition or a medicament to the individual, including but not limited to, administering a medicament containing the fusion protein described herein to an individual in need thereof.

The present disclosure provides an antibody, a fusion protein and uses thereof, which will be described in detail below.

## ANTIBODY, FIRST NUCLEIC ACID MOLECULE, FIRST EXPRESSION VECTOR, FIRST RECOMBINANT CELL AND USE THEREOF

According to the present disclosure, different sites of the CDR region of the Trastuzumab (Trs) antibody are mutated, and the antibody of the present disclosure is obtained through a large number of experiments. The antibody has extremely weak binding to its antigen Her2 and does not bind to any other antigens. The antibody has extremely low antigen-binding activity. Thus, the antibody can be used as a neutral antibody to fuse with a biologically active molecule to obtain a fusion protein. The fusion protein can bind to a specific binding substance of the biologically active molecule, for example, the fusion protein obtained by combining the neutral antibody with PLA2R can specifically bind to PLA2R antibody, without binding to other antibodies. In this way, the side effects of non-specific binding can be avoided, when the fusion protein is used as a medicament. The fusion protein obtained by fusing a neutral antibody with an antigen, as a medicament, can bind to the specific antibody of the antigen without binding to other antibodies, and thus it can specifically clear the cells producing the specific antibody of the antigen.

Therefore, in a first aspect of the present disclosure, the present disclosure provides an antibody. According to an embodiment of the present disclosure, the antibody includes a light chain variable region CDR and a heavy chain variable region CDR. A heavy chain variable region CDR1 sequence is as set forth in $GX_1X_2X_3X_4X_5X_6X_7X_8X_9$. A heavy chain variable region CDR2 sequence is as set forth in $X_{10}X_{11}X_{12}X_{13}X_{14}X_{15}GX_{16}X_{17}X_{18}X_{19}X_{20}\ X_{21}\ X_{22}\ X_{23}$. A heavy chain variable region CDR3 sequence is as set forth in $X_{24}GGX_{25}X_{26}X_{27}X_{28}X_{29}X_{30}X_{31}Y$. $X_1$ is G or F, $X_2$ is G or N, $X_3$ is G or I, $X_4$ is G, K or S, $X_5$ is G or D, $X_6$ is G or T, $X_7$ is G or Y, $X_8$ is G or I, $X_9$ is H or S, $X_{10}$ is R or G, $X_{11}$ is G or I, $X_{12}$ is G or Y, $X_{13}$ is G or P, $X_{14}$ is T or S, $X_{15}$ is G or N, $X_{16}$ is G or Y, $X_{17}$ is G or T, $X_{18}$ is G, S or R, $X_{19}$ is G or Y, $X_{20}$ is G or A, $X_{21}$ is G or D, $X_{22}$ is G or S, $X_{23}$ is S or V, $X_{24}$ is G or W, $X_{25}$ is G or D, $X_{26}$ is G or S, $X_{27}$ is G or F, $X_{28}$ is G or Y, $X_{29}$ is G or A, $X_{30}$ is G or M, and $X_{31}$ is S, G or D, with a provision that the followings do not occur at the same time: $X_1$ is F, $X_2$ is N, $X_3$ is I, $X_4$ is K, $X_5$ is D, $X_6$ is T, $X_7$ is Y, $X_8$ is I, $X_9$ is H, $X_{10}$ is R, $X_{11}$ is I, $X_{12}$ is Y, $X_{13}$ is P, $X_{14}$ is T, $X_{15}$ is N, $X_{16}$ is Y, $X_{17}$ is T, $X_{18}$ is R, $X_{19}$ is Y, $X_{20}$ is A, $X_{21}$ is D, $X_{22}$ is S, $X_{23}$ is V, $X_{24}$ is W, $X_{25}$ is D, $X_{26}$ is G, $X_{27}$ is F, $X_{28}$ is Y, $X_{29}$ is A, $X_{30}$ is M, and $X_{31}$ is D. A light chain variable region CDR1 sequence is as set forth in $X_{32}X_{33}X_{34}X_{35}X_{36}X_{37}X_{38}X_{39}X_{40}X_{41}A$. A light chain variable region CDR2 sequence is as set forth in $X_{42}X_{43}X_{44}X_{45}X_{46}X_{47}S$. A light chain variable region CDR3 sequence is as set forth in $X_{48}X_{49}X_{50}X_{51}X_{52}X_{53}X_{54}X_{55}X_{56}$. $X_{32}$ is G or R, $X_{33}$ is G or A, $X_{34}$ is G or S, $X_{35}$ is G or Q, $X_{36}$ is G, D or S, $X_{37}$ is G or V, $X_{38}$ is G or N, $X_{39}$ is G or T, $X_{40}$ is G or A, $X_{41}$ is S or V, $X_{42}$ is G or S, $X_{43}$ is G or A, $X_{44}$ is G or S, $X_{45}$ is G or F, $X_{46}$ is L or S, $X_{47}$ is G or Y, $X_{48}$ is G or Q, $X_{49}$ is G or Q, $X_{50}$ is G or H, $X_{51}$ is G or Y, $X_{52}$ is S or T, $X_{53}$ is G or T, $X_{54}$ is G or P, $X_{55}$ is G or P, and $X_{56}$ is G or T, with a provision that the followings do not occur at the same time: $X_{32}$ is R, $X_{33}$ is A, $X_{34}$ is S, $X_{35}$ is Q, $X_{36}$ is D, $X_{37}$ is V, $X_{38}$ is N, $X_{39}$ is T, $X_{40}$ is A, $X_{41}$ is V, $X_{42}$ is S, $X_{43}$ is A, $X_{44}$ is S, $X_{45}$ is F, $X_{46}$ is L, $X_{47}$ is Y, $X_{48}$ is Q, $X_{49}$ is Q, $X_{50}$ is H, $X_{51}$ is Y, $X_{52}$ is T, $X_{53}$ is T, $X_{54}$ is P, $X_{55}$ is P, and $X_{56}$ is T. Through a large number of experiments, the inventor found that the above-mentioned antibody has the advantages of extremely low antigen-binding activity and high expression level.

According to an embodiment of the present disclosure, the heavy chain variable region CDR and the light chain variable region CDR satisfy at least one of the following conditions: $X_4$ is G or S, $X_5$ is G, $X_{21}$ is G, $X_{31}$ is S or G, and $X_{36}$ is G or S.

According to the embodiment of the present disclosure, in the heavy chain variable region CDR, $X_1$ is G or F, $X_2$ is G or N, $X_3$ is G or I, $X_4$ is G or S, $X_5$ is G, $X_6$ is G or T, $X_7$ is G or Y, $X_8$ is G or I, $X_9$ is H or S, $X_{10}$ is G, $X_{11}$ is G or I, $X_{12}$ is G or Y, $X_{13}$ is G or P, $X_{14}$ is T or S, $X_{15}$ is G or N, $X_{16}$ is G, $X_{17}$ is G or T, $X_{18}$ is G or S, $X_{19}$ is G or Y, $X_{20}$ is G or A, $X_{21}$ is G, $X_{22}$ is G or S, $X_{23}$ is S or V, $X_{24}$ is G, $X_{25}$ is G or D, $X_{26}$ is G or S, $X_{27}$ is G or F, $X_{28}$ is G, $X_{29}$ is G or A, $X_{30}$ is G or M, and $X_{31}$ is S or G; in the light chain variable region CDR, $X_{32}$ is G or R, $X_{33}$ is G or A, $X_{34}$ is G or S, $X_{35}$ is G or Q, $X_{36}$ is G or S, $X_{37}$ is G or V, $X_{38}$ is G, $X_{39}$ is G or T, $X_{40}$ is G or A, $X_{41}$ is S or V, $X_{42}$ is G or S, $X_{43}$ is G or A, $X_{44}$ is G or S, $X_{45}$ is G, $X_{46}$ is L or S, $X_{47}$ is G or Y, $X_{48}$ is G or Q, $X_{49}$ is G or Q, $X_{50}$ is G or H, $X_{51}$ is G, $X_{52}$ is S or T, $X_{53}$ is G or T, $X_{54}$ is G or P, $X_{55}$ is G or P, and $X_{56}$ is G or T.

According to an embodiment of the present disclosure, the antibody includes a CDR sequence selected from at least one of: light chain variable region CDR sequences as set forth in SEQ ID NO: 1, SEQ ID NO: 2, SEQ ID NO: 3, SEQ ID NO: 7, SEQ ID NO: 8, SEQ ID NO: 9, SEQ ID NO: 83, SEQ ID NO: 84, and SEQ ID NO: 85; and heavy chain variable region CDR sequences as set forth in SEQ ID NO: 4, SEQ ID NO: 5, SEQ ID NO: 6, SEQ ID NO: 10, SEQ ID NO: 11, SEQ ID NO: 12, SEQ ID NO: 86, SEQ ID NO: 87, SEQ ID NO: 88, SEQ ID NO: 89, SEQ ID NO: 90, and SEQ ID NO: 91.

        RASQGVGTAVA (SEQ ID NO: 1).
        SASGLYS (SEQ ID NO: 2).
        QQHGTTPPT (SEQ ID NO: 3).
        GFNIGGTGIH (SEQ ID NO: 4).
        GIYPTNGGTGYAGSV (SEQ ID NO: 5).
        GGGDGFGAMGY (SEQ ID NO: 6).
        GGGGSGGGGSA (SEQ ID NO: 7).

GGGGSGS (SEQ ID NO: 8).
GGGGSGGGG (SEQ ID NO: 9).
GGGGSGGGGS (SEQ ID NO: 10).
GGGGSGGGGSGGGGS (SEQ ID NO: 11).
GGGGSGGGGSY (SEQ ID NO: 12).
RASQDVGTAVA (SEQ ID NO: 83).
SASGLYS (SEQ ID NO: 84).
QQHGTTPPT (SEQ ID NO: 85).
GFNIKDTYIH (SEQ ID NO: 86).
RIYPTNGGTRYADSV (SEQ ID NO: 87).
WGGDGFGAMDY (SEQ ID NO: 88).
GFNIKDTGIH (SEQ ID NO: 89).
GIYPTNGGTGYADSV (SEQ ID NO: 90).
GGGDGFGAMDY (SEQ ID NO: 91).

In other embodiments, the antibody provided by the present disclosure has a conservative amino acid substitution compared with the heavy chain and light chain above-mentioned. The "conservative amino acid substitution" means that an amino acid is replaced by another amino acid residue with a biological, chemical or structural similarity. The "biological similarity" means that the substitution does not destroy the biological activity of the antibody with low antigen-binding activity. The "structural similarity" means that the amino acids have side chains of similar length, such as alanine, glycine or serine, or have side chains of similar size. The "chemical similarity" means that amino acids have the same charge or are all hydrophilic or hydrophobic. For example, the hydrophobic residues of isoleucine, valine, leucine or methionine are substituted by each other. Alternatively, lysine is substituted by a polar amino acid such as arginine, aspartic acid glutamic acid, aspartic acid is substituted by glutamine, and threonine is substituted by serine, etc.

According to an embodiment of the present disclosure, the light chain variable region CDR sequences include at least one of amino acid sequences having at least 85% similarity to SEQ ID NO: 1 to SEQ ID NO: 3, SEQ ID NO: 7 to SEQ ID NO: 9, and SEQ ID NO: 83 to SEQ ID NO: 85; and the heavy chain variable region CDR sequences include at least one of amino acid sequences having at least 85% similarity to SEQ ID NO: 4 to SEQ ID NO: 6, SEQ ID NO: 10 to SEQ ID NO: 12, SEQ ID NO: 86 to SEQ ID NO: 88, and SEQ ID NO: 89 to SEQ ID NO: 91.

According to an embodiment of the present disclosure, the antibody includes the light chain variable region CDR1 sequence, the light chain variable region CDR2 sequence, and the light chain variable region CDR3 sequence as set forth in SEQ ID NO: 1, SEQ ID NO: 2, and SEQ ID NO: 3, respectively, or as set forth in amino acid sequences having at least 85% similarity to SEQ ID NO: 1, SEQ ID NO: 2, and SEQ ID NO: 3, respectively; or the antibody includes the light chain variable region CDR1 sequence, the light chain variable region CDR2 sequence, and the light chain variable region CDR3 sequence as set forth in SEQ ID NO: 7, SEQ ID NO: 8, and SEQ ID NO: 9, respectively, or as set forth in amino acid sequences having at least 85% similarity to SEQ ID NO: 7, SEQ ID NO: 8, and SEQ ID NO: 9, respectively; or the antibody includes the light chain variable region CDR1 sequence, the light chain variable region CDR2 sequence, and the light chain variable region CDR3 sequence as set forth in SEQ ID NO: 83, SEQ ID NO: 84, and SEQ ID NO: 85, respectively, or as set forth in amino acid sequences having at least 85% similarity to SEQ ID NO: 83, SEQ ID NO: 84, and SEQ ID NO: 85, respectively.

According to an embodiment of the present disclosure, the antibody includes the heavy chain variable region CDR1 sequence, the heavy chain variable region CDR2 sequence, and the heavy chain variable region CDR3 sequence as set forth in SEQ ID NO: 4, SEQ ID NO: 5, and SEQ ID NO: 6, respectively, or as set forth in amino acid sequences having at least 85% similarity to SEQ ID NO: 4, SEQ ID NO: 5, and SEQ ID NO: 6, respectively; or the antibody includes the heavy chain variable region CDR1 sequence, the heavy chain variable region CDR2 sequence, and the heavy chain variable region CDR3 sequence as set forth in SEQ ID NO: 10, SEQ ID NO: 11, and SEQ ID NO: 12, respectively, or as set forth in amino acid sequences having at least 85% similarity to SEQ ID NO: 10, SEQ ID NO: 11, and SEQ ID NO: 12, respectively; or the antibody includes the heavy chain variable region CDR1 sequence, the heavy chain variable region CDR2 sequence, and the heavy chain variable region CDR3 sequence as set forth in SEQ ID NO: 86, SEQ ID NO: 87, and SEQ ID NO: 88, respectively, or as set forth in amino acid sequences having at least 85% similarity to SEQ ID NO: 86, SEQ ID NO: 87, and SEQ ID NO: 88, respectively; or the antibody includes the heavy chain variable region CDR1 sequence, the heavy chain variable region CDR2 sequence, and the heavy chain variable region CDR3 sequence as set forth in SEQ ID NO: 89, SEQ ID NO: 90, and SEQ ID NO: 91, respectively, or as set forth in amino acid sequences having at least 85% similarity to SEQ ID NO: 89, SEQ ID NO: 90, and SEQ ID NO: 91, respectively.

According to an embodiment of the present disclosure, the antibody includes: the light chain variable region CDR1 sequence, the light chain variable region CDR2 sequence, and the light chain variable region CDR3 sequence as set forth in SEQ ID NO: 1, SEQ ID NO: 2, and SEQ ID NO: 3, respectively, or as set forth in amino acid sequences having at least 85% similarity to SEQ ID NO: 1, SEQ ID NO: 2, and SEQ ID NO: 3, respectively, and the heavy chain variable region CDR1 sequence, the heavy chain variable region CDR2 sequence, and the heavy chain variable region CDR3 sequence as set forth in SEQ ID NO: 4, SEQ ID NO: 5, and SEQ ID NO: 6, respectively, or as set forth in amino acid sequences having at least 85% similarity to SEQ ID NO: 4, SEQ ID NO: 5, and SEQ ID NO: 6, respectively; or the antibody includes: the light chain variable region CDR1 sequence, the light chain variable region CDR2 sequence, and the light chain variable region CDR3 sequence as set forth in SEQ ID NO: 7, SEQ ID NO: 8, and SEQ ID NO: 9, respectively, or as set forth in amino acid sequences having at least 85% similarity to SEQ ID NO: 7, SEQ ID NO: 8, and SEQ ID NO: 9, respectively, and the heavy chain variable region CDR1 sequence, the heavy chain variable region CDR2 sequence, and the heavy chain variable region CDR3 sequence as set forth in SEQ ID NO: 10, SEQ ID NO: 11, and SEQ ID NO: 12, respectively, or as set forth in amino acid sequences having at least 85% similarity to SEQ ID NO: 10, SEQ ID NO: 11, and SEQ ID NO: 12, respectively; or the antibody includes: the light chain variable region CDR1 sequence, the light chain variable region CDR2 sequence, and the light chain variable region CDR3 sequence as set forth in SEQ ID NO: 83, SEQ ID NO: 84, and SEQ ID NO: 85, respectively, or as set forth in amino acid sequences having at least 85% similarity to SEQ ID NO: 83, SEQ ID NO: 84, and SEQ ID NO: 85, respectively, and the heavy chain variable region CDR1 sequence, the heavy chain variable region CDR2 sequence, and the heavy chain variable region CDR3 sequence as set forth in SEQ ID NO: 86, SEQ ID NO: 87, and SEQ ID NO: 88, respectively, or as set forth in amino acid sequences having at least 85% similarity to SEQ ID NO: 86, SEQ ID NO: 87, and SEQ ID NO: 88, respectively; or the antibody includes: the light chain variable region CDR1 sequence, the light chain variable region CDR2 sequence, and the light chain variable region CDR3 sequence as set forth in SEQ ID NO: 83, SEQ ID NO: 84, and SEQ ID NO: 85, respectively, or as set forth in amino acid sequences having at least 85% similarity to SEQ ID NO: 83, SEQ ID NO: 84, and SEQ ID NO: 85, respectively, and the heavy chain variable region CDR1 sequence, the heavy chain variable region CDR2 sequence, and the heavy chain variable region CDR3 sequence as set forth in SEQ ID NO: 89, SEQ ID NO: 90, and SEQ ID NO: 91, respectively, or as set forth in amino acid sequences having at least 85% similarity to SEQ ID NO: 89, SEQ ID NO: 90, and SEQ ID NO: 91, respectively.

According to an embodiment of the present disclosure, the antibody includes at least one of a heavy chain frame region sequence and a light chain frame region sequence. At least a part of the at least one of the heavy chain frame region sequence and the light chain frame region sequence is derived from at least one of a murine antibody, a humanized antibody, a primate-derived antibody, or a mutant thereof, and preferably, a humanized antibody.

According to an embodiment of the present disclosure, the antibody includes: a light chain variable region, an amino acid sequence of the light chain variable region being as set forth in SEQ ID NO: 13, SEQ ID NO: 14, or SEQ ID NO: 95; and/or a heavy chain variable region, an amino acid sequence of the heavy chain variable region being as set forth in SEQ ID NO: 15, SEQ ID NO: 16, SEQ ID NO: 96, or SEQ ID NO: 97. The CDR regions of the heavy chain variable region sequence (as set forth in SEQ ID NO: 4 to SEQ ID NO: 6 and SEQ ID NO: 10 to SEQ ID NO: 12) and the CDR regions of the light chain variable region sequence (as set forth in SEQ ID NO: 1 to SEQ ID NO: 3 and SEQ ID NO: 7 to SEQ ID NO: 9) of the above antibody can be obtained based on the antibody sequence alignment database (NCBI or IMGT) or related software.

DIQMTQSPSSLSASVGDRVTITCRASQGVGTAVAWYQQKPGKAPKLLIYSA

SGLYSGVPSRFSGSRSGTDFTLTISSLQPEDFATYYCQQHGTTPPTFGQGTKVEIK  (SEQ

ID NO: 13).

DIQMTQSPSSLSASVGDRVTITCGGGGSGGGGSAWYQQKPGKAPKLLIYG

GGGSGSGVPSRFSGSRSGTDFTLTISSLQPEDFATYYCGGGGSGGGGFGQGTKVEIK

(SEQ ID NO: 14).

EVQLVESGGGLVQPGGSLRLSCAASGFNIGGTGIHWVRQAPGKGLEWVAG

IYPTNGGTGYAGSVKGRFTISADTSKNTAYLQMNSLRAEDTAVYYCSRGGGDGFGAM

GYWGQGTLVTV (SEQ ID NO: 15).

EVQLVESGGGLVQPGGSLRLSCAASGGGGSGGGGSWVRQAPGKGLEWVA
GGGGSGGGGSGGGGSKGRFTISADTSKNTAYLQMNSLRAEDTAVYYCSRGGGGSGG
GGSYWGQGTLVTV (SEQ ID NO: 16).

DIQMTQSPSSLSASVGDRVTITCRASQDVGTAVAWYQQKPGKAPKLLIYSA
SGLYSGVPSRFSGSRSGTDFTLTISSLQPEDFATYYCQQHGTTPPTFGQGTKVEIK (SEQ
ID NO: 95).

EVQLVESGGGLVQPGGSLRLSCAASGFNIKDTYIHWVRQAPGKGLEWVAR
IYPTNGGTRYADSVKGRFTISADTSKNTAYLQMNSLRAEDTAVYYCSRWGGDGFGAM
DYWGQGTLVTV (SEQ ID NO: 96).

EVQLVESGGGLVQPGGSLRLSCAASGFNIKDTGIHWVRQAPGKGLEWVAG
IYPTNGGTGYADSVKGRFTISADTSKNTAYLQMNSLRAEDTAVYYCSRGGGDGFGAM
DYWGQGTLVTV (SEQ ID NO: 97).

According to an embodiment of the present disclosure, the light chain variable region sequence of the antibody has a conservative amino acid substitution compared with the amino acid sequence as set forth in SEQ ID NO: 13 or SEQ ID NO: 14; or the heavy chain variable region sequence of the antibody has a conservative amino acid substitution compared with the amino acid sequence as set forth in SEQ ID NO: 15 or SEQ ID NO: 16. The biological function of the antibody is not changed by the conservative amino acid substitution. In some specific embodiments, the conservative amino acid substitution may occur at amino acids outside the CDR region in the heavy chain variable region and the light chain variable region.

According to an embodiment of the present disclosure, the amino acid sequence of the light chain variable region of the antibody is as set forth in SEQ ID NO: 13, and the heavy chain variable region with the amino acid sequence as set forth in SEQ ID NO: 15.

According to an embodiment of the present disclosure, the amino acid sequence of the light chain variable region of the antibody is as set forth in SEQ ID NO: 14, and the heavy chain variable region with the amino acid sequence as set forth in SEQ ID NO: 16.

According to an embodiment of the present disclosure, the amino acid sequence of the light chain variable region of the antibody is as set forth in SEQ ID NO: 95, and the heavy chain variable region with the amino acid sequence as set forth in SEQ ID NO: 96.

According to an embodiment of the present disclosure, the amino acid sequence of the light chain variable region of the antibody is as set forth in SEQ ID NO: 95, and the amino acid sequence of the light chain variable region of the antibody is as set forth in SEQ ID NO: 97.

According to an embodiment of the present disclosure, the antibody includes at least one of a heavy chain constant region and a light chain constant region. At least a part of the at least one of the heavy chain constant region and the light chain constant region is derived from at least one of a murine antibody, a humanized antibody, a primate-derived antibody, or a mutant thereof, and preferably a humanized antibody.

According to an embodiment of the present disclosure, the antibody includes a heavy chain and a light chain. An amino acid sequence of the heavy chain of the antibody is as set forth in SEQ ID NO: 17, SEQ ID NO: 18, SEQ ID NO: 33, or SEQ ID NO: 35. The amino acid sequence of the light chain is as set forth in SEQ ID NO: 19, SEQ ID NO: 20, or SEQ ID NO: 34.

EVQLVESGGGLVQPGGSLRLSCAASGFNIGGTGIHWVRQAPGKGLEWVAG IYPTNGGTGYAGSVKGRFTISADTSKNTAYLQMNSLRAEDTAVYYCSRGGGDGFGAM GYWGQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSC

DKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEK TISKAKGQPREPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKSLSLSPGK (SEQ ID NO: 17).

EVQLVESGGGLVQPGGSLRLSCAASGGGGSGGGGSWVRQAPGKGLEWVA GGGGSGGGGSGGGGSKGRFTISADTSKNTAYLQMNSLRAEDTAVYYCSRGGGGSGG GGSYWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKKVEPK SCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAP IEKTISKAKGQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQKSLSLSPGK (SEQ ID NO: 18).

DIQMTQSPSSLSASVGDRVTITCRASQGVGTAVAWYQQKPGKAPKLLIYSA SGLYSGVPSRFSGSRSGTDFTLTISSLQPEDFATYYCQQHGTTPPTFGQGTKVEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 19).

DIQMTQSPSSLSASVGDRVTITCGGGGSGGGGSAWYQQKPGKAPKLLIYG GGGSGSGVPSRFSGSRSGTDFTLTISSLQPEDFATYYCGGGGSGGGGFGQGTKVEIKRT VAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC (SEQ ID NO: 20).

According to an embodiment of the present disclosure, the amino acid sequence of the heavy chain of the antibody is as set forth in SEQ ID NO: 17, and the amino acid sequence of the light chain is as set forth in SEQ ID NO: 19.

According to an embodiment of the present disclosure, the amino acid sequence of the heavy chain of the antibody is as set forth in SEQ ID NO: 18, and the amino acid sequence of the light chain is as set forth in SEQ ID NO: 20.

According to an embodiment of the present disclosure, the amino acid sequence of the heavy chain of the antibody is as set forth in SEQ ID NO: 33, and the amino acid sequence of the light chain is as set forth in SEQ ID NO: 34.

According to an embodiment of the present disclosure, the amino acid sequence of the heavy chain of the antibody is as set forth in SEQ ID NO: 35, and the amino acid sequence of the light chain is as set forth in SEQ ID NO: 34.

In the process of preparing or obtaining the above-mentioned antibodies, nucleic acid molecules expressing these antibodies can be linked to different carriers and then expressed in different cells to obtain the corresponding antibodies.

To this end, in a second aspect of the present disclosure, the present disclosure provides a first nucleic acid molecule. According to an embodiment of the present disclosure, the first nucleic acid molecule encodes the antibody described in the first aspect. The antibody encoded by the first nucleic acid molecule according to the embodiment of the present disclosure has the advantages of extremely low antigen-binding activity and high expression level.

According to an embodiment of the present disclosure, the nucleic acid molecule is DNA.

The nucleotide sequence includes SEQ ID NO: 25 or a nucleotide sequence having at least 80% similarity to SEQ ID NO: 25. The heavy chain having the amino acid sequence as set forth in SEQ ID NO: 17 is encoded by SEQ ID NO: 25.

GAAGTCCAGCTGGTGGAAAGTGGCGGTGGACTGGTTCAACCAGGAGG

AAGCCTAAGACTAAGCTGCGCCGCCAGCGGTTTTAACATCGGTGGCACAGGCATC

CATTGGGTCCGGCAGGCACCCGGCAAAGGTTTAGAGTGGGTCGCAGGCATCTACC

CCACTAATGGTGGCACCGGCTACGCTGGTAGCGTGAAGGGCAGGTTCACCATTAG

CGCTGACACCAGCAAGAACACTGCTTATCTGCAGATGAATAGCCTGCGTGCCGAA

GACACAGCCGTCTATTACTGTAGTAGAGGCGGAGGTGATGGGTTCGGCGCGATGG

GCTACTGGGGACAGGGCACACTCGTGACCGTTAGTAGCGCTAGCACCAAGGGCCC

ATCGGTCTTCCCCCTGGCACCCTCCTCCAAGAGCACCTCTGGGGGCACAGCGGCC

CTGGGCTGCCTGGTCAAGGACTACTTCCCCGAACCGGTGACGGTGTCGTGGAACT

CAGGCGCCCTGACCAGCGGCGTGCACACCTTCCCGGCTGTCCTACAGTCCTCAGG

ACTCTACTCCCTCAGCAGCGTGGTGACCGTGCCCTCCAGCAGCTTGGGCACCCAG

ACCTACATCTGCAACGTGAATCACAAGCCCAGCAACACCAAGGTGGACAAGAAA
GTAGAGCCCAAATCTTGTGACAAAACTCACACATGCCCACCGTGCCCAGCACCTG
AACTCCTGGGGGGACCGTCAGTCTTCCTCTTCCCCCCAAAACCCAAGGACACCCT
CATGATCTCCCGGACCCCTGAGGTCACATGCGTGGTGGTGGACGTGAGCCACGAA
GACCCTGAGGTCAAGTTCAACTGGTACGTGGACGGCGTGGAGGTGCATAATGCCA
AGACAAAGCCGCGGGAGGAGCAGTACAACAGCACGTACCGTGTGGTCAGCGTCC
TCACCGTCCTGCACCAGGACTGGCTGAATGGCAAGGAGTACAAGTGCAAGGTCTC
CAACAAAGCCCTCCCAGCCCCCATCGAGAAAACCATCTCCAAAGCCAAAGGGCA
GCCCCGAGAACCACAGGTGTACACCCTGCCCCCATCCCGGGATGAGCTGACCAAG
AACCAGGTCAGCCTGACCTGCCTGGTCAAAGGCTTCTATCCCAGCGACATCGCCG
TGGAGTGGGAGAGCAATGGGCAGCCGGAGAACAACTACAAGACCACGCCTCCCG
TGCTGGACTCCGACGGCTCCTTCTTCCTCTACAGCAAGCTCACCGTGGACAAGAG
CAGGTGGCAGCAGGGGAACGTCTTCTCATGCTCCGTGATGCATGAGGCTCTGCAC
AACCACTACACGCAGAAGAGCCTCTCCCTGTCTCCGGGTAAA (SEQ ID NO: 25).

The nucleotide sequence includes SEQ ID NO: 26 or a nucleotide sequence having at least 80% similarity to SEQ ID NO: 26. The light chain having the amino acid sequence as set forth in SEQ ID NO: 19 is encoded by SEQ ID NO: 26.

GATATACAGATGACGCAGAGCCCTAGCAGTCTAAGTGCAAGCGTTGGAG
ACCGTGTCACCATTACCTGCCGCGCTAGCCAGGGCGTCGGCACCGCAGTCGCCTG
GTATCAGCAAAAACCGGGTAAGGCTCCAAAGCTGTTGATCTACAGTGCCAGCGGC
CTGTACAGCGGTGTGCCAAGTAGGTTCAGCGGGAGCCGGAGTGGGACAGACTTTA
CACTCACTATTAGCAGTCTGCAGCCCGAGGACTTCGCCACCTACTACTGTCAGCAG
CATGGCACAACACCCCAACATTCGGGCAGGGAACCAAAGTTGAGATAAAACGA
ACGGTTGCCGCGCCAAGCGTATTTATCTTTCCCCCCAGTGATGAGCAACTGAAGAG
TGGTACAGCCAGCGTTGTCTGCCTTTTGAATAACTTCTATCCTCGGGAGGCAAAGG
TCCAGTGGAAAGTGGATAATGCACTCCAAAGCGGTAACAGCCAGGAGAGTGTGAC
AGAACAGGACAGCAAAGATAGTACCTACAGCCTCAGTAGTACCCTCACACTGAGC
AAAGCGGACTATGAAAAGCACAAGGTGTACGCTTGTGAAGTGACCCACCAGGGC
CTAAGCAGCCCTGTTACTAAGAGCTTCAACAGAGGCGAATGT (SEQ ID NO: 26).

The nucleotide sequence includes SEQ ID NO: 27 or a nucleotide sequence having at least 80% similarity to SEQ ID NO: 27. The heavy chain having the amino acid sequence as set forth in SEQ ID NO: 18 is encoded by SEQ ID NO: 27.

GAAGTCCAGCTGGTGGAAAGTGGCGGTGGACTGGTTCAACCAGGAGG

AAGCCTAAGACTAAGCTGCGCCGCCAGCGGTGGCGGGGGTAGCGGTGGCGGGGG

TTCGTGGGTCCGGCAGGCACCCGGCAAAGGTTTAGAGTGGGTCGCAGGTGGCGG

GGGTAGCGGTGGCGGGGGTTCGGGCGGGGGTGGTTCCAAGGGCAGGTTCACCATT

AGCGCTGACACCAGCAAGAACACTGCTTATCTGCAGATGAATAGCCTGCGTGCCG

AAGACACAGCCGTCTATTACTGTAGTAGAGGCGGAGGTGGTAGCGGTGGCGGGGG

TTCGTACTGGGGACAGGGCACACTCGTGACCGTTAGTAGCGCTAGCACCAAGGGC

CCATCGGTCTTCCCCCTGGCACCCTCCTCCAAGAGCACCTCTGGGGGCACAGCGG

CCCTGGGCTGCCTGGTCAAGGACTACTTCCCCGAACCGGTGACGGTGTCGTGGAA

CTCAGGCGCCCTGACCAGCGGCGTGCACACCTTCCCGGCTGTCCTACAGTCCTCA

GGACTCTACTCCCTCAGCAGCGTGGTGACCGTGCCCTCCAGCAGCTTGGGCACCC

AGACCTACATCTGCAACGTGAATCACAAGCCCAGCAACACCAAGGTGGACAAGA

AAGTAGAGCCCAAATCTTGTGACAAAACTCACACATGCCCACCGTGCCCAGCACC

TGAACTCCTGGGGGGACCGTCAGTCTTCCTCTTCCCCCCAAAACCCAAGGACACC

CTCATGATCTCCCGGACCCCTGAGGTCACATGCGTGGTGGTGGACGTGAGCCACG

AAGACCCTGAGGTCAAGTTCAACTGGTACGTGGACGGCGTGGAGGTGCATAATGC

CAAGACAAAGCCGCGGGAGGAGCAGTACAACAGCACGTACCGTGTGGTCAGCGT

CCTCACCGTCCTGCACCAGGACTGGCTGAATGGCAAGGAGTACAAGTGCAAGGTC

TCCAACAAAGCCCTCCCAGCCCCCATCGAGAAAACCATCTCCAAAGCCAAAGGGC

AGCCCCGAGAACCACAGGTGTACACCCTGCCCCCATCCCGGGATGAGCTGACCAA

GAACCAGGTCAGCCTGACCTGCCTGGTCAAAGGCTTCTATCCCAGCGACATCGCC

GTGGAGTGGGAGAGCAATGGGCAGCCGGAGAACAACTACAAGACCACGCCTCCC

GTGCTGGACTCCGACGGCTCCTTCTTCCTCTACAGCAAGCTCACCGTGGACAAGA

GCAGGTGGCAGCAGGGGAACGTCTTCTCATGCTCCGTGATGCATGAGGCTCTGCA

CAACCACTACACGCAGAAGAGCCTCTCCCTGTCTCCGGGTAAA (SEQ ID NO: 27).

The nucleotide sequence includes SEQ ID NO: 28 or a nucleotide sequence having at least 80% similarity to SEQ ID NO: 28. The light chain having the amino acid sequence as set forth in SEQ ID NO: 20 is encoded by SEQ ID NO: 28.

GATATACAGATGACGCAGAGCCCTAGCAGTCTAAGTGCAAGCGTTGGAG

ACCGTGTCACCATTACCTGCGGTGGCGGGGGTAGCGGTGGCGGGGGTTCGGCCTG

GTATCAGCAAAAACCGGGTAAGGCTCCAAAGCTGTTGATCTACGGTGGCGGGGGT

AGCGGCAGCGGTGTGCCAAGTAGGTTCAGCGGGAGCCGGAGTGGGACAGACTTT

ACACTCACTATTAGCAGTCTGCAGCCCGAGGACTTCGCCACCTACTACTGTGGTGG

CGGGGGTAGCGGTGGCGGGGGTTTCGGGCAGGGAACCAAAGTTGAGATAAAACG

AACGGTTGCCGCGCCAAGCGTATTTATCTTTCCCCCCAGTGATGAGCAACTGAAGA

GTGGTACAGCCAGCGTTGTCTGCCTTTTGAATAACTTCTATCCTCGGGAGGCAAAG

GTCCAGTGGAAAGTGGATAATGCACTCCAAAGCGGTAACAGCCAGGAGAGTGTGA

CAGAACAGGACAGCAAAGATAGTACCTACAGCCTCAGTAGTACCCTCACACTGAG

CAAAGCGGACTATGAAAGCACAAGGTGTACGCTTGTGAAGTGACCCACCAGGG

CCTAAGCAGCCCTGTTACTAAGAGCTTCAACAGAGGCGAATGT (SEQ ID NO: 28).

Those skilled in the art can understand that the features and advantages described above in terms of the antibody are also applicable to the first nucleic acid molecule, which will not be repeated here.

In a third aspect of the present disclosure, the present disclosure provides a first expression vector. According to an embodiment of the present disclosure, the first expression vector carries the first nucleic acid molecule described in the second aspect. When the above-mentioned isolated polynucleotide is linked to the first expression vector, the polynucleotide can be directly or indirectly linked to control elements on the first expression vector, as long as these control elements can control the translation and expression of the polynucleotide. These control elements may be directly from the first expression vector itself or may be exogenous, i.e., not from the first expression vector itself. The polynucleotide may be operably linked to the control element.

As used herein, "operably linked" refers to linking an exogenous gene to a vector, enabling control elements in the vector such as transcription control sequences and translation control sequences to play their expected function of regulating the transcription and translation of the exogenous gene. The polynucleotides for encoding the heavy chain and light chain of the antibody can be independently inserted into different vectors, but usually inserted into the same vector. Common vectors can be, for example, plasmids, phages and the like.

According to an embodiment of the present disclosure, the first expression vector is a eukaryotic expression vector, and preferably, the first expression vector is a lentivirus vector.

Those skilled in the art can understand that the features and advantages described above in terms of the antibody are also applicable to the first expression vector, which will not be repeated herein.

In a fourth aspect of the present disclosure, the present disclosure provides a first recombinant cell. According to an embodiment of the present disclosure, the first recombinant cell carries a first nucleic acid molecule described in the second aspect or expresses the antibody described in the first aspect. The first recombinant cells can be constructed by introducing the first expression vector into mammalian cells, and then the first recombinant cells can be used to express the antibodies provided by the present disclosure. The corresponding antibodies can be obtained by culturing the first recombinant cells. The available mammalian cells can be, for example, CHO cells and the like.

According to an embodiment of the present disclosure, the recombinant cell is obtained by introducing the first expression vector into a host cell.

According to an embodiment of the present disclosure, the recombinant cell is a eukaryotic cell.

According to an embodiment of the present disclosure, the recombinant cell is a mammalian cell.

In a fifth aspect of the present disclosure, the present disclosure provides use of the antibody described in the first aspect in the preparation of a fusion protein drug. As mentioned above, the antibody of the present disclosure has no antigen-binding activity, and thus it can be used as a neutral drug carrier to prepare a medicament for treating or preventing a disease. For example, the above antibody can be fused with an antigen epitope peptide to prepare a fusion protein. Since the above antibody has no antigen-binding activity, the prepared fusion protein can specifically bind to the antibody corresponding to the antigen epitope peptide without binding to other antibody, thereby avoiding the side effects of the non-specific binding of the fusion protein. Therefore, the fusion protein can be used to treat or prevent a disease caused by the antibody corresponding to the antigen epitope peptide.

According to an embodiment of the present disclosure, the medicament includes a fusion protein or an antibody-drug conjugate (ADC) drug.

In a sixth aspect of the present disclosure, the present disclosure provides use of the above-mentioned antibody as a drug carrier or a negative antibody. As mentioned above, the above-mentioned antibody of the present disclosure has no antigen-binding activity. Therefore, the antibody of the present disclosure can be used as a neutral drug carrier to prepare drugs or can directly serve as a negative antibody reference without antigen-binding activity.

According to an embodiment of the present disclosure, the drug carrier or the negative antibody has no antigen-binding ability.

Those skilled in the art can understand that the features and advantages described above in terms of the antibody are also applicable to the first nucleic acid molecule, the first expression vector, the first recombinant cell and uses thereof, which will not be repeated herein.

## FUSION PROTEIN

In a seventh aspect of the present disclosure, the present disclosure provides a fusion protein. According to an embodiment of the present disclosure, the fusion protein includes a neutral antibody and one or more PLA2R antigen epitope peptides. The neutral antibody includes an antibody FC segment or the antibody described in the first aspect. The PLA2R antigen epitope peptide is linked to the neutral antibody.

The inventors have found through experiments that the fusion protein obtained by fusing the PLA2R antigen epitope peptide with the above-mentioned neutral antibody can competitively bind to the PLA2R antibody, to prevent the PLA2R antibody from binding to the PLA2R in the body. Thus, the fusion protein can effectively treat a PLA2R antibody-related disease. Meanwhile, the PLA2R antigen epitope peptide in the fusion protein of the present disclosure can bind to the PLA2R antibody, and the neutral antibody in the fusion protein contains Fc fragment capable of binding to FcγR receptor of NK cells, thereby mediating NK cells to directly kill the B cells expressing membrane-type PLA2R antibody. Additionally, the Fc fragment can also bind to complement C1q and activate complement system, thereby exerting a lytic effect on the B cells expressing membrane-type PLA2R antibody. Since the fusion protein of the present disclosure specifically binds to PLA2R antibody and does not bind to other antibodies, the fusion protein can specifically eliminate B cells and avoid the side effects of nonspecific immunity. Moreover, the fusion protein of the present disclosure can also bind to liver FcRn receptor and get protection. In this way, the fusion protein can be prevented from being degraded and thus has a very long plasma half-life. Therefore, the fusion protein, as a drug, has the advantages of a long half-life, thereby reducing the frequency of administration, and the fusion protein further has the advantages such as high expression, high yield, high stability, high affinity, good solubility and druggability.

Those skilled in the art can understand that an amino acid fragment can be usually divided into an N-terminus and a C-terminus. The connection of neutral antibody and the PLA2R antigen epitope peptide is not strictly limited in the present disclosure, as long as the N-terminus of one fragment is linked to the C-terminus of the other fragment.

According to the embodiment of the present disclosure, the C-terminus of the PLA2R antigen epitope peptide is linked to the N-terminus of the neutral antibody.

According to an embodiment of the present disclosure, the PLA2R antigen epitope peptide includes CysR, FnII, and PLA2R binding region 1 and/or PLA2R binding region 2.

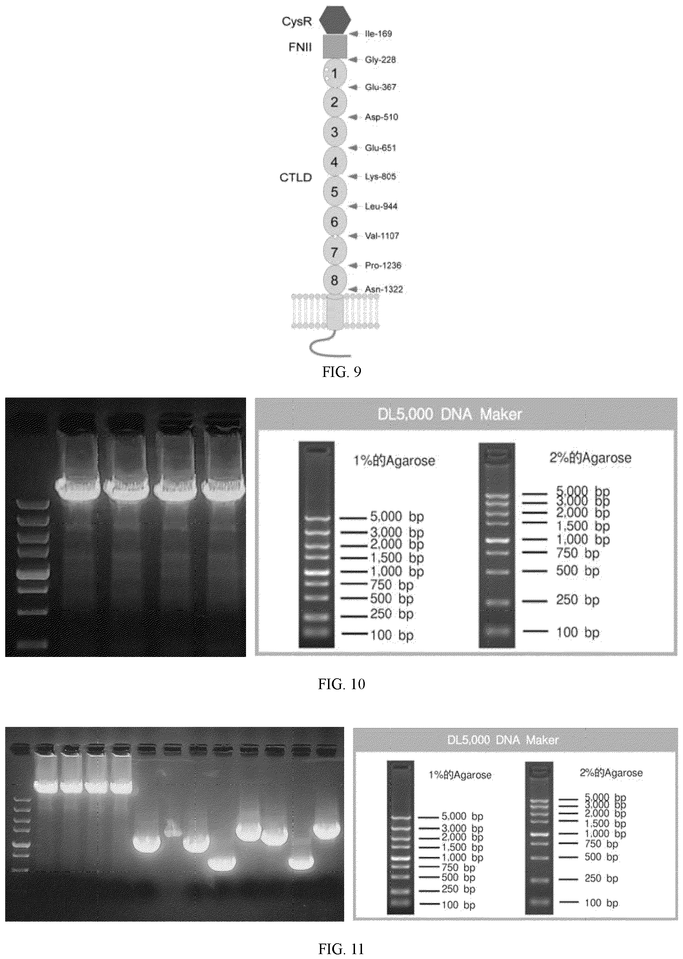

It should be noted that the specific structures of CysR, FnII, PLA2R binding region 1 and PLA2R binding region 2 are illustrated in FIG. 9.

According to the embodiment of the present disclosure, the C-terminus of the CysR is linked to the N-terminus of the FnII, and the C-terminus of the FnII is linked to the N-terminus of the PLA2R binding region 1.

According to the embodiment of the present disclosure, the C-terminus of the CysR is linked to the N-terminus of the FnII, the C-terminus of the FnII is linked to the N-terminus of the PLA2R binding region 1, and the C-terminus of the PLA2R binding region 1 is linked to the N-terminus of the PLA2R binding region 2.

According to an embodiment of the present disclosure, the PLA2R antigen epitope peptide has an amino acid sequence as set forth in SEQ ID NO: 21 or SEQ ID NO: 22 or an amino acid sequence having at least 95% similarity to SEQ ID NO: 21 or SEQ ID NO: 22.

EGVAAALTPERLLEWQDKGIFVIQSESLKKCIQAGKSVLTLENCKQANKHM

LWKWVSNHGLFNIGGSGCLGLNFSAPEQPLSLYECDSTLVSLRWRCNRKMITGPLQY

SVQVAHDNTVVASRKYIHKWISYGSGGGDICEYLHKDLHTIKGNTHGMPCMFPFQYN

HQWHHECTREGREDDLLWCATTSRYERDEKWGFCPDPTSAEVGCDTIWEKDLNSHI

CYQFNLLSSLSWSEAHSSCQMQGGTLLSITDETEENFIREHMSSKTVEVWMGLNQLD

EHAGWQWSDGTPLNYLNWSPEVNFEPFVEDHCGTFSSFMPSAWRSRDCESTLPYICK

KYLNHIDHEIVE (SEQ ID NO: 21).

EGVAAALTPERLLEWQDKGIFVIQSESLKKCIQAGKSVLTLENCKQANKHM

LWKWVSNHGLFNIGGSGCLGLNFSAPEQPLSLYECDSTLVSLRWRCNRKMITGPLQY

SVQVAHDNTVVASRKYIHKWISYGSGGGDICEYLHKDLHTIKGNTHGMPCMFPFQYN

HQWHHECTREGREDDLLWCATTSRYERDEKWGFCPDPTSAEVGCDTIWEKDLNSHI

CYQFNLLSSLSWSEAHSSCQMQGGTLLSITDETEENFIREHMSSKTVEVWMGLNQLD

EHAGWQWSDGTPLNYLNWSPEVNFEPFVEDHCGTFSSFMPSAWRSRDCESTLPYICK

KYLNHIDHEIVEKDAWKYYATHCEPGWNPYNRNCYKLQKEEKTWHEALRSCQADN

SALIDITSLAEVEFLVTLLGDENASETWIGLSSNKIPVSFEWSNDSSVIFTNWHTLEPHI

FPNRSQLCVSAEQSEGHWKVKNCEERLFYICKKAGHVLSD (SEQ ID NO: 22).

According to an embodiment of the present disclosure, the neutral antibody is an antibody FC segment, and the C-terminus of the PLA2R antigen epitope peptide is linked to the N-terminus of at least one antibody FC segment.

According to an embodiment of the present disclosure, the neutral antibody is the antibody described in the first aspect, and the C-terminus of the PLA2R antigen epitope peptide is linked to the N-terminus of at least one light chain or heavy chain of the antibody.

According to an embodiment of the present disclosure, the neutral antibody is a double-stranded antibody, and the N-terminus of each chain of the antibody FC segments is linked to the C-terminus of one PLA2R antigen epitope peptide. The fusion protein contains two PLA2R antigen epitope peptides, that is, a bivalent molecule. The inventors have found through experiments that, compared with monovalent natural PLA2R, the fusion protein of the present disclosure has a stronger binding ability to PLA2R antibody and thus can effectively neutralize PLA2R antibody, thereby protecting the natural PLA2R on the surface of glomerular cells from being attacked by the PLA2R antibody.

According to an embodiment of the present disclosure, the N-terminus of each light chain of the antibody described in the first aspect is linked to the C-terminus of one PLA2R antigen epitope peptide. The fusion protein contains two PLA2R antigen epitope peptides, that is, a bivalent molecule. The inventors have found through experiments that, compared with monovalent natural PLA2R, the fusion protein of the present disclosure has a stronger binding ability with PLA2R antibody and thus can effectively neutralize PLA2R antibody, thereby protecting the natural PLA2R on the surface of glomerular cells from being attacked by the PLA2R antibody.

According to the embodiment of the present disclosure, the N-terminus of each heavy chain of the antibody described in the first aspect is linked to the C-terminus of one PLA2R antigen epitope peptide, respectively. The fusion protein contains two PLA2R antigen epitope peptides, that is, a bivalent molecule. The inventors found through experiments that, compared with monovalent natural PLA2R, the fusion protein of the present disclosure has a stronger binding ability with PLA2R antibody and thus can effectively neutralize PLA2R antibody, thereby protecting the natural PLA2R on the surface of glomerular cells from being attacked by the PLA2R antibody.

According to the embodiment of the present disclosure, each of the N-terminus of each heavy chain and the N-terminus of each light chain of the antibody is linked to the C-terminuses of the two PLA2R antigen epitope peptide. The inventors have found through experiments that the fusion protein contains four PLA2R antigen epitope peptides. Compared with monovalent natural PLA2R, the fusion protein of the present disclosure has a stronger binding ability with PLA2R antibody and thus can effectively neutralize PLA2R antibody, thereby protecting the natural PLA2R on the surface of glomerular cells from being attacked by the PLA2R antibody.

According to an embodiment of the present disclosure, the fusion protein further includes a connecting peptide, and the connecting peptide is disposed between the PLA2R antigen epitope peptide and the antibody.

According to an embodiment of the present disclosure, the N-terminus of the connecting peptide is linked to the C-terminus of the PLA2R antigen epitope peptide, and the C-terminus of the connecting peptide is linked to the N-terminus of the neutral antibody. Therefore, by using the above-mentioned connecting peptide to fuse the PLA2R antigen epitope peptide and the neutral antibody, the obtained fusion protein can have improved binding ability to the PLA2R antibody.

According to an embodiment of the present disclosure, the amino acid sequence of the connecting peptide is $(GGGGS)_n$, where n is an integer greater than or equal to 1, and preferably 1, 2, 3, 4, 5, 6, 7, 8, 9, or 10.

According to an embodiment of the present disclosure, the connecting peptide includes an amino acid sequence as set forth in SEQ ID NO: 23.
GGGGSGGGGS (SEQ ID NO: 23).

According to an embodiment of the present disclosure, the antibody FC segment has an amino acid sequence as set forth in SEQ ID NO: 24 or an amino acid sequence having at least 90% similarity to SEQ ID NO: 24.

THTCPPCPAPELLGGPSVFLFPPKPKDTLMISRTPEVTCVVVDVSHEDPEVK

FNWYVDGVEVHNAKTKPREEQYNSTYRVVSVLTVLHQDWLNGKEYKCKVSNKALP

APIEKTISKAKGQPREPQVYTLPPSRDELTKNQVSLTCLVKGFYPSDIAVEWESNGQPE

NNYKTTPPVLDSDGSFFLYSKLTVDKSRWQQGNVFSCSVMHEALHNHYTQKSLSLSP

GK (SEQ ID NO: 24).

According to an embodiment of the present disclosure, the fusion protein has a first peptide segment with an amino acid sequence as set forth in SEQ ID NO: 57 and a second peptide segment with an amino acid sequence as set forth in SEQ ID NO: 19; or the fusion protein has a first peptide segment with an amino acid sequence as set forth in SEQ ID NO: 17 and a second peptide segment with an amino acid sequence as set forth in SEQ ID NO: 59; or an amino acid sequence of the fusion protein is as set forth in SEQ ID NO: 61; or the fusion protein has a first peptide segment with an amino acid sequence as set forth in SEQ ID NO: 63 and a second peptide segment with an amino acid sequence as set forth in SEQ ID NO: 19; or the fusion protein has a first peptide segment with an amino acid sequence as set forth in SEQ ID NO: 17 and a second peptide segment with an amino acid sequence as set forth in SEQ ID NO: 65; or an amino acid sequence of the fusion protein is as set forth in SEQ ID NO: 67; or the fusion protein has a first peptide segment with an amino acid sequence as set forth in SEQ ID NO: 57 and a second peptide segment with an amino acid sequence as set forth in SEQ ID NO: 59; or the fusion protein has a first peptide segment with an amino acid sequence as set forth in SEQ ID NO: 63 and a second peptide segment with an amino acid sequence as set forth in SEQ ID NO: 65.

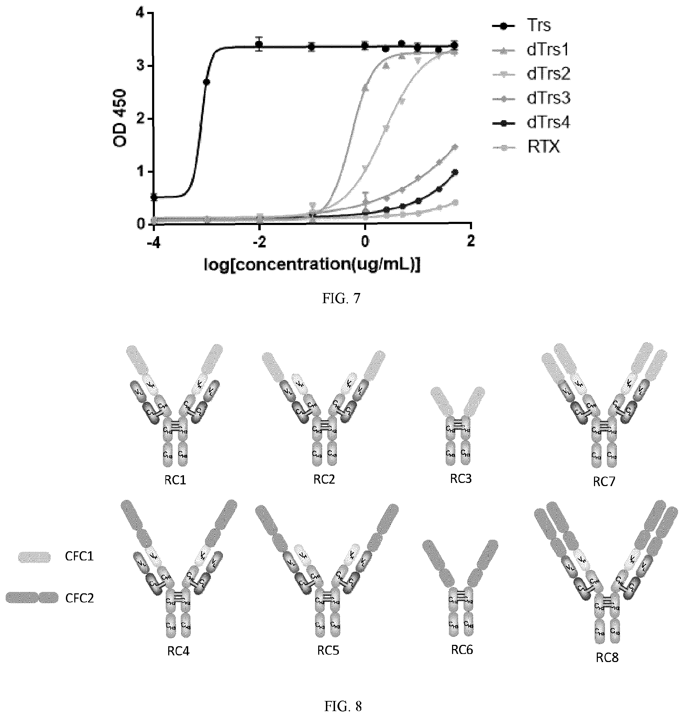

As an example, referring to FIG. 8, when the neutral antibody is the antibody described above, the first peptide segment of the fusion protein is a peptide segment including the heavy chain of the first antibody, and the second peptide segment of the fusion protein is a peptide segment including the light chain of the first antibody, and the first peptide segment and the second peptide segment are linked by an interchain disulfide bond; and when the neutral antibody is the antibody Fc fragment described above, the fusion protein is a peptide fragment including the antibody Fc fragment.

Those skilled in the art can understand that the features and advantages described above in terms of the antibody are also applicable to the fusion protein, which will not be repeated herein.

**SECOND NUCLEIC ACID MOLECULE, SECOND EXPRESSION VECTOR AND SECOND RECOMBINANT CELL**

In the process of preparing or obtaining these fusion proteins, the nucleic acid molecules expressing these fusion proteins can be used to link to different vectors and then expressed in different cells to obtain the corresponding fusion proteins.

To this end, in the eighth aspect of the present disclosure, the present disclosure provides a second nucleic acid molecule. According to an embodiment of the present disclosure, the nucleic acid molecule encodes the fusion protein described in the seventh aspect. The second nucleic acid molecule can be used to effectively express the fusion protein described above, especially in prokaryotic or lower eukaryotic expression systems. It should be noted that the second nucleic acid molecule herein is not required to encode the entire sequence of the above-mentioned fusion protein. For example, the second nucleic acid molecule can include two types of nucleic acid molecules each encoding two monomers, which can be further assembled *in vitro* to obtain a heterodimer, thereby forming a fusion protein containing a double-stranded antibody.

According to an embodiment of the present disclosure, the second nucleic acid molecule is DNA.

In a ninth aspect of the present disclosure, the present disclosure provides a second expression vector. According to an embodiment of the present disclosure, the second expression vector carries the second nucleic acid molecule described in the eighth aspect. The second expression vector can be used to effectively express the fusion protein, especially in prokaryotic or lower eukaryotic expression systems.

According to an embodiment of the present disclosure, the second expression vector is a eukaryotic expression vector, and preferably, the second expression vector is a lentivirus vector.

In the tenth aspect of the present disclosure, the present disclosure provides a second recombinant cell. According to an embodiment of the present disclosure, the second recombinant cell carries a second nucleic acid molecule described in the eighth aspect or expresses the fusion protein described in the seventh aspect. The recombinant cell according to the embodiment of the present disclosure can be used for *in vitro* expression and mass production of the fusion protein described in the seventh aspect.

According to an embodiment of the present disclosure, the second recombinant cell is a eukaryotic cell.

According to an embodiment of the present disclosure, the second recombinant cell is a mammalian cell.

Those skilled in the art can understand that the features and advantages described above in terms of the fusion protein are also applicable to the second nucleic acid molecule, the second expression vector and the second recombinant cell, which will not be repeated herein.

## PHARMACEUTICAL COMPOSITION, IMMUNOSORBENT, KIT, ADSORPTION PRODUCT AND USE THEREOF

In an eleventh aspect of the present disclosure, the present disclosure provides a pharmaceutical composition. According to an embodiment of the present disclosure, the pharmaceutical composition includes the fusion protein described in the seventh aspect. The inventors have found through a large number of experiments that the above-mentioned pharmaceutical composition can effectively prevent and treat a PLA2R antibody-related disease, for example, PLA2R antibody-positive membranous nephropathy.

According to the embodiment of the present disclosure, the pharmaceutical composition further includes a pharmaceutically acceptable excipient.

In a twelfth aspect of the present disclosure, the present disclosure provides an immunosorbent. According to an embodiment of the present disclosure, the immunosorbent includes the fusion protein described in the seventh aspect. The inventors have found through a large number of experiments that the immunosorbent can adsorb the PLA2R antibody, and can effectively treat a PLA2R antibody-related disease such as PLA2R antibody-positive membranous nephropathy through immunoadsorption.

In the thirteenth aspect of the present disclosure, the present disclosure provides a kit. According to an embodiment of the present disclosure, the kit includes the fusion protein described in the seventh aspect. The inventors have found through a large number of experiments that the kit can effectively detect the content of PLA2R antibody, and can also be used to diagnose or treat a PLA2R antibody-related disease, for example, PLA2R antibody-positive membranous nephropathy.

Those skilled in the art can understand that the features and advantages described above in terms of the fusion protein, the second nucleic acid molecule, the second expression vector and the second recombinant cell are also applicable to the pharmaceutical composition, the immunosorbent and the kit, which will not be repeated herein.

In the fourteenth aspect of the present disclosure, the present disclosure provides an adsorption product. According to an embodiment of the present disclosure, the adsorption product includes the immunosorbent described in the twelfth aspect and a solid phase carrier, and the immunosorbent is linked to the solid phase carrier. The adsorption product of the present disclosure can be used to adsorb the PLA2R antibody, for example, adsorb the PLA2R antibody in blood or plasma. By adsorbing the PLA2R antibody in blood or plasma, the adsorption product can be used to prevent or treat a PLA2R antibody-related disease.

It should be noted that the connection mode of the immunosorbent and the solid phase carrier is not particularly limited, and they can be coupled or connected by chemical bonds.

As used herein, the blood or plasma may be derived from any animal, for example, a mammal.

As an example, the blood or plasma is selected from human blood or plasma.

According to an embodiment of the present disclosure, the adsorption product is selected from an immunosorbent column or a blood purifier.

Those skilled in the art can understand that the features and advantages described above in terms of the immunosorbent are also applicable to this use, which will not be repeated herein.

In the fifteenth aspect of the present disclosure, the present disclosure provides use of the immunosorbent described in the twelfth aspect in the preparation of an adsorption product for adsorbing the PLA2R antibody.

According to an embodiment of the present disclosure, the adsorption product is selected from an immunosorbent column or a blood purifier.

According to an embodiment of the present disclosure, the adsorption product is an immunosorbent column, and the adsorption product is used to prevent or treat a PLA2R antibody-related disease. As mentioned above, the immunosorbent described above can adsorb the PLA2R antibody. Thus, the immunosorbent column containing the immunosorbent described above can adsorb the PLA2R antibody through the immunosorbent, and the immunosorbent column can effectively treat a PLA2R antibody-related disease.

According to the embodiment of the present disclosure, the PLA2R antibody-related disease is selected from PLA2R antibody-positive membranous nephropathy.

According to the embodiment of the present disclosure, the adsorption product is a blood purifier, and the adsorption product is used to adsorb the PLA2R antibody in blood or plasma. As mentioned above, the immunosorbent described above can adsorb the PLA2R antibody. Thus, the blood purifier containing the immunosorbent described above can adsorb the PLA2R antibody through the immunosorbent, and the blood purifier can effectively adsorb the PLA2R antibody in blood or plasma.

Those skilled in the art can understand that the features and advantages described above in terms of immunosorbents are also applicable to the use, which will not be repeated herein.

In the sixteenth aspect of the present disclosure, the present disclosure provides use of the fusion protein described in the seventh aspect, the second nucleic acid molecule described in the eighth aspect, the second expression vector described in the ninth aspect, the second recombinant cell described in the tenth aspect, or the pharmaceutical composition described in the eleventh aspect in the preparation of a medicament for preventing or treating a PLA2R antibody-related disease.

According to the embodiment of the present disclosure, the PLA2R antibody-related disease is selected from PLA2R antibody-positive membranous nephropathy.

According to an embodiment of the present disclosure, a formulation of the pharmaceutical composition includes an injection.

According to an embodiment of the present disclosure, an administration route of the pharmaceutical composition includes subcutaneous injection or intravenous injection.

Those skilled in the art can understand that the features and advantages described above in terms of the pharmaceutical composition, the immunosorbent, and the kit, etc., are also applicable to the use, which will not be repeated herein.

In the seventeenth aspect of the present disclosure, the present disclosure provides use of the fusion protein described in the seventh aspect, the second nucleic acid molecule described in the eighth aspect, the second expression vector described in the ninth aspect, the second recombinant cell described in the tenth aspect, or the pharmaceutical composition described in the eleventh aspect in the preparation of an immunosorbent for adsorbing the PLA2R antibody.

Those skilled in the art can understand that the features and advantages described above in terms of the pharmaceutical composition, the immunosorbent, and the kit, etc., are also applicable to the use, which will not be repeated herein.

In the eighteenth aspect of the present disclosure, the present disclosure provides use of the fusion protein described in the seventh aspect, the second nucleic acid molecule described in the eighth aspect, the second expression vector described in the ninth aspect, the second recombinant cell described in the tenth aspect, the pharmaceutical composition described in the eleventh aspect, or the immunosorbent described in the twelfth aspect in the preparation of a kit for detecting the PLA2R antibody.

Those skilled in the art can understand that the features and advantages described above in terms of the fusion protein, the pharmaceutical composition, the immunosorbent, and the kit, etc., are also applicable to the use described above, which will not be repeated herein.

**METHODS**

In the nineteenth aspect of the present disclosure, the present disclosure provides a method for preventing or treating a PLA2R antibody-related disease. According to an embodiment of the present disclosure, the method includes: administering a pharmaceutically acceptable amount of the fusion protein described in the seventh aspect or the pharmaceutical composition described in the eleventh aspect to a subject. Therefore, the PLA2R antibody-related disease can be effectively treated.

According to the embodiment of the present disclosure, an administration route is subcutaneous injection or intravenous injection.

According to the embodiment of the present disclosure, the PLA2R antibody-related disease is selected from PLA2R antibody-positive membranous nephropathy.

In the twentieth aspect of the present disclosure, the present disclosure provides an immunoadsorption therapy method for preventing or treating a PLA2R antibody-related disease. According to an embodiment of the present disclosure, the method includes treating a subject with the immunosorbent described in the twelfth aspect.

According to the embodiment of the present disclosure, the subject is treated with the extracorporeal blood purification in the method.

According to the embodiment of the present disclosure, the PLA2R antibody-related disease is selected from PLA2R antibody-positive membranous nephropathy.

In the twenty-first aspect of the present disclosure, the present disclosure provides a method for diagnosing a PLA2R antibody-related disease. According to the embodiment of the present disclosure, the method includes: detecting the PLA2R antibody in a sample to be tested by using the fusion protein described in the seventh aspect, the second nucleic acid molecule described in the eighth aspect, the second expression vector described in the ninth aspect, the second recombinant cell described in the tenth aspect, the pharmaceutical composition described in the eleventh aspect, the immunosorbent described in the twelfth aspect, or the kit described in the thirteenth aspect; and determining a content of PLA2R antibody in the sample to be tested based on a detection result. According to the embodiment of the present disclosure, the content of PLA2R antibody in the sample to be tested being not less than 14 RU/mL is an indication that the sample to be tested is derived from a patient suffering from the PLA2R antibody-related disease, and preferably, the content of PLA2R antibody in the sample to be tested being not less than 20 RU/ mL is an indication that the sample to be tested is derived from a patient suffering from the PLA2R antibody-related disease.

According to an embodiment of the present disclosure, the sample to be tested is blood.

According to the embodiment of the present disclosure, the PLA2R antibody-related disease is selected from PLA2R antibody-positive membranous nephropathy.

In the twenty-second aspect of the present disclosure, the present disclosure provides a method for evaluating a stage of a PLA2R antibody-related disease. According to an embodiment of the present disclosure, the method includes: detecting the PLA2R antibody in a sample to be detected by using the fusion protein described in the seventh aspect, the second nucleic acid molecule described in the eighth aspect, the second expression vector described in the ninth aspect, the second recombinant cell described in the tenth aspect, the pharmaceutical composition described in the eleventh aspect, the immunosorbent described in the twelfth aspect, or the kit described in the thirteenth aspect; and determining a content of PLA2R antibody in the sample to be tested based on a detection result.

According to the embodiment of the present disclosure, the stage of the PLA2R antibody-related disease in the patient to be tested is determined based on the content of PLA2R antibody in the sample to be tested.

According to the embodiment of the present disclosure, the PLA2R antibody-related disease is selected from PLA2R antibody-positive membranous nephropathy.

According to the embodiment of the present disclosure, the PLA2R antibody-positive membranous nephropathy is divided into four stages.

According to an embodiment of the present disclosure, the sample to be tested is blood.

In the twenty-third aspect of the present disclosure, the present disclosure provides a method for evaluating a prognosis of a PLA2R antibody-related disease. According to the embodiment of the present disclosure, the method includes: detecting the PLA2R antibody in a sample to be detected by using the fusion protein described in the seventh aspect, the second nucleic acid molecule described in the eighth aspect, the second expression vector described in the ninth aspect, the second recombinant cell described in the tenth aspect, the pharmaceutical composition described in the eleventh aspect, the immunosorbent described in the twelfth aspect, or the kit described in the thirteenth aspect; and determining a content of PLA2R antibody in the sample to be tested based on a detection result.

According to the embodiment of the present disclosure, the sample to be tested is derived from a patient suffering from the PLA2R antibody-related disease before or after treatment.

According to the embodiment of the present disclosure, a prognosis effect on the PLA2R antibody-related disease is determined based on the content of PLA2R antibody in the sample to be tested of the patients suffering from the PLA2R antibody-related disease before or after treatment.

According to the embodiment of the present disclosure, a decrease in the content of PLA2R antibody in the sample to be tested of the patient suffering from the PLA2R antibody-related disease after treatment is an indication of a good prognosis of the patient.

According to an embodiment of the present disclosure, the sample to be tested is blood.

According to the embodiment of the present disclosure, the PLA2R antibody-related disease is selected from PLA2R antibody-positive membranous nephropathy.

Those skilled in the art can understand that the features and advantages described above in terms of the fusion protein, the pharmaceutical composition, the immunosorbent and the kit are also applicable to the methods described above, which will not be repeated herein.

The solutions of the present disclosure will be explained with examples. Those skilled in the art will understand that the following examples are only used to illustrate the present disclosure and should not be regarded as limitations of the scope of the present disclosure. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

**Example 1: Preparation and Selection of Neutral Antibodies**

1. Expression construction

Firstly, the plasmid vector used was a pcDNA3.4 transient expression vector, which contains a native full-length CMV promoter and a WPRE element downstream of the cloning sites. The expression level using the vector based on CMV promoter is usually good for the transient expression system of the CHO cells. The WPRE element is located downstream of the multiple cloning sites and can effectively improve the transcription and expression of genes. In *Escherichia coli,* it has an ampicillin resistance gene. Then, the vector gene pcDNA3.4 and the target gene were amplified by PCR technology, and the PCR template was obtained by gene synthesis. The vector fragment and the target gene fragment were connected by using a homologous recombination kit. A single clone was obtained by transformation in DH5α competent cells. The single clone was amplified and sequenced, and after sequencing confirmation, the plasmid was extracted to obtain the target gene recombinant expression plasmid, in which the coding DNA of the target sequence together with the coding DNA expressing the secretory signal peptide were synthesized and cloned between the CMV promoter and the WPRE gene of the plasmid pcDNA3.4, as illustrated in FIG. 1 for details.

1.1 The names and sequences of the target genes are shown in Table 1, and the primers and templates of pcDNA3.4 vector and target genes (or target fragments) are shown in Table 2, where RtxH and RtxL are used to synthesize Rituximab (Rtx for short).

[Table 1] Names and sequences of target genes

| Name | Primer | Amino acid sequence | Nucleotide sequence |
|---|---|---|---|
| RtxH | RtxH-f, RtxH-r | SEQ ID NO: 29 | SEQ ID NO: 36 |
| RtxL | RtxL-f, RtxL-r | SEQ ID NO: 30 | SEQ ID NO: 37 |
| TrsH | TrsH-f, TrsH-r | SEQ ID NO: 31 | SEQ ID NO: 38 |
| TrsL | TrsL-f, TrsL-r | SEQ ID NO: 32 | SEQ ID NO: 39 |
| dTrs1H | dTrsH-f, dTrsH-r | SEQ ID NO: 33 | SEQ ID NO: 40 |
| dTrs1L(dTrs2L) | dTrsL-f, dTrsL-r | SEQ ID NO: 34 | SEQ ID NO: 41 |
| dTrs2H | dTrsH-f, dTrsH-r | SEQ ID NO: 35 | SEQ ID NO: 42 |
| dTrs3H | dTrsH-f, dTrsH-r | SEQ ID NO: 18 | SEQ ID NO: 27 |
| dTrs3L | dTrsL-f, dTrsL-r | SEQ ID NO: 20 | SEQ ID NO: 28 |
| dTrs4H (dTrsH) | dTrsH-f, dTrsH-r | SEQ ID NO: 17 | SEQ ID NO: 25 |
| dTrs4L(dTrsL) | dTrsL-f, dTrsL-r | SEQ ID NO: 19 | SEQ ID NO: 26 |
| Note: dTrs1L and dTrs2L in this example are the same target genes. | | | |

The amino acid sequence of RtxH:

QVQLQQPGAELVKPGASVKMSCKASGYTFTSYNMHWVKQTPGRGLEWIGAIYPGN

GDTSYNQKFKGKATLTADKSSSTAYMQLSSLTSEDSAVYYCARSTYYGGDWYFNVW

GAGTTVTVSAASTKGPSVFPLAPSSKSTSGGTAALGCLVKDYFPEPVTVSWNSGALTS

GVHTFPAVLQSSGLYSLSSVVTVPSSSLGTQTYICNVNHKPSNTKVDKKAEPKSCDKT

HTCPPCPAPELLGGPSVFLFPPKPKDTLMISRTPEVTCVVVDVSHEDPEVKFNWYVDG

VEVHNAKTKPREEQYNSTYRVVSVLTVLHQDWLNGKEYKCKVSNKALPAPIEKTISK

AKGQPREPQVYTLPPSRDELTKNQVSLTCLVKGFYPSDIAVEWESNGQPENNYKTTPP

VLDSDGSFFLYSKLTVDKSRWQQGNVFSCSVMHEALHNHYTQKSLSLSPG  (SEQ  ID

NO: 29).

The amino acid sequence of RtxL:

QIVLSQSPAILSASPGEKVTMTCRASSSVSYIHWFQQKPGSSPKPWIYATSNLASGVPV

RFSGSGSGTSYSLTISRVEAEDAATYYCQQWTSNPPTFGGGTKLEIKRTVAAPSVFIFPP

SDEQLKSGTASVVCLLNNFYPREAKVQWKVDNALQSGNSQESVTEQDSKDSTYSLSS

TLTLSKADYEKHKVYACEVTHQGLSSPVTKSFNRGEC (SEQ ID NO: 30).

The amino acid sequence of TrsH:

EVQLVESGGGLVQPGGSLRLSCAASGFNIKDTYIHWVRQAPGKGLEWVARIYPTNGY

TRYADSVKGRFTISADTSKNTAYLQMNSLRAEDTAVYYCSRWGGDGFYAMDYWGQG

TLVTVSSASTKGPSVFPLAPSSKSTSGGTAALGCLVKDYFPEPVTVSWNSGALTSGVH

TFPAVLQSSGLYSLSSVVTVPSSSLGTQTYICNVNHKPSNTKVDKKVEPKSCDKTHTCP

PCPAPELLGGPSVFLFPPKPKDTLMISRTPEVTCVVVDVSHEDPEVKFNWYVDGVEVH

NAKTKPREEQYNSTYRVVSVLTVLHQDWLNGKEYKCKVSNKALPAPIEKTISKAKGQ

PREPQVYTLPPSRDELTKNQVSLTCLVKGFYPSDIAVEWESNGQPENNYKTTPPVLDS

DGSFFLYSKLTVDKSRWQQGNVFSCSVMHEALHNHYTQKSLSLSPGK (SEQ  ID  NO:

31).

The amino acid sequence of TrsL:

DIQMTQSPSSLSASVGDRVTITCRASQDVNTAVAWYQQKPGKAPKLLIYSASFLYSGV PSRFSGSRSGTDFTLTISSLQPEDFATYYCQQHYTTPPTFGQGTKVEIKRTVAAPSVFIFP PSDEQLKSGTASVVCLLNNFYPREAKVQWKVDNALQSGNSQESVTEQDSKDSTYSLS STLTLSKADYEKHKVYACEVTHQGLSSPVTKSFNRGEC (SEQ ID NO: 32).

The amino acid sequence of dTrs1H:

EVQLVESGGGLVQPGGSLRLSCAASGFNIKDTYIHWVRQAPGKGLEWVARIYPTNGG TRYADSVKGRFTISADTSKNTAYLQMNSLRAEDTAVYYCSRWGGDGFGAMDYWGQ GTLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK (SEQ ID NO: 33).

The amino acid sequence of dTrs1L:

DIQMTQSPSSLSASVGDRVTITCRASQDVGTAVAWYQQKPGKAPKLLIYSASGLYSGV PSRFSGSRSGTDFTLTISSLQPEDFATYYCQQHGTTPPTFGQGTKVEIKRTVAAPSVFIFP PSDEQLKSGTASVVCLLNNFYPREAKVQWKVDNALQSGNSQESVTEQDSKDSTYSLS STLTLSKADYEKHKVYACEVTHQGLSSPVTKSFNRGEC (SEQ ID NO: 34).

The amino acid sequence of dTrs2H:

EVQLVESGGGLVQPGGSLRLSCAASGFNIKDTGIHWVRQAPGKGLEWVAGIYPTNGG

TGYADSVKGRFTISADTSKNTAYLQMNSLRAEDTAVYYCSRGGGDGFGAMDYWGQ

GTLVTVSSASTKGPSVFPLAPSSKSTSGGTAALGCLVKDYFPEPVTVSWNSGALTSGV

HTFPAVLQSSGLYSLSSVVTVPSSSLGTQTYICNVNHKPSNTKVDKKVEPKSCDKTHT

CPPCPAPELLGGPSVFLFPPKPKDTLMISRTPEVTCVVVDVSHEDPEVKFNWYVDGVE

VHNAKTKPREEQYNSTYRVVSVLTVLHQDWLNGKEYKCKVSNKALPAPIEKTISKAK

GQPREPQVYTLPPSRDELTKNQVSLTCLVKGFYPSDIAVEWESNGQPENNYKTTPPVL

DSDGSFFLYSKLTVDKSRWQQGNVFSCSVMHEALHNHYTQKSLSLSPGK (SEQ ID NO: 35).

The nucleotide sequence of RtxH:

CAAGTGCAGCTCCAGCAGCCCGGGGCTGAACTGGTCAAGCCTGGAGCCTCAGTG

AAGATGTCCTGCAAAGCCTCCGGCTACACCTTTACATCTTACAACATGCACTGGGT

CAAGCAGACCCCAGGACGGGGCTTGGAATGGATTGGTGCCATCTACCCGGGAAAC

GGCGACACTAGCTATAACCAGAAGTTCAAGGGCAAAGCAACCCTCACCGCCGATA

AGAGCAGCAGCACTGCGTACATGCAGCTGTCGTCCCTGACGTCCGAGGATTCCGC

CGTGTATTACTGCGCAAGGTCAACCTACTATGGAGGAGATTGGTACTTCAACGTCT

GGGGAGCCGGGACCACTGTGACTGTGTCCGCCGCGTCAACTAAGGGCCCTTCAGT

GTTCCCCCTCGCGCCAAGCTCCAAGTCCACTAGCGGCGGGACCGCCGCGCTGGGT

TGCCTGGTCAAGGACTACTTCCCCGAGCCCGTGACCGTGTCCTGGAACTCCGGCG

CGCTGACCTCCGGGGTGCATACTTTCCCGGCTGTGCTTCAATCGAGCGGACTGTAC

TCCCTGAGCTCCGTGGTCACGGTGCCGTCCTCGAGCCTGGGAACCCAGACCTACA

TTTGCAACGTGAACCACAAGCCCAGCAACACCAAGGTCGACAAGAAGGCCGAGC

CTAAGTCCTGTGACAAGACCCACACTTGCCCTCCGTGCCCCGCCCCTGAGCTGCT

GGGAGGGCCGTCTGTGTTTCTTCCCGCCCAAGCCTAAGGACACTCTCATGATTT

CCCGCACCCCGAAGTCACTTGCGTGGTGGTGGATGTGTCGCATGAAGATCCAGA

AGTGAAATTCAATTGGTACGTGGATGGAGTGGAAGTGCACAACGCCAAGACCAAA

CCGCGCGAGGAGCAGTACAATTCAACCTACCGGGTGGTGTCGGTGTTGACTGTGC

TGCACCAGGACTGGCTGAACGGAAAGGAGTACAAGTGCAAAGTGTCGAACAAGG

CCCTCCCTGCTCCGATCGAAAAGACCATCAGCAAGGCCAAGGGACAGCCGAGGG

AGCCCCAGGTCTACACTCTGCCTCCGTCCCGCGACGAGCTGACCAAGAACCAAGT

GTCCCTCACCTGTCTTGTGAAGGGGTTCTACCCAAGCGACATCGCGGTCGAATGG

GAATCAAATGGCCAGCCTGAGAACAACTACAAGACAACGCCCCGGTGCTGGACT

CGGACGGCTCGTTCTTCCTGTACTCCAAGCTGACTGTGGACAAGTCCCGGTGGCA

ACAGGGAAACGTGTTCAGCTGTAGCGTGATGCATGAAGCCCTGCACAACCACTAC

ACCCAGAAGTCGCTCTCCCTGTCACCCGGC (SEQ ID NO: 36).

The nucleotide sequence of RtxL:

CAGATCGTGTTGTCGCAATCCCCGGCCATCCTGTCCGCTTCGCCGGGAGAAAAGGT

CACCATGACTTGTCGCGCCTCAAGCAGCGTGTCCTACATTCATTGGTTTCAGCAGA

AGCCCGGGTCCAGCCCGAAGCCTTGGATCTACGCAACTAGCAACCTCGCCTCCGG

CGTGCCAGTGCGCTTCTCCGGTTCCGGATCGGGAACGTCCTATAGCCTGACTATCT

CGAGAGTGGAGGCCGAGGACGCTGCCACTTACTACTGCCAGCAGTGGACCTCGAA

TCCTCCAACTTTCGGTGGCGGCACCAAGCTGGAGATTAAGAGGACCGTCGCGGCC

CCTAGCGTGTTCATTTTCCCCCCGTCCGACGAGCAGCTGAAGTCCGGGACAGCCTC

AGTGGTCTGCCTGCTCAACAACTTCTACCCCGGGAAGCCAAAGTGCAGTGGAAA

GTGGATAACGCGCTTCAGAGCGGCAACTCACAAGAGTCCGTGACCGAACAGGAC

TCGAAGGACAGCACCTACAGCCTCTCCTCCACCCTGACCCTGTCAAAGGCGGATT

ACGAAAAGCACAAGGTCTACGCCTGCGAAGTGACCCACCAGGGGCTGTCCTCTCC

CGTGACCAAGTCCTTCAACCGGGGAGAGTGC (SEQ ID NO: 37).

The nucleotide sequence of TrsH:

GAAGTCCAGCTGGTGGAAAGTGGCGGTGGACTGGTTCAACCAGGAGGAAGCCTA
AGACTAAGCTGCGCCGCCAGCGGTTTTAACATCAAGGACACATACATCCATTGGGT
CCGGCAGGCACCCGGCAAAGGTTTAGAGTGGGTCGCAAGGATCTACCCCACTAAT
GGTTATACCAGGTACGCTGACAGCGTGAAGGGCAGGTTCACCATTAGCGCTGACA
CCAGCAAGAACACTGCTTATCTGCAGATGAATAGCCTGCGTGCCGAAGACACAGC
CGTCTATTACTGTAGTAGATGGGGAGGTGATGGGTTCTATGCGATGGATTACTGGGG
ACAGGGCACACTCGTGACCGTTAGTAGCGCTAGCACCAAGGGCCCATCGGTCTTC
CCCCTGGCACCCTCCTCCAAGAGCACCTCTGGGGGCACAGCGGCCCTGGGCTGCC
TGGTCAAGGACTACTTCCCCGAACCGGTGACGGTGTCGTGGAACTCAGGCGCCCT
GACCAGCGGCGTGCACACCTTCCCGGCTGTCCTACAGTCCTCAGGACTCTACTCCC
TCAGCAGCGTGGTGACCGTGCCCTCCAGCAGCTTGGGCACCCAGACCTACATCTG
CAACGTGAATCACAAGCCCAGCAACACCAAGGTGGACAAGAAAGTAGAGCCCAA
ATCTTGTGACAAAACTCACACATGCCCACCGTGCCCAGCACCTGAACTCCTGGGG
GGACCGTCAGTCTTCCTCTTCCCCCCAAAACCCAAGGACACCCTCATGATCTCCCG
GACCCCTGAGGTCACATGCGTGGTGGTGGACGTGAGCCACGAAGACCCTGAGGTC
AAGTTCAACTGGTACGTGGACGGCGTGGAGGTGCATAATGCCAAGACAAAGCCGC
GGGAGGAGCAGTACAACAGCACGTACCGTGTGGTCAGCGTCCTCACCGTCCTGCA
CCAGGACTGGCTGAATGGCAAGGAGTACAAGTGCAAGGTCTCCAACAAAGCCCT
CCCAGCCCCCATCGAGAAAACCATCTCCAAAGCCAAAGGGCAGCCCCGAGAACC
ACAGGTGTACACCCTGCCCCCATCCCGGGATGAGCTGACCAAGAACCAGGTCAGC
CTGACCTGCCTGGTCAAAGGCTTCTATCCCAGCGACATCGCCGTGGAGTGGGAGA
GCAATGGGCAGCCGGAGAACAACTACAAGACCACGCCTCCCGTGCTGGACTCCG
ACGGCTCCTTCTTCCTCTACAGCAAGCTCACCGTGGACAAGAGCAGGTGGCAGCA
GGGGAACGTCTTCTCATGCTCCGTGATGCATGAGGCTCTGCACAACCACTACACGC
AGAAGAGCCTCTCCCTGTCTCCGGGTAAA (SEQ ID NO: 38).

The nucleotide sequence of TrsL:

GATATACAGATGACGCAGAGCCCTAGCAGTCTAAGTGCAAGCGTTGGAGACCGTG

TCACCATTACCTGCCGCGCTAGCCAGGATGTCAACACCGCAGTCGCCTGGTATCAG

CAAAAACCGGGTAAGGCTCCAAAGCTGTTGATCTACAGTGCCAGCTTTCTGTACA

GCGGTGTGCCAAGTAGGTTCAGCGGGAGCCGGAGTGGGACAGACTTTACACTCAC

TATTAGCAGTCTGCAGCCCGAGGACTTCGCCACCTACTACTGTCAGCAGCATTATA

CAACACCCCAACATTCGGGCAGGGAACCAAAGTTGAGATAAAACGAACGGTTG

CCGCGCCAAGCGTATTTATCTTTCCCCCCAGTGATGAGCAACTGAAGAGTGGTACA

GCCAGCGTTGTCTGCCTTTTGAATAACTTCTATCCTCGGGAGGCAAAGGTCCAGTG

GAAAGTGGATAATGCACTCCAAAGCGGTAACAGCCAGGAGAGTGTGACAGAACA

GGACAGCAAAGATAGTACCTACAGCCTCAGTAGTACCCTCACACTGAGCAAAGCG

GACTATGAAAAGCACAAGGTGTACGCTTGTGAAGTGACCCACCAGGGCCTAAGCA

GCCCTGTTACTAAGAGCTTCAACAGAGGCGAATGT (SEQ ID NO: 39).

The nucleotide sequence of dTrs1H:

GAAGTCCAGCTGGTGGAAAGTGGCGGTGGACTGGTTCAACCAGGAGGAAGCCTA

AGACTAAGCTGCGCCGCCAGCGGTTTTAACATCAAGGACACATACATCCATTGGGT

CCGGCAGGCACCCGGCAAAGGTTTAGAGTGGGTCGCAAGGATCTACCCCACTAAT

GGTGGCACCAGGTACGCTGACAGCGTGAAGGGCAGGTTCACCATTAGCGCTGACA

CCAGCAAGAACACTGCTTATCTGCAGATGAATAGCCTGCGTGCCGAAGACACAGC

CGTCTATTACTGTAGTAGATGGGGAGGTGATGGGTTCGGCGCGATGGATTACTGGG

GACAGGGCACACTCGTGACCGTTAGTAGCGCTAGCACCAAGGGCCCATCGGTCTT

CCCCCTGGCACCCTCCTCCAAGAGCACCTCTGGGGGCACAGCGGCCCTGGGCTGC

CTGGTCAAGGACTACTTCCCCGAACCGGTGACGGTGTCGTGGAACTCAGGCGCCC

TGACCAGCGGCGTGCACACCTTCCCGGCTGTCCTACAGTCCTCAGGACTCTACTCC

CTCAGCAGCGTGGTGACCGTGCCCTCCAGCAGCTTGGGCACCCAGACCTACATCT

GCAACGTGAATCACAAGCCCAGCAACACCAAGGTGGACAAGAAAGTAGAGCCCA

AATCTTGTGACAAAACTCACACATGCCCACCGTGCCCAGCACCTGAACTCCTGGG

GGGACCGTCAGTCTTCCTCTTCCCCCCAAAACCCAAGGACACCCTCATGATCTCCC

GGACCCCTGAGGTCACATGCGTGGTGGTGGACGTGAGCCACGAAGACCCTGAGG

TCAAGTTCAACTGGTACGTGGACGGCGTGGAGGTGCATAATGCCAAGACAAAGCC

GCGGGAGGAGCAGTACAACAGCACGTACCGTGTGGTCAGCGTCCTCACCGTCCTG

CACCAGGACTGGCTGAATGGCAAGGAGTACAAGTGCAAGGTCTCCAACAAAGCC

CTCCCAGCCCCCATCGAGAAAACCATCTCCAAAGCCAAAGGGCAGCCCCGAGAAC

CACAGGTGTACACCCTGCCCCCATCCCGGGATGAGCTGACCAAGAACCAGGTCAG

CCTGACCTGCCTGGTCAAAGGCTTCTATCCCAGCGACATCGCCGTGGAGTGGGAG

AGCAATGGGCAGCCGGAGAACAACTACAAGACCACGCCTCCCGTGCTGGACTCC

GACGGCTCCTTCTTCCTCTACAGCAAGCTCACCGTGGACAAGAGCAGGTGGCAGC

AGGGGAACGTCTTCTCATGCTCCGTGATGCATGAGGCTCTGCACAACCACTACACG

CAGAAGAGCCTCTCCCTGTCTCCGGGTAAA (SEQ ID NO: 40).

The nucleotide sequence of dTrs1L (dTrs2L):

GATATACAGATGACGCAGAGCCCTAGCAGTCTAAGTGCAAGCGTTGGAGACCGTG

TCACCATTACCTGCCGCGCTAGCCAGGATGTCGGCACCGCAGTCGCCTGGTATCAG

CAAAAACCGGGTAAGGCTCCAAAGCTGTTGATCTACAGTGCCAGCGGCCTGTACA

GCGGTGTGCCAAGTAGGTTCAGCGGGAGCCGGAGTGGGACAGACTTTACACTCAC

TATTAGCAGTCTGCAGCCCGAGGACTTCGCCACCTACTACTGTCAGCAGCATGGCA

CAACACCCCAACATTCGGGCAGGGAACCAAAGTTGAGATAAAACGAACGGTTG

CCGCGCCAAGCGTATTTATCTTTCCCCCCAGTGATGAGCAACTGAAGAGTGGTACA

GCCAGCGTTGTCTGCCTTTTGAATAACTTCTATCCTCGGGAGGCAAAGGTCCAGTG

GAAAGTGGATAATGCACTCCAAAGCGGTAACAGCCAGGAGAGTGTGACAGAACA

GGACAGCAAAGATAGTACCTACAGCCTCAGTAGTACCCTCACACTGAGCAAAGCG

GACTATGAAAAGCACAAGGTGTACGCTTGTGAAGTGACCCACCAGGGCCTAAGCA

GCCCTGTTACTAAGAGCTTCAACAGAGGCGAATGT (SEQ ID NO: 41).

The nucleotide sequence of dTrs2H:

GAAGTCCAGCTGGTGGAAAGTGGCGGTGGACTGGTTCAACCAGGAGGAAGCCTA

AGACTAAGCTGCGCCGCCAGCGGTTTTAACATCAAGGACACAGGCATCCATTGGG

TCCGGCAGGCACCCGGCAAAGGTTTAGAGTGGGTCGCAGGCATCTACCCCACTAA

TGGTGGCACCGGCTACGCTGACAGCGTGAAGGGCAGGTTCACCATTAGCGCTGAC

ACCAGCAAGAACACTGCTTATCTGCAGATGAATAGCCTGCGTGCCGAAGACACAG

CCGTCTATTACTGTAGTAGAGGCGGAGGTGATGGGTTCGGCGCGATGGATTACTGG

GGACAGGGCACACTCGTGACCGTTAGTAGCGCTAGCACCAAGGGCCCATCGGTCT

TCCCCCTGGCACCCTCCTCCAAGAGCACCTCTGGGGGCACAGCGGCCCTGGGCTG

CCTGGTCAAGGACTACTTCCCCGAACCGGTGACGGTGTCGTGGAACTCAGGCGCC

CTGACCAGCGGCGTGCACACCTTCCCGGCTGTCCTACAGTCCTCAGGACTCTACTC

CCTCAGCAGCGTGGTGACCGTGCCCTCCAGCAGCTTGGGCACCCAGACCTACATC

TGCAACGTGAATCACAAGCCCAGCAACACCAAGGTGGACAAGAAAGTAGAGCCC

AAATCTTGTGACAAAACTCACACATGCCCACCGTGCCCAGCACCTGAACTCCTGG

GGGGACCGTCAGTCTTCCTCTTCCCCCCAAAACCCAAGGACACCCTCATGATCTCC

CGGACCCCTGAGGTCACATGCGTGGTGGTGGACGTGAGCCACGAAGACCCTGAG

GTCAAGTTCAACTGGTACGTGGACGGCGTGGAGGTGCATAATGCCAAGACAAAGC

CGCGGGAGGAGCAGTACAACAGCACGTACCGTGTGGTCAGCGTCCTCACCGTCCT

GCACCAGGACTGGCTGAATGGCAAGGAGTACAAGTGCAAGGTCTCCAACAAAGC

CCTCCCAGCCCCCATCGAGAAACCATCTCCAAAGCCAAAGGGCAGCCCCGAGAA

CCACAGGTGTACACCCTGCCCCCATCCCGGGATGAGCTGACCAAGAACCAGGTCA

GCCTGACCTGCCTGGTCAAAGGCTTCTATCCCAGCGACATCGCCGTGGAGTGGGA

GAGCAATGGGCAGCCGGAGAACAACTACAAGACCACGCCTCCCGTGCTGGACTC

CGACGGCTCCTTCTTCCTCTACAGCAAGCTCACCGTGGACAAGAGCAGGTGGCAG

CAGGGGAACGTCTTCTCATGCTCCGTGATGCATGAGGCTCTGCACAACCACTACAC

GCAGAAGAGCCTCTCCCTGTCTCCGGGTAAA (SEQ ID NO: 42).

[Table 2] Primers and templates of pcDNA3.4 vector and the target genes.

| Name | Sequence | Nucleotide sequence | Template |
|---|---|---|---|
| Vector-f | CCTCTAGAGTCCGGAGGCTG | SEQ ID NO: 43 | Synthetic gene pcDNA3.4 Vector |
| Vector-r | CCCTACCGGTTAGTAATGAGTTTGAT | SEQ ID NO: 44 | |
| RtxH-f | GCCTCCGGACTCTAGAGGGCCACCATGG GTTGGTCCC | SEQ ID NO: 45 | Synthetic gene RtxH |
| RtxH-r | CATTACTAACCGGTAGGGTCAGCCGGGTG ACAGGGAGAG | SEQ ID NO: 46 | |
| RtxL-f | GCCTCCGGACTCTAGAGGGCCACCATGG ACTTCCAAGTG | SEQ ID NO: 47 | Synthetic gene RtxL |
| RtxL-r | CATTACTAACCGGTAGGGTCAGCACTCTC CCCGGTTGAA | SEQ ID NO: 48 | |
| TrsH-f | GCCTCCGGACTCTAGAGGGCCACCATGA GTGTGCCCAC | SEQ ID NO: 49 | Synthetic gene TrsH |
| TrsH-r | CATTACTAACCGGTAGGGTCTCATTTACCC GGAGACAGGGAGAG | SEQ ID NO: 50 | |
| TrsL-f | GCCTCCGGACTCTAGAGGGCCACCATGG AATGGAGCTG | SEQ ID NO: 51 | Synthetic gene TrsL |
| TrsL-r | CATTACTAACCGGTAGGGTCAACATTCGC CTCTGTTGAAGCTC | SEQ ID NO: 52 | |
| dTrsH-f | GCCTCCGGACTCTAGAGGGCCACCATGA GTGTGCCCAC | SEQ ID NO: 53 | Synthetic gene dTrs1H, dTrs2H, dTrs3H, dTrs4H |
| dTrsH-r | CATTACTAACCGGTAGGGTCATTTACCCG GAGACAGGGAGAG | SEQ ID NO: 54 | |

(continued)

| Name | Sequence | Nucleotide sequence | Template |
|---|---|---|---|
| dTrsL-f | GCCTCCGGACTCTAGAGGGCCACCATGG AATGGAGCTG | SEQ ID NO: 55 | Synthetic gene dTrs1L(dTrs2L), dTrs3L, dTrs4L |
| dTrsL-r | CATTACTAACCGGTAGGGTCAACATTCGC CTCTGTTGAAGCTC | SEQ ID NO: 56 | |

1.2 The KOD reaction system of TAKARA (Cat.KFX-101), the reaction conditions, reagents and information of PCR target fragment adopted in the PCR reaction are shown in Table 3 to Table 6.

[Table 3] KOD reaction system of target genes or pcDNA3.4

| Reagents | Concentration | Final concentration | Volume ($\mu$L) |
|---|---|---|---|
| 2*PCR buffer for KOD | 2* | 1* | 25 |
| dNTP | 2mM each | 0.4mM each | 10 |
| 5' primer | 10$\mu$M | 0.5$\mu$M | 2.5 |
| 3' primer | 10$\mu$M | 0.5$\mu$M | 2.5 |
| Template (target gene or pcDNA3.4) | 10 ng/ $\mu$L | 0.2 ng/ $\mu$L | 1 |
| ddH$_2$O | | N/A | 8 |
| KOD FX | 1 U/ $\mu$L | 20 mU/ $\mu$L | 1 |
| Total | | | 50 |

[Table 4] PCR reaction conditions of vector pcDNA3.4

| Procedure | Temperature | Time | Condition |
|---|---|---|---|
| 1 | 94°C | 2min | |
| 2 | 98°C | 10s | |
| 3 | 60°C | 30s | |
| 4 | 68°C | 6min | Goto Step 2: 30cycles |
| 5 | 10°C | permanent | |

[Table 5] PCR reaction conditions of target genes

| Procedure | Temperature | Time | Condition |
|---|---|---|---|
| 1 | 94°C | 2min | |
| 2 | 98°C | 10s | |
| 3 | 60°C | 30s | |
| 4 | 68°C | 2-6min | Goto Step 2: 30cycles |
| 5 | 10°C | permanent | |

[Table 6] Information of PCR target fragment

| DNA | Primer | Template | Theoretical length |
|---|---|---|---|
| pcDNA3.4 vector | Vector-f, Vector-r | Synthetic gene pcDNA3.4 Vector | 6000bp |
| RtxH | RtxH-f, RtxH-r | Synthetic gene RtxH | 1500bp |
| RtxL | RtxL-f, RtxL-r | Synthetic gene RtxL | 800bp |

(continued)

| DNA | Primer | Template | Theoretical length |
|---|---|---|---|
| TrsH | TrsH-f, TrsH-r | Synthetic gene TrsH | 1500bp |
| TrsL | TrsL-f, TrsL-r | Synthetic gene TrsL | 800bp |
| dTrs1H | dTrsH-f, dTrsH-r | Synthetic gene dTrs1H | 1455bp |
| dTrs1L(dTrs2L) | dTrsL-f, dTrsL-r | Synthetic gene dTrs1L (dTrs2L) | 744bp |
| dTrs2H | dTrsH-f, dTrsH-r | Synthetic gene dTrs2H | 1455bp |
| dTrs3H | dTrsH-f, dTrsH-r | Synthetic gene dTrs3H | 1455bp |
| dTrs3L | dTrsL-f, dTrsL-r | Synthetic gene dTrs3L | 744bp |
| dTrs4H | dTrsH-f, dTrsH-r | Synthetic gene dTrs4H | 1455bp |
| dTrs4L | dTrsL-f, dTrsL-r | Synthetic gene dTrs4L | 744bp |

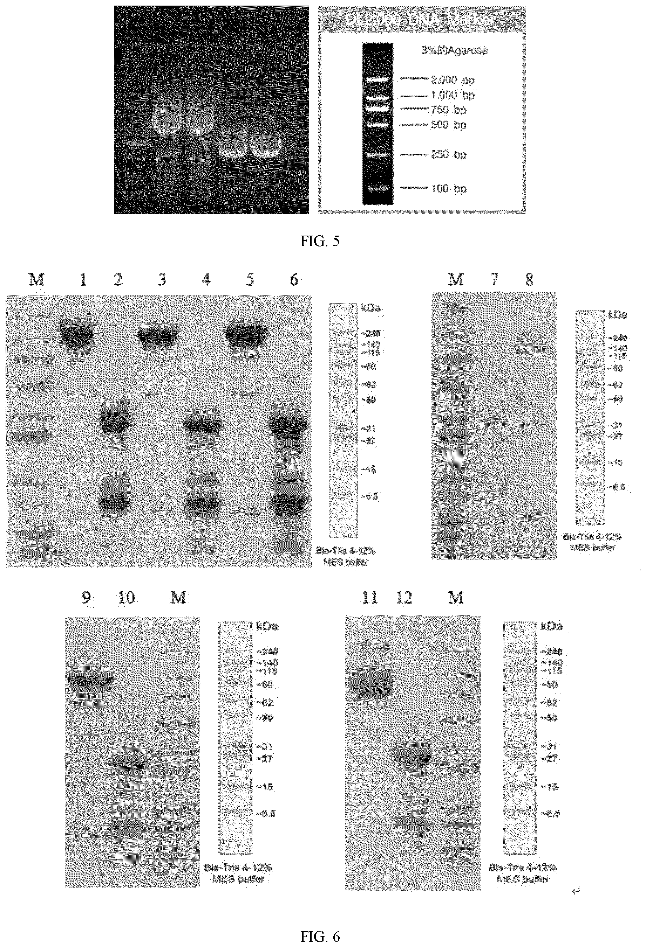

1.3 After PCR, the samples were subjected to 1.5% agarose gel electrophoresis. 1*TAE 300mL was added to the electrophoresis tank, and the gel was run at 100V for 30 min. The results showed that the band size of agarose gel electrophoresis was consistent with the theoretical size. The electrophoresis results were recovered by using the Omega Gel Extraction Kit (Cat. D2500-01), and eluted with 20 $\mu$L of eluent to obtain the purified PCR products. The electrophoresis results are shown in FIG. 2 to FIG. 5, in which lanes 1 to 9 from left to right in FIG. 2 are DL5000, pcDNA3.4, pcDNA3.4, pcDNA3.4, trsH, trsL, RtxH and RtxL, respectively; lanes 1 to 7 from left to right in FIG. 3 are DL2000, dTrs1H, dTrs1H, dTrs2H, dTrs2H, dTrs1L, and dTrs1L, respectively; lanes 1 to 5 from left to right in FIG. 4 are DL2000, dTrs3H, dTrs3H, dTrs3L, and dTrs3L, respectively; and lanes 1 to 5 from left to right in FIG. 5 are DL2000, dTrs4H, dTrs4H, dTrs4L, and dTrs4L, respectively.

1.4. The PCR bands obtained in 1.3 after gel excision and purification were subjected to homologous recombination, and the fragments were assembled by NEB's kit (Nebbuilder® HiFi DNA Assembly Master Mix, Cat. E2621S). The homologous recombination reaction system was incubated at 50°C for 1 hour, and the obtained reaction products were purified by using the Omega Cycle Pure Kit (Cat.D6492-01) and eluted with 15 $\mu$L of eluent. The reaction system and plasmid table are shown in Table 7 and Table 8.

[Table 7] Reaction system of homologous recombination

| Reagents | Volume ($\mu$L) |
|---|---|
| NEBuilder HiFi DNA Assembly Master Mix | 10 |
| pcDNA3.4 | 3 |
| Target fragment | 0.5-1 |
| de-ionized water | 6-6.5 |
| Total | 20 |

[Table 8] Plasmid Table

| Plasmid name | Target fragment | Volume of target fragment | Volume of de-ionized water |
|---|---|---|---|
| pcDNA3.4-RtxH | RtxH | 0.5 $\mu$L | 6.5 $\mu$L |
| pcDNA3.4-RtxL | RtxL | 0.5 $\mu$L | 6.5 $\mu$L |
| pcDNA3.4-TrsH | TrsH | 0.5 $\mu$L | 6.5 $\mu$L |
| pcDNA3.4-TrsL | TrsL | 0.5 $\mu$L | 6.5 $\mu$L |
| pcDNA3.4-dTrs1H | dTrs1H | 0.5 $\mu$L | 6.5 $\mu$L |
| pcDNA3.4-dTrs1L (pcDNA3.4-dTrs2L) | dTrs1L (dTrs2L) | 0.5 $\mu$L | 6.5 $\mu$L |
| pcDNA3.4-dTrs2H | dTrs2H | 0.5 $\mu$L | 6.5 $\mu$L |
| pcDNA3.4-dTrs3H | dTrs3H | 0.5 $\mu$L | 6.5 $\mu$L |
| pcDNA3.4-dTrs3L | dTrs3L | 0.5 $\mu$L | 6.5 $\mu$L |

(continued)

| Plasmid name | Target fragment | Volume of target fragment | Volume of de-ionized water |
|---|---|---|---|
| pcDNA3.4-dTrs4H =pcDNA3.4-dTrsH | dTrs4H | 0.5 $\mu$L | 6.5 $\mu$L |
| pcDNA3.4-dTrs4L =pcDNA3.4-dTrsL | dTrs4L | 0.5 $\mu$L | 6.5 $\mu$L |

1.5 The $CaCl_2$ competent cell DH5a was used for transformation, each plasmid sample obtained in step 1.4 was added to the competent cells at a ratio of 1:10, and placed on ice for 10 min, then incubated in a metal bath at 42°C for 45s, and then placed on ice for 10 min. The obtained solution was coated on LB solid medium with ampicillin (Amp) (final concentration of Amp was 50 $\mu$g/mL). After overnight culture at 37°C, the next day, a single clone was picked and placed in 2 mL of LB liquid medium (final concentration of Amp was 50 $\mu$g/mL), and the strains were shaken at 37°C and 200 rpm. The obtained bacterial solution was subjected to Sanger's sequencing to obtain the tested sequence. The sequencing primer was CMV-F: CGCAAATGGGGGGGGGGGGTG (SEQ ID NO: 79). Based on the comparison of the sequencing results, it was confirmed that all the tested sequences were consistent with the original sequences. 100 $\mu$L of bacteria solution was added to 50 mL of LB liquid medium with ampicillin (final concentration of 50 $\mu$g/mL), and cultured overnight at 37°C and 200 rpm. The strains were preserved, and the plasmid was extracted to obtain the target gene expression plasmid.

2. Transient expression of Trs, dTrs1-4 and Rtx proteins.

2.1. About 24 hours before transfection, FreeStyle™ CHO-S® cells (with cell density of 5 to $6\times10^5$/mL, from FreeStyle™ MAX CHO expression system of Gibco Company, USA, catalog number: K900020) were taken and cultured at 37°C, 5% $CO_2$ and 120-135 rpm/min.

2.2. On the day of transfection, the cells were diluted to a cell density of $1\times10^6$/mL, and 10 mL cells were added to each shake flask (the cell survival rate was above 95%).

2.3. FreeStyle™ MAX Reagent was mixed gently and carefully several times, without causing swirl.

2.4. 12.5 $\mu$g of the plasmids corresponding to each protein in Table 9 were taken, Opti-Pro™ SFM was added to a total volume of 0.2 mL, and mixed gently.

2.5. 25 $\mu$L of FreeStyle™ MAX Reagent was taken, Opti-Pro™ SFM was added to a total volume of 0.4 mL, and mixed gently (and carefully without causing swirl). After mixing evenly, the mixture was evenly added to the plasmid mixture and mixed gently to obtain 0.4 mL of two DNA-FreeStyle™ MAX mixtures, and incubated at room temperature for 10 minutes to form a complex.

2.6. 0.4 mL of the two DNA-FreeStyle™ MAX mixtures were slowly added into the cells, while the flask was rotated slowly.

2.7. The cells were cultured at 37°C, 5% $CO_2$, 120-135 rpm/min, without replacing or supplementing the culture medium, and the cell fluid was collected after 7 days.

[Table 9] Plasmids corresponding to different proteins

| Protein name | Plasmid name | |
|---|---|---|
| Trs | pcDNA3.4-TrsH | pcDNA3.4-TrsL |
| dTrs1 | pcDNA3.4-dTrs1H | pcDNA3.4-dTrs1L |
| dTrs2 | pcDNA3.4-dTrs2H | pcDNA3.4-dTrs2L (pcDNA3.4-dTrs1L) |
| dTrs3 | pcDNA3.4-dTrs3H | pcDNA3.4-dTrs3L |
| dTrs4 | pcDNA3.4-dTrs4H | pcDNA3.4-dTrs4L |
| Rtx | pcDNA3.4-RtxH | pcDNA3.4-RtxL |

3. Purification and identification of Trs, dTrs1-4 and Rtx proteins.

The culture supernatants of Trs, dTrs1-4 and Rtx were centrifuged at 4°C and 7,000 g for 15 min, and then filtered with a 0.22 $\mu$m syringe filter for sterilization. Buffer A1 (20 mM phosphate buffer, pH 7.4) at a volume 5 times the column volumes was used to balance the Protein A chromatographic medium (flow rate was 2 mL/min), the treated sample was added, rinsed with Buffer A1 until the absorption value reached the baseline, and eluted with Buffer B1 (0.1 M Glycine-HCl, pH 3.0). The elution peak sample was collected, and the target protein eluent was adjusted to neutral with 1M Tris-HCl (pH 9.0), and ultrafiltered into PBS buffer.

Identification of the target proteins: 10-well of SDS-PAGE precast gel was selected and run for 50 min at 150V. The sample was heated at 90°C for 5 min, and 10 $\mu$g of the sample was loaded into each well.

The purification effects of Trs, dTrs1-4 and Rtx proteins detected by SDS-PAGE were shown in FIG. 6. In FIG. 6, lane 1 was Trs elution without reduction, lane 2 was Trs elution with reduction, lane 3 was dTrs1 elution without reduction, lane 4 was dTrs1 elution with reduction, lane 5 was dTrs2 elution without reduction, lane 6 was dTrs2 elution with reduction, lane 7 was dTrs3 elution with reduction, lane 8 was dTrs3 elution without reduction, lane 9 was dTrs4 elution without reduction, lane 10 was dTrs4 elution with reduction, lane 11 was Rtx elution without reduction, and lane 12 was Rtx elution with reduction. The expression levels of Trs, dTrs1-4 and Rtx proteins were shown in Table 10.

[Table 10] Expression levels of Trs, dTrs1-4 and Rtx proteins

| Protein sample | Protein concentration in culture medium (mg/mL) |
|---|---|
| Trs | 0.0086 |
| dTrs1 | 0.0317 |
| dTrs2 | 0.0182 |
| dTrs3 | 0.00017 |
| dTrs4 | 0.172 |
| Rtx | 0.025 |

4. ELISA for binding of Trs, dTrs1-4 and Rtx proteins to Her2.

4.1 The binding of Her2 protein to Trs, dTrs1-4 or Rtx protein was detected by enzyme-linked immunosorbent assay using the purchased Her2 (10004-HCCH-B, Sino Biological) with binding activity as substrate, using Trs, dTrs1-4 or Rtx protein as primary antibody, and using HRP-conjugated goat anti-human IgG as the secondary antibody.

20 $\mu$g of Her2 protein solid was dissolved in 80 $\mu$L of sterile water to prepare 0.25 mg/mL stock solution, which was dispensed in 0.5 mL EP tubes, 10 $\mu$L/tube, and stored at - 80°C.

Primary antibody: Trs (0.44 mg/mL), dTrs1 (0.38 mg/mL), dTrs2 (0.42 mg/mL), dTrs3 (0.115 mg/mL), dTrs4 (1.4 mg/mL), Rtx(0.21 mg/mL), in which Trs was the positive control and Rtx was the negative control.

Secondary antibody: HRP-conjugated goat anti-human IgG (Sangon Biotechnology (Shanghai) Co., Ltd., Cat. No. D110150).

4.2 Detection scheme:

1) Coating: the culture supernatant was diluted with coating solution, 0.1 $\mu$g/100 $\mu$L Her2 protein (34 $\mu$L of stock solution + 8466 $\mu$L of coating solution) was added to each well, the plate was sealed, and incubated at 37°C for 2 hours. The liquid in the hole was discarded, the plate was washed with washing solution for 3 times, 200 $\mu$L each time, and still-stood for 2 min.
2) Blocking: 100 $\mu$L of blocking solution was added to each well, and kept overnight at 4°C, and the liquid in the hole was discarded.
3) Incubation with primary antibody (Trs, dTrs1-4 or Rtx): Trs, dTrs1-4 and Rtx was diluted with antibody diluent, 100 $\mu$L was add into each well, the plate was sealed, and incubated at 37°C for 1 h. The liquid in the well was discarded. The plate was washed with washing solution for 3 times, 200 $\mu$L each time, and still-stood for 2 min.
4) Incubation of secondary antibody: secondary antibody was diluted with antibody diluent at a ratio of 1: 2000, 100 $\mu$L of the solution was added into each well, the plate was sealed, and incubated at 37°C for 1 h. The liquid in the well was discarded. The plate was washed with washing solution for 3 times, 200 $\mu$L each time, and still-stood for 2 min.
5) Detection: 100 $\mu$L of TMB was added to each well, the plate was sealed, and incubated at 37°C in the dark for 15 min. At this time, the wells turned blue, and 100 $\mu$L of stop solution was added to each well, the wells turned yellow immediately, and the OD value was detected at 450 nm. The detection results were shown in FIG.7 and Table 11. The results showed that the binding capacity of dTrs1-dTrs4 to Her2 decreased gradually compared with Trs, in which, the binding capacity of dTrs4 to Her2 was the lowest, which was close to the negative control Rtx. In addition, it was found that the expression level of dTrs4 was high. Therefore, dTrs4 can be selected as the drug skeleton.

The preparation of coating solution was shown in the following table.

| Coating solution:0.05 M carbonate-bicarbonate | | |
|---|---|---|
| Volume: | 1L | Preparation method |
| Solvent: | ultrapure water | Reagent was weighed on filter paper, and transferred to a glass, 1 L of ultrapure water was added, and stirred until it was completely dissolved, and the pH was measured. If the pH was lower than 9.5, it was adjusted to 9.5 with 1N NaOH. |
| Storage temperature: | RT | |
| Storage period: | 365 days | |
| pH: | 9.68 | |
| Ingredient | | Weight |
| $Na_2CO_3$ | | 5.81g |
| $NaHCO_3$ | | 2.93g |

The preparation of blocking solution was shown in the following table:

| Blocking solution:5% BSA in TBST | | |
|---|---|---|
| Volume: | 20 mL | Preparation method |
| Solvent: | TBST | BSA was weighed on filter paper, and transferred to a 50 mL centrifuge tube, 15 mL if TBST was added, and vortexed until it was completely dissolved, and then TBST was added to 20 mL. |
| Storage temperature: | - | |
| Storage period: | prepared before use | |
| pH: | - | |
| Ingredient | | Weight |
| BSA | | 1 g |
| TBST | | 20 mL |

The preparation of antibody diluent was shown in the following table:

| Antibody diluent:1% BSA in TBST | | |
|---|---|---|
| Volume: | 20 mL | Preparation method |
| Solvent: | TBST | BSA was weighed on filter paper, and transferred to a 50 mL centrifuge tube, 15 mL of TBST was added, and vortexed until it was completely dissolved, and then TBST was added to 20 mL. |
| Storage temperature: | - | |
| Storage period: | prepared before use | |
| pH: | - | |
| ingredient | | Weight |
| BSA | | 0.2 g |
| TBST | | 20 mL |

The preparation of washing solution was shown in the following table:

| Washing solution: 10*Tris-buffered saline (pH 7.4) with 0.1 % (v/v) Tween 20(TBST) | | | | | |
|---|---|---|---|---|---|
| Volume: | 100mL | Preparation method | | | |
| Solvent: | ultrapure water | Reagent was weighed in a PP measuring cup, 90 mL of ultrapure water was added, after it was complete dissolved, the pH was adjusted to 7.6 with HCl and the volume was made up to 100 mL. | | | |
| Storage temperature: | RT | | | | |
| Storage period: | 365 days | | | | |
| pH: | 7.6 | | | | |
| Concentration | Ingredient | Reagent | Molecular weight | Weight | |
| 200mM | Tris-HCl | Tris base | 121.14 | 2.42g | |
| 1.5M | NaCl | NaCl | 58.5 | 8.78g | |
| 2% | Tween 20 | Tween 20 | | 2mL | |

[Table 11] Binding results of TRS, dTrs1-4 and Rtx proteins to Her2

| | Trs | dTrs1 | dTrs2 | dTrs3 | dTrs4 | RTX |
|---|---|---|---|---|---|---|
| $EC_{50}$ | Below the lower limit | 0.4655 to 0.6385 | 1.997 to 2.747 | Above the upper limit | Above the upper limit | Above the upper limit |

Note: Values below the lower limit and above the upper limit go beyond the calculation range; value below the lower limit means strong binding capacity; and value above the upper limit means weak binding capacity.

5. Binding of Trs, dTrs1-4 and Rtx proteins to IgA, IgM or HAS

5.1 The experimental steps were as follows:

1) The plate was sealed with 100 μL/well coating solution containing 10 μg/mL IgA/IgM/HSA, and kept overnight at 4°C. IgA was derived from human serum (Sigma-Aldrich, I4036) and a concentration thereof was 1 mg/mL. IgM was derived from human serum (Sigma-Aldrich, I8260) and a concentration thereof was 1 mg/mL. A concentration of HSA (Absin, abs47047388) was 20 mg/mL;

2) The solution from step 1) was discarded, and the plate was washed with PBST (10 times dilution of Solebold 10*PBS with double distilled water (sterilized) and +0.1% Tween), washed with a plate washer for 4 times, and spin-dried with a plate spinner at 600×g for 1 min, and pat-dried for several times;

3) 100 μL/well blocking solution (0.5% Casein (pH 7.09, 1M NaOH>50 mg/mL was added to prepare in small aliquots, PBST was added and the pH was adjusted with HCl, and then prepared at a constant volume), and incubated at 37°C for 60min; then the plate washing in step 2) was repeated;

4) 100 μL/well of 10-fold gradient dilution (10, 1, 0.1, 0.01, 0.001, 0.0001, 0.00001) of each group of samples (Trs, dTrs1-4 or Rtx) were added, the plate was sealed and incubated at room temperature for 30 min; then the plate washing in step 2) was repeated;

5) 100 μL of detection antibody (Goat Anti-Human IgG-Fc Secondary Antibody (HRP μg/mL), *Sino Biological* and SSA001, diluted 1: 3K with PBST to a working concentration of 1.2 μg/mL) was added into each well, the plate was sealed and incubated at room temperature for 30 min; then the plate washing in step 2) was repeated;

6) 100 μL of Soledad TMB substrate chromogenic solution was added to each well, incubated at 25°C in the dark for 6 min, then 100 μL of Soledad stop solution was added to each well, $OD_{450}$ was measured.

5.2 Results

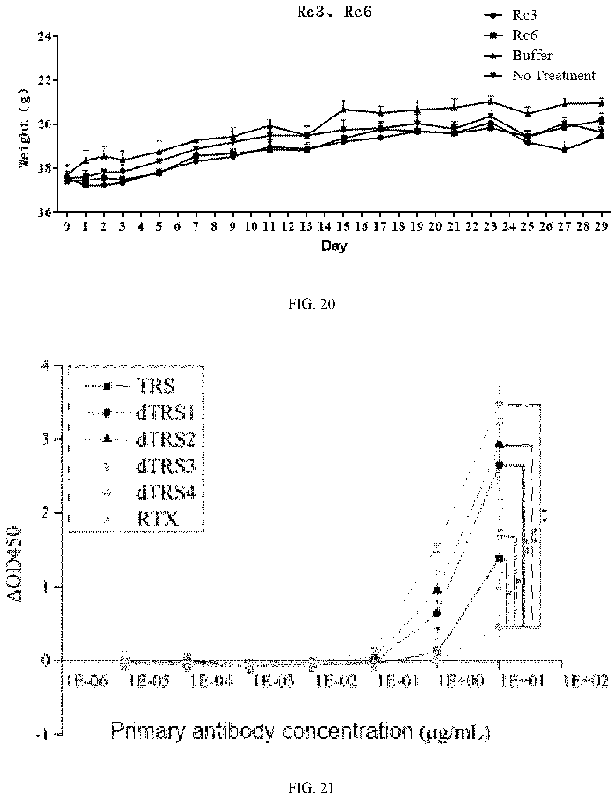

The results of ELISA detection with human IgA plating were shown in FIG. 21, in which, $p<0.01$** and $p<0.05$*. The results indicated that dTrs4, as a detection molecule, generated very low signal, even lower than that of wild-type Trs and the irrelevant antibody RTX, indicating that dTrs4 did not bind to human IgA.

The difference analysis of FIG. 21 was shown in the following table:

| Primary antibody concentration 10 μg/mL T test (n=3) | TRS | dTRS1 | dTRS2 | dTRS3 | dTRS4 | RTX |
|---|---|---|---|---|---|---|
| p | 0.015 | 0.010 | 0.002 | 0.008 | - | 0.018 |

The p value of t-test between dTRS4 and other molecules at 10 μg/mL was obtained with human IgA-plated plate.

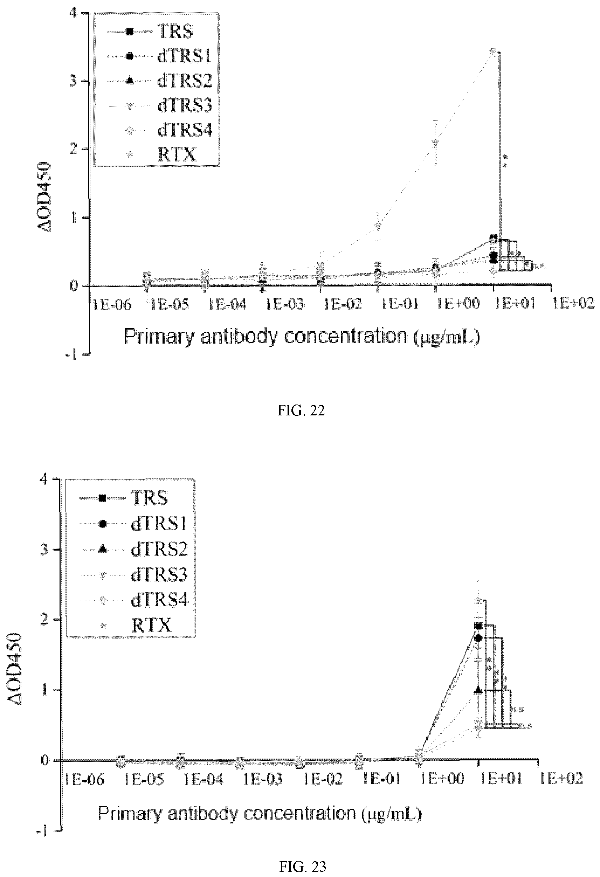

The results of ELISA detection with human IgM plating were shown in FIG. 22, in which, $p<0.01$** and $p<0.05$*. The results indicated that dTrs4, as a detection molecule, generated very low signal, even lower than that of the wild-type Trs and the irrelevant antibody RTX, indicating that dTrs4 did not bind to human IgM.

The difference analysis of FIG. 22 is shown in the following table:

| Primary antibody concentration 10 μg/mL T test (n=3) | TRS | dTRS1 | dTRS2 | dTRS3 | dTRS4 | RTX |
|---|---|---|---|---|---|---|
| p | 0.031 | 0.026 | 0.113 | 0.000 | - | 0.017 |

The p value of t-test between dTRS4 and other molecules at 10 μg/mL was obtained with human HAS-plated plate.

The results of ELISA detection with human HSA plating were shown in FIG. 23, in which, $p<0.01$** and $p<0.05$*. The results indicated that the signal generated by unrelated antibody RTX was slightly higher, and other molecular signals were very low. dTrs4, as a detection molecule, generated the lowest signal, even lower than that of the wild-type Trs and irrelevant antibody RTX, indicating that dTrs4 did not bind to human HSA.

The difference analysis of FIG.23 is shown in the following table:

| Primary antibody concentration 10 μg/mL T test (n=3) | TRS | dTRS1 | dTRS2 | dTRS3 | dTRS4 | RTX |
|---|---|---|---|---|---|---|
| p | 0.005 | 0.004 | 0.083 | 0.755 | - | 0.003 |

The p value of t-test between dTRS4 and other molecules at 10 μg/mL was obtained with human HAS-plated plate.

**Example 2: Preparation and Selection of RC1 to RC8 proteins**

1. Expression construction: the steps of expression construction of RC1 to RC8 proteins were the same as those of expression construction in Example 1, except for the difference in target genes. FIG. 8 is a schematic structural diagram of the RC1 to RC8 proteins, and FIG. 8 is a schematic structural diagram of PLA2R. PLA2R antigen epitope peptide CFC1 (SEQ ID NO: 21) in RC1 to RC8 proteins is the amino acid sequence from 1 to 367 (Glu) of the PLA2R fragment, and the PLA2R antigen epitope peptide CFC2 (SEQ ID NO: 22) is amino acid sequence from 1 to 510 (Asp) of the PLA2R fragment.

The name of the target gene, primer, sequence and composition of the heavy chain, light chain and its signal peptide are shown in Table 12, the name and sequence of the target genes are shown in Table 13, the primer and template of the pcDNA3.4 vector and the target genes are shown in Table 14, and the information of the target fragment is shown in Table 15.

The nucleotide sequence of PLA2R signal peptide:

ATGCTGCTGTCGCCGTCGCTGCTGCTGCTGCTGCTGCTGGGGGGCGCCGCGGGGCT

GCGCC (SEQ ID NO: 80).

The nucleotide sequence of Trs heavy chain signal peptide:

ATGAGTGTGCCCACTCAGGTCCTGGGGGTTGCTGCTGCTGTGGCTTACAGATGCCAG

ATGT (SEQ ID NO: 81).

The nucleotide sequence of Trs light chain signal peptide:

ATGGAATGGAGCTGGGTCTTTCTCTTCTTCCTGTCAGTAACTACAGGTGTCCACTC
C (SEQ ID NO: 82).

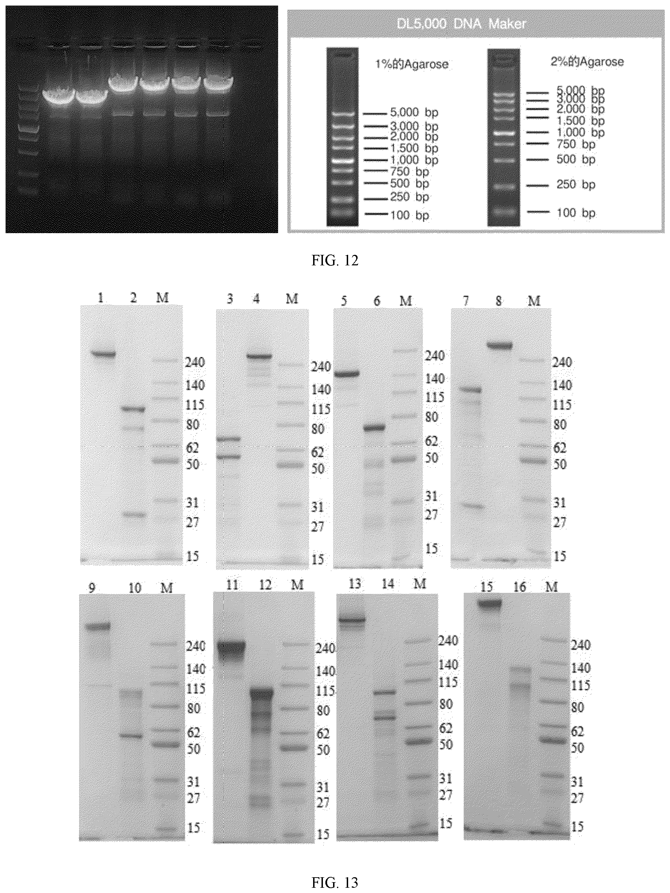

The electrophoresis results of the purified PCR products are shown in FIG. 10 to 12, in which lanes 1 to 5 from left to right in FIG. 10 are DL5000, pcDNA3.4, pcDNA3.4, pcDNA3.4, and PCDNA3.4, respectively; the lanes 1 to 13 from left to right in FIG. 11 are DL5000, pcDNA3.4, pcDNA3.4, pcDNA3.4, RC1H-1, RC1H-2, RC2L-2, RC4H-1, RC4H-2, RC5L-1, and RC5L-2, respectively; lanes 1 to 7 in FIG. 12 from left to right are DL5000, irrelevant, irrelevant, RC3, RC3, RC6, and RC6, respectively.

The purified PCR bands after gel excision and purification were subjected to homologous recombination, and the system was consistent with Table 7. The constructed plasmid table is shown in Table 16.

[Table 12] Composition of heavy chain and light chain of RC1 to RC8 Proteins

| Protein | Heavy chain | Heavy chain signal peptide | Light chain | Light chain signal peptide |
|---|---|---|---|---|
| RC1 | RC1H (CFC1+ connecting peptide +dTrsH) | PLA2R signal peptide | dTrsL | Trs light chain signal peptide |
| RC2 | dTrsH | Trs heavy chain signal peptide | RC2L(CFC1+connect ing peptide +dTrsL) | PLA2R signal peptide |
| RC3 | CFC1+connecting peptide +Fc | PLA2R signal peptide | - | - |
| RC4 | RC4H (CFC2+connecting peptide +dTrsH) | PLA2R signal peptide | dTrsL | Trs light chain signal peptide |
| RC5 | dTrsH | Trs heavy chain signal peptide | RC5L(CFC2+connect ing peptide +dTrsL) | PLA2R signal peptide |
| RC6 | CFC2+connecting peptide +Fc | PLA2R signal peptide | - | - |
| RC7 | RC1H (CFC1+connecting peptide +dTrsH) | PLA2R signal peptide | RC2L(CFC1+connect ing peptide +dTrsL) | PLA2R signal peptide |
| RC8 | RC4H (CFC2+connecting peptide +dTrsH) | PLA2R signal peptide | CRC5L(CFC2+conne cting peptide +dTrsL) | PLA2R signal peptide |

[Table 13] Names and sequences of target genes

| Name | | Primer | Amino acid sequence | Nucleotide sequence |
|---|---|---|---|---|
| RC1H | RC1H-1 | RCH-f1, RCH-r1 | SEQ ID NO: 57 | SEQ ID NO: 58 |
| | RC1H-2 | RCH-f2, RCH-r2 | | |
| RC2L | RC2L-1 | RCL-f1, RCL-r1 | SEQ ID NO: 59 | SEQ ID NO: 60 |
| | RC2L-2 | RCL-f2, RCL-r2 | | |
| RC3 | | RC3,6-f, RC3,6-r | SEQ ID NO: 61 | SEQ ID NO: 62 |
| RC4H | RC4H-1 | RCH-f1, RCH-r1 | SEQ ID NO: 63 | SEQ ID NO: 64 |
| | RC4H-2 | RCH-f2, RCH-r2 | | |
| RC5L | RC5L-1 | RCL-f1, RCL-r1 | SEQ ID NO: 65 | SEQ ID NO: 66 |
| | RC5L-2 | RCL-f2, RCL-r2 | | |
| RC6 | | RC3,6-f, RC3,6-r | SEQ ID NO: 67 | SEQ ID NO: 68 |
| Note: RC1H, RC2L, RC4H and RC5L are all formed by splicing by two PCR fragments, that is, RC1H is formed by splicing RC1H-1 and RC1H-2; RC2L is formed by splicing RC2L-1 and RC2L-2; RC4H is formed by splicing RC4H-1 and RC4H-2; and RC5L is formed by splicing RC5L-1 and RC5L-2. | | | | |

The amino acid sequence of RC1H:

EGVAAALTPERLLEWQDKGIFVIQSESLKKCIQAGKSVLTLENCKQANKHMLWKWVS NHGLFNIGGSGCLGLNFSAPEQPLSLYECDSTLVSLRWRCNRKMITGPLQYSVQVAHD NTVVASRKYIHKWISYGSGGGDICEYLHKDLHTIKGNTHGMPCMFPFQYNHQWHHE CTREGREDDLLWCATTSRYERDEKWGFCPDPTSAEVGCDTIWEKDLNSHICYQFNLL SSLSWSEAHSSCQMQGGTLLSITDETEENFIREHMSSKTVEVWMGLNQLDEHAGWQ WSDGTPLNYLNWSPEVNFEPFVEDHCGTFSSFMPSAWRSRDCESTLPYICKKYLNHID HEIVEGGGGSGGGGSEVQLVESGGGLVQPGGSLRLSCAASGFNIGGTGIHWVRQAPG KGLEWVAGIYPTNGGTGYAGSVKGRFTISADTSKNTAYLQMNSLRAEDTAVYYCSRG GGDGFGAMGYWGQGTLVTVSSASTKGPSVFPLAPSSKSTSGGTAALGCLVKDYFPEP VTVSWNSGALTSGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQK SLSLSPGK (SEQ ID NO: 57).

The nucleotide sequence of RC1H:

GAGGGTGTGGCGGCGGCGCTTACCCCCGAGCGGCTCCTGGAGTGGCAGGATAAAG GAATATTTGTTATCCAAAGTGAGAGTCTCAAGAAATGCATTCAAGCAGGTAAATCG GTTCTGACCCTGGAGAACTGCAAGCAAGCAAACAAGCACATGCTGTGGAAATGG

GTTTCAAACCATGGCCTCTTTAACATAGGAGGCAGTGGTTGCCTGGGCCTGAATTT

CTCCGCCCCAGAGCAGCCATTAAGCTTATATGAATGTGACTCCACCCTCGTTTCCTT

ACGGTGGCGCTGTAACAGGAAGATGATCACAGGCCCGCTGCAGTACTCTGTCCAG

GTGGCGCATGACAACACAGTGGTGGCCTCACGGAAGTATATTCATAAGTGGATTTC

TTATGGGTCAGGTGGTGGAGACATTTGTGAATATCTACACAAAGATTTGCATACAAT

CAAAGGGAACACCCACGGGATGCCGTGTATGTTTCCCTTCCAGTATAACCATCAGT

GGCATCATGAATGTACCCGTGAAGGTCGGGAAGATGACTTACTGTGGTGTGCCACG

ACAAGCCGTTATGAAAGAGATGAAAGTGGGGATTTTGCCCTGATCCCACCTCTGC

AGAAGTAGGTTGTGATACTATTTGGGAGAAGGACCTCAATTCACACATTTGCTACC

AGTTCAACCTGCTTTCATCTCTCTCTTGGAGTGAGGCACATTCTTCATGCCAGATGC

AAGGAGGTACGCTGTTAAGTATTACAGATGAAACTGAAGAAAATTTCATAAGGGA

GCACATGAGCAGTAAAACAGTGGAGGTGTGGATGGGCCTCAATCAGCTGGATGAA

CACGCTGGCTGGCAGTGGTCTGATGGAACGCCGCTCAACTATCTGAATTGGAGCC

CAGAGGTAAATTTTGAGCCATTTGTTGAAGATCACTGTGGAACATTTAGTTCATTTA

TGCCAAGTGCCTGGAGGAGTCGGGATTGTGAGTCCACCTTGCCATATATATGTAAA

AAATATCTAAACCACATTGATCATGAAATAGTTGAAGGCGGTGGAGGCTCCGGCGG

AGGTGGAAGCGAAGTCCAGCTGGTGGAAAGTGGCGGTGGACTGGTTCAACCAGG

AGGAAGCCTAAGACTAAGCTGCGCCGCCAGCGGTTTTAACATCGGTGGCACAGGC

ATCCATTGGGTCCGGCAGGCACCCGGCAAAGGTTTAGAGTGGGTCGCAGGCATCT

ACCCCACTAATGGTGGCACCGGCTACGCTGGTAGCGTGAAGGGCAGGTTCACCAT

TAGCGCTGACACCAGCAAGAACACTGCTTATCTGCAGATGAATAGCCTGCGTGCCG

AAGACACAGCCGTCTATTACTGTAGTAGAGGCGGAGGTGATGGGTTCGGCGCGAT

GGGCTACTGGGGACAGGGCACACTCGTGACCGTTAGTAGCGCTAGCACCAAGGGC

CCATCGGTCTTCCCCCTGGCACCCTCCTCCAAGAGCACCTCTGGGGGCACAGCGG

CCCTGGGCTGCCTGGTCAAGGACTACTTCCCCGAACCGGTGACGGTGTCGTGGAA

CTCAGGCGCCCTGACCAGCGGCGTGCACACCTTCCCGGCTGTCCTACAGTCCTCA

GGACTCTACTCCCTCAGCAGCGTGGTGACCGTGCCCTCCAGCAGCTTGGGCACCC

AGACCTACATCTGCAACGTGAATCACAAGCCCAGCAACACCAAGGTGGACAAGA

AAGTAGAGCCCAAATCTTGTGACAAAACTCACACATGCCCACCGTGCCCAGCACC

TGAACTCCTGGGGGGACCGTCAGTCTTCCTCTTCCCCCCAAAACCCAAGGACACC

CTCATGATCTCCCGGACCCCTGAGGTCACATGCGTGGTGGTGGACGTGAGCCACG
AAGACCCTGAGGTCAAGTTCAACTGGTACGTGGACGGCGTGGAGGTGCATAATGC
CAAGACAAAGCCGCGGGAGGAGCAGTACAACAGCACGTACCGTGTGGTCAGCGT
CCTCACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAAGGGC
AGCCCCGAGAACCACAGGTGTACACCCTGCCCCCATCCCGGGATGAGCTGACCAA
GAACCAGGTCAGCCTGACCTGCCTGGTCAAAGGCTTCTATCCCAGCGACATCGCC
GTGGAGTGGGAGAGCAATGGGCAGCCGGAGAACAACTACAAGACCACGCCTCCC
GTGCTGGACTCCGACGGCTCCTTCTTCCTCTACAGCAAGCTCACCGTGGACAAGA
GCAGGTGGCAGCAGGGGAACGTCTTCTCATGCTCCGTGATGCATGAGGCTCTGCA
CAACCACTACACGCAGAAGAGCCTCTCCCTGTCTCCGGGTAAA (SEQ ID NO: 58).

The amino acid sequence of RC2L:

EGVAAALTPERLLEWQDKGIFVIQSESLKKCIQAGKSVLTLENCKQANKHMLWKWVS
NHGLFNIGGSGCLGLNFSAPEQPLSLYECDSTLVSLRWRCNRKMITGPLQYSVQVAHD
NTVVASRKYIHKWISYGSGGGDICEYLHKDLHTIKGNTHGMPCMFPFQYNHQWHHE
CTREGREDDLLWCATTSRYERDEKWGFCPDPTSAEVGCDTIWEKDLNSHICYQFNLL
SSLSWSEAHSSCQMQGGTLLSITDETEENFIREHMSSKTVEVWMGLNQLDEHAGWQ
WSDGTPLNYLNWSPEVNFEPFVEDHCGTFSSFMPSAWRSRDCESTLPYICKKYLNHID
HEIVEGGGGSGGGGSDIQMTQSPSSLSASVGDRVTITCRASQGVGTAVAWYQQKPGK
APKLLIYSASGLYSGVPSRFSGSRSGTDFTLTISSLQPEDFATYYCQQHGTTPPTFGQGT
KVEIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC (SEQ
ID NO: 59).

The nucleotide sequence of RC2L:

GAGGGTGTGGCGGCGGCGCTTACCCCCGAGCGGCTCCTGGAGTGGCAGGATAAAG

GAATATTTGTTATCCAAAGTGAGAGTCTCAAGAAATGCATTCAAGCAGGTAAATCG

GTTCTGACCCTGGAGAACTGCAAGCAAGCAAACAAGCACATGCTGTGGAAATGG

GTTTCAAACCATGGCCTCTTTAACATAGGAGGCAGTGGTTGCCTGGGCCTGAATTT

CTCCGCCCCAGAGCAGCCATTAAGCTTATATGAATGTGACTCCACCCTCGTTTCCTT

ACGGTGGCGCTGTAACAGGAAGATGATCACAGGCCCGCTGCAGTACTCTGTCCAG

GTGGCGCATGACAACACAGTGGTGGCCTCACGGAAGTATATTCATAAGTGGATTTC

TTATGGGTCAGGTGGTGGAGACATTTGTGAATATCTACACAAAGATTTGCATACAAT

CAAAGGGAACACCCACGGGATGCCGTGTATGTTTCCCTTCCAGTATAACCATCAGT

GGCATCATGAATGTACCCGTGAAGGTCGGGAAGATGACTTACTGTGGTGTGCCACG

ACAAGCCGTTATGAAAGAGATGAAAGTGGGGATTTTGCCCTGATCCCACCTCTGC

AGAAGTAGGTTGTGATACTATTTGGGAGAAGGACCTCAATTCACACATTTGCTACC

AGTTCAACCTGCTTTCATCTCTCTCTTGGAGTGAGGCACATTCTTCATGCCAGATGC

AAGGAGGTACGCTGTTAAGTATTACAGATGAAACTGAAGAAAATTTCATAAGGGA

GCACATGAGCAGTAAAACAGTGGAGGTGTGGATGGGCCTCAATCAGCTGGATGAA

CACGCTGGCTGGCAGTGGTCTGATGGAACGCCGCTCAACTATCTGAATTGGAGCC

CAGAGGTAAATTTTGAGCCATTTGTTGAAGATCACTGTGGAACATTTAGTTCATTTA

TGCCAAGTGCCTGGAGGAGTCGGGATTGTGAGTCCACCTTGCCATATATATGTAAA

AAATATCTAAACCACATTGATCATGAAATAGTTGAAGGCGGTGGAGGCTCCGGCGG

AGGTGGAAGCGATATACAGATGACGCAGAGCCCTAGCAGTCTAAGTGCAAGCGTT

GGAGACCGTGTCACCATTACCTGCCGCGCTAGCCAGGGCGTCGGCACCGCAGTCG

CCTGGTATCAGCAAAAACCGGGTAAGGCTCCAAAGCTGTTGATCTACAGTGCCAG

CGGCCTGTACAGCGGTGTGCCAAGTAGGTTCAGCGGGAGCCGGAGTGGGACAGA

CTTTACACTCACTATTAGCAGTCTGCAGCCCGAGGACTTCGCCACCTACTACTGTC

AGCAGCATGGCACAACACCCCCAACATTCGGGCAGGGAACCAAAGTTGAGATAA

AACGAACGGTTGCCGCGCCAAGCGTATTTATCTTTCCCCCCAGTGATGAGCAACTG

AAGAGTGGTACAGCCAGCGTTGTCTGCCTTTTGAATAACTTCTATCCTCGGGAGGC

AAAGGTCCAGTGGAAAGTGGATAATGCACTCCAAAGCGGTAACAGCCAGGAGAG

TGTGACAGAACAGGACAGCAAAGATAGTACCTACAGCCTCAGTAGTACCCTCACA

CTGAGCAAAGCGGACTATGAAAAGCACAAGGTGTACGCTTGTGAAGTGACCCACC

AGGGCCTAAGCAGCCCTGTTACTAAGAGCTTCAACAGAGGCGAATGT (SEQ ID NO:

60).

The amino acid sequence of RC3:

EGVAAALTPERLLEWQDKGIFVIQSESLKKCIQAGKSVLTLENCKQANKHMLWKWVS

NHGLFNIGGSGCLGLNFSAPEQPLSLYECDSTLVSLRWRCNRKMITGPLQYSVQVAHD

NTVVASRKYIHKWISYGSGGGDICEYLHKDLHTIKGNTHGMPCMFPFQYNHQWHHE

CTREGREDDLLWCATTSRYERDEKWGFCPDPTSAEVGCDTIWEKDLNSHICYQFNLL

SSLSWSEAHSSCQMQGGTLLSITDETEENFIREHMSSKTVEVWMGLNQLDEHAGWQ

WSDGTPLNYLNWSPEVNFEPFVEDHCGTFSSFMPSAWRSRDCESTLPYICKKYLNHID

HEIVEGGGGSGGGGSTHTCPPCPAPELLGGPSVFLFPPKPKDTLMISRTPEVTCVVVDV

SHEDPEVKFNWYVDGVEVHNAKTKPREEQYNSTYRVVSVLTVLHQDWLNGKEYKC

KVSNKALPAPIEKTISKAKGQPREPQVYTLPPSRDELTKNQVSLTCLVKGFYPSDIAVE

WESNGQPENNYKTTPPVLDSDGSFFLYSKLTVDKSRWQQGNVFSCSVMHEALHNHY

TQKSLSLSPGK (SEQ ID NO: 61).

The nucleotide sequence of RC3:

GAGGGTGTGGCGGCGGCGCTTACCCCCGAGCGGCTCCTGGAGTGGCAGGATAAAG

GAATATTTGTTATCCAAAGTGAGAGTCTCAAGAAATGCATTCAAGCAGGTAAATCG

GTTCTGACCCTGGAGAACTGCAAGCAAGCAAACAAGCACATGCTGTGGAAATGG

GTTTCAAACCATGGCCTCTTTAACATAGGAGGCAGTGGTTGCCTGGGCCTGAATTT

CTCCGCCCCAGAGCAGCCATTAAGCTTATATGAATGTGACTCCACCCTCGTTTCCTT

ACGGTGGCGCTGTAACAGGAAGATGATCACAGGCCCGCTGCAGTACTCTGTCCAG

GTGGCGCATGACAACACAGTGGTGGCCTCACGGAAGTATATTCATAAGTGGATTTC

TTATGGGTCAGGTGGTGGAGACATTTGTGAATATCTACACAAAGATTTGCATACAAT

CAAAGGGAACACCCACGGGATGCCGTGTATGTTTCCCTTCCAGTATAACCATCAGT

GGCATCATGAATGTACCCGTGAAGGTCGGGAAGATGACTTACTGTGGTGTGCCACG

ACAAGCCGTTATGAAAGAGATGAAAGTGGGGATTTTGCCCTGATCCCACCTCTGC

AGAAGTAGGTTGTGATACTATTTGGGAGAAGGACCTCAATTCACACATTTGCTACC

AGTTCAACCTGCTTTCATCTCTCTCTTGGAGTGAGGCACATTCTTCATGCCAGATGC

AAGGAGGTACGCTGTTAAGTATTACAGATGAAACTGAAGAAAATTTCATAAGGGA

GCACATGAGCAGTAAAACAGTGGAGGTGTGGATGGGCCTCAATCAGCTGGATGAA

CACGCTGGCTGGCAGTGGTCTGATGGAACGCCGCTCAACTATCTGAATTGGAGCC

CAGAGGTAAATTTTGAGCCATTTGTTGAAGATCACTGTGGAACATTTAGTTCATTTA

TGCCAAGTGCCTGGAGGAGTCGGGATTGTGAGTCCACCTTGCCATATATATGTAAA

AAATATCTAAACCACATTGATCATGAAATAGTTGAAGGCGGTGGAGGCTCCGGCGG

AGGTGGAAGCACTCACACATGCCCACCGTGCCCAGCACCTGAACTCCTGGGGGGA

CCGTCAGTCTTCCTCTTCCCCCCAAAACCCAAGGACACCCTCATGATCTCCCGGAC

CCCTGAGGTCACATGCGTGGTGGTGGACGTGAGCCACGAAGACCCTGAGGTCAA

GTTCAACTGGTACGTGGACGGCGTGGAGGTGCATAATGCCAAGACAAAGCCGCGG

GAGGAGCAGTACAACAGCACGTACCGTGTGGTCAGCGTCCTCACCGTCCTGCACC

AGGACTGGCTGAATGGCAAGGAGTACAAGTGCAAGGTCTCCAACAAAGCCCTCC

CAGCCCCCATCGAGAAACCATCTCCAAAGCCAAAGGGCAGCCCCGAGAACCAC

AGGTGTACACCCTGCCCCCATCCCGGGATGAGCTGACCAAGAACCAGGTCAGCCT

GACCTGCCTGGTCAAAGGCTTCTATCCCAGCGACATCGCCGTGGAGTGGGAGAGC

AATGGGCAGCCGGAGAACAACTACAAGACCACGCCTCCCGTGCTGGACTCCGAC

GGCTCCTTCTTCCTCTACAGCAAGCTCACCGTGGACAAGAGCAGGTGGCAGCAGG

GGAACGTCTTCTCATGCTCCGTGATGCATGAGGCTCTGCACAACCACTACACGCAG

AAGAGCCTCTCCCTGTCTCCGGGTAAA (SEQ ID NO: 62).

The amino acid sequence of RC4H:

EGVAAALTPERLLEWQDKGIFVIQSESLKKCIQAGKSVLTLENCKQANKHMLWKWVS

NHGLFNIGGSGCLGLNFSAPEQPLSLYECDSTLVSLRWRCNRKMITGPLQYSVQVAHD

NTVVASRKYIHKWISYGSGGGDICEYLHKDLHTIKGNTHGMPCMFPFQYNHQWHHE

CTREGREDDLLWCATTSRYERDEKWGFCPDPTSAEVGCDTIWEKDLNSHICYQFNLL

SSLSWSEAHSSCQMQGGTLLSITDETEENFIREHMSSKTVEVWMGLNQLDEHAGWQ

WSDGTPLNYLNWSPEVNFEPFVEDHCGTFSSFMPSAWRSRDCESTLPYICKKYLNHID

HEIVEKDAWKYYATHCEPGWNPYNRNCYKLQKEEKTWHEALRSCQADNSALIDITS

LAEVEFLVTLLGDENASETWIGLSSNKIPVSFEWSNDSSVIFTNWHTLEPHIFPNRSQL

CVSAEQSEGHWKVKNCEERLFYICKKAGHVLSDGGGGSGGGGSEVQLVESGGGLVQ

PGGSLRLSCAASGFNIGGTGIHWVRQAPGKGLEWVAGIYPTNGGTGYAGSVKGRFTIS

ADTSKNTAYLQMNSLRAEDTAVYYCSRGGGDGFGAMGYWGQGTLVTVSSASTKGPS

VFPLAPSSKSTSGGTAALGCLVKDYFPEPVTVSWNSGALTSGVHTFPAVLQSSGLYSLS

SVVTVPSSSLGTQTYICNVNHKPSNTKVDKKVEPKSCDKTHTCPPCPAPELLGGPSVF

LFPPKPKDTLMISRTPEVTCVVVDVSHEDPEVKFNWYVDGVEVHNAKTKPREEQYNS

TYRVVSVLTVLHQDWLNGKEYKCKVSNKALPAPIEKTISKAKGQPREPQVYTLPPSR

DELTKNQVSLTCLVKGFYPSDIAVEWESNGQPENNYKTTPPVLDSDGSFFLYSKLTVD

KSRWQQGNVFSCSVMHEALHNHYTQKSLSLSPGK (SEQ ID NO: 63).

The nucleotide sequence of RC4H:

GAGGGTGTGGCGGCGGCGCTTACCCCCGAGCGGCTCCTGGAGTGGCAGGATAAAG

GAATATTTGTTATCCAAAGTGAGAGTCTCAAGAAATGCATTCAAGCAGGTAAATCG

GTTCTGACCCTGGAGAACTGCAAGCAAGCAAACAAGCACATGCTGTGGAAATGG

GTTTCAAACCATGGCCTCTTTAACATAGGAGGCAGTGGTTGCCTGGGCCTGAATTT

CTCCGCCCCAGAGCAGCCATTAAGCTTATATGAATGTGACTCCACCCTCGTTTCCTT

ACGGTGGCGCTGTAACAGGAAGATGATCACAGGCCCGCTGCAGTACTCTGTCCAG

GTGGCGCATGACAACACAGTGGTGGCCTCACGGAAGTATATTCATAAGTGGATTTC

TTATGGGTCAGGTGGTGGAGACATTTGTGAATATCTACACAAAGATTTGCATACAAT

CAAAGGGAACACCCACGGGATGCCGTGTATGTTTCCCTTCCAGTATAACCATCAGT

GGCATCATGAATGTACCCGTGAAGGTCGGGAAGATGACTTACTGTGGTGTGCCACG

ACAAGCCGTTATGAAAGAGATGAAAAGTGGGGATTTTGCCCTGATCCCACCTCTGC

AGAAGTAGGTTGTGATACTATTTGGGAGAAGGACCTCAATTCACACATTTGCTACC

AGTTCAACCTGCTTTCATCTCTCTCTTGGAGTGAGGCACATTCTTCATGCCAGATGC

AAGGAGGTACGCTGTTAAGTATTACAGATGAAACTGAAGAAAATTTCATAAGGGA

GCACATGAGCAGTAAAACAGTGGAGGTGTGGATGGGCCTCAATCAGCTGGATGAA

CACGCTGGCTGGCAGTGGTCTGATGGAACGCCGCTCAACTATCTGAATTGGAGCC

CAGAGGTAAATTTTGAGCCATTTGTTGAAGATCACTGTGGAACATTTAGTTCATTTA

TGCCAAGTGCCTGGAGGAGTCGGGATTGTGAGTCCACCTTGCCATATATATGTAAA

AAATATCTAAACCACATTGATCATGAAATAGTTGAAAAAGATGCGTGGAAATATTAT

GCTACCCACTGTGAGCCTGGCTGGAATCCCTACAATCGTAATTGCTACAAACTTCA

GAAAGAAGAAAAGACCTGGCATGAGGCTCTGCGTTCTTGTCAGGCTGATAACAGT

GCATTAATAGACATAACCTCATTAGCAGAGGTGGAGTTTCTTGTAACCCTCCTTGGA

GATGAAAATGCATCAGAAACATGGATTGGTTTGAGCAGCAATAAAATTCCAGTTTC

CTTTGAATGGTCTAATGACTCTTCAGTCATCTTTACTAATTGGCACACACTTGAGCC

CCACATTTTTCCAAATAGAAGCCAGCTGTGTGTCTCAGCAGAGCAGTCTGAGGGA

CACTGGAAAGTCAAAAATTGTGAAGAAAGACTTTTTTACATTTGTAAAAAAGCAG

GCCATGTCCTCTCTGATGGCGGTGGAGGCTCCGGCGGAGGTGGAAGCGAAGTCCA

GCTGGTGGAAAGTGGCGGTGGACTGGTTCAACCAGGAGGAAGCCTAAGACTAAG

CTGCGCCGCCAGCGGTTTTAACATCGGTGGCACAGGCATCCATTGGGTCCGGCAG

GCACCCGGCAAAGGTTTAGAGTGGGTCGCAGGCATCTACCCCACTAATGGTGGCA

CCGGCTACGCTGGTAGCGTGAAGGGCAGGTTCACCATTAGCGCTGACACCAGCAA

GAACACTGCTTATCTGCAGATGAATAGCCTGCGTGCCGAAGACACAGCCGTCTATT

ACTGTAGTAGAGGCGGAGGTGATGGGTTCGGCGCGATGGGCTACTGGGGACAGGG

CACACTCGTGACCGTTAGTAGCGCTAGCACCAAGGGCCCATCGGTCTTCCCCCTGG

CACCCTCCTCCAAGAGCACCTCTGGGGGCACAGCGGCCCTGGGCTGCCTGGTCAA

GGACTACTTCCCCGAACCGGTGACGGTGTCGTGGAACTCAGGCGCCCTGACCAGC

GGCGTGCACACCTTCCCGGCTGTCCTACAGTCCTCAGGACTCTACTCCCTCAGCAG

CGTGGTGACCGTGCCCTCCAGCAGCTTGGGCACCCAGACCTACATCTGCAACGTG

AATCACAAGCCCAGCAACACCAAGGTGGACAAGAAAGTAGAGCCCAAATCTTGT

GACAAAACTCACACATGCCCACCGTGCCCAGCACCTGAACTCCTGGGGGGACCGT

CAGTCTTCCTCTTCCCCCCAAAACCCAAGGACACCCTCATGATCTCCCGGACCCCT

GAGGTCACATGCGTGGTGGTGGACGTGAGCCACGAAGACCCTGAGGTCAAGTTC

AACTGGTACGTGGACGGCGTGGAGGTGCATAATGCCAAGACAAAGCCGCGGGAG

GAGCAGTACAACAGCACGTACCGTGTGGTCAGCGTCCTCACCGTCCTGCACCAGG

ACTGGCTGAATGGCAAGGAGTACAAGTGCAAGGTCTCCAACAAAGCCCTCCCAGC

CCCCATCGAGAAAACCATCTCCAAAGCCAAAGGGCAGCCCCGAGAACCACAGGT

GTACACCCTGCCCCCATCCCGGGATGAGCTGACCAAGAACCAGGTCAGCCTGACC

TGCCTGGTCAAAGGCTTCTATCCCAGCGACATCGCCGTGGAGTGGGAGAGCAATG

GGCAGCCGGAGAACAACTACAAGACCACGCCTCCCGTGCTGGACTCCGACGGCT

CCTTCTTCCTCTACAGCAAGCTCACCGTGGACAAGAGCAGGTGGCAGCAGGGGAA

CGTCTTCTCATGCTCCGTGATGCATGAGGCTCTGCACAACCACTACACGCAGAAGA

GCCTCTCCCTGTCTCCGGGTAAA (SEQ ID NO: 64).

The amino acid sequence of RC5L:

EGVAAALTPERLLEWQDKGIFVIQSESLKKCIQAGKSVLTLENCKQANKHMLWKWVS
NHGLFNIGGSGCLGLNFSAPEQPLSLYECDSTLVSLRWRCNRKMITGPLQYSVQVAHD
NTVVASRKYIHKWISYGSGGGDICEYLHKDLHTIKGNTHGMPCMFPFQYNHQWHHE
CTREGREDDLLWCATTSRYERDEKWGFCPDPTSAEVGCDTIWEKDLNSHICYQFNLL
SSLSWSEAHSSCQMQGGTLLSITDETEENFIREHMSSKTVEVWMGLNQLDEHAGWQ
WSDGTPLNYLNWSPEVNFEPFVEDHCGTFSSFMPSAWRSRDCESTLPYICKKYLNHID
HEIVEKDAWKYYATHCEPGWNPYNRNCYKLQKEEKTWHEALRSCQADNSALIDITS
LAEVEFLVTLLGDENASETWIGLSSNKIPVSFEWSNDSSVIFTNWHTLEPHIFPNRSQL
CVSAEQSEGHWKVKNCEERLFYICKKAGHVLSDGGGGSGGGGSDIQMTQSPSSLSAS
VGDRVTITCRASQGVGTAVAWYQQKPGKAPKLLIYSASGLYSGVPSRFSGSRSGTDFT
LTISSLQPEDFATYYCQQHGTTPPTFGQGTKVEIKRTVAAPSVFIFPPSDEQLKSGTASV
VCLLNNFYPREAKVQWKVDNALQSGNSQESVTEQDSKDSTYSLSSTLTLSKADYEK
HKVYACEVTHQGLSSPVTKSFNRGEC (SEQ ID NO: 65).

The nucleotide sequence of RC5L:

GAGGGTGTGGCGGCGGCGCTTACCCCCGAGCGGCTCCTGGAGTGGCAGGATAAAG

GAATATTTGTTATCCAAAGTGAGAGTCTCAAGAAATGCATTCAAGCAGGTAAATCG

GTTCTGACCCTGGAGAACTGCAAGCAAGCAAACAAGCACATGCTGTGGAAATGG

GTTTCAAACCATGGCCTCTTTAACATAGGAGGCAGTGGTTGCCTGGGCCTGAATTT

CTCCGCCCCAGAGCAGCCATTAAGCTTATATGAATGTGACTCCACCCTCGTTTCCTT

ACGGTGGCGCTGTAACAGGAAGATGATCACAGGCCCGCTGCAGTACTCTGTCCAG

GTGGCGCATGACAACACAGTGGTGGCCTCACGGAAGTATATTCATAAGTGGATTTC

TTATGGGTCAGGTGGTGGAGACATTTGTGAATATCTACACAAAGATTTGCATACAAT

CAAAGGGAACACCCACGGGATGCCGTGTATGTTTCCCTTCCAGTATAACCATCAGT

GGCATCATGAATGTACCCGTGAAGGTCGGGAAGATGACTTACTGTGGTGTGCCACG

ACAAGCCGTTATGAAAGAGATGAAAAGTGGGGATTTTGCCCTGATCCCACCTCTGC

AGAAGTAGGTTGTGATACTATTTGGGAGAAGGACCTCAATTCACACATTTGCTACC

AGTTCAACCTGCTTTCATCTCTCTCTTGGAGTGAGGCACATTCTTCATGCCAGATGC

AAGGAGGTACGCTGTTAAGTATTACAGATGAAACTGAAGAAAATTTCATAAGGGA

GCACATGAGCAGTAAAACAGTGGAGGTGTGGATGGGCCTCAATCAGCTGGATGAA

CACGCTGGCTGGCAGTGGTCTGATGGAACGCCGCTCAACTATCTGAATTGGAGCC

CAGAGGTAAATTTTGAGCCATTTGTTGAAGATCACTGTGGAACATTTAGTTCATTTA

TGCCAAGTGCCTGGAGGAGTCGGGATTGTGAGTCCACCTTGCCATATATATGTAAA

AAATATCTAAACCACATTGATCATGAAATAGTTGAAAAAGATGCGTGGAAATATTAT

GCTACCCACTGTGAGCCTGGCTGGAATCCCTACAATCGTAATTGCTACAAACTTCA

GAAAGAAGAAAGACCTGGCATGAGGCTCTGCGTTCTTGTCAGGCTGATAACAGT

GCATTAATAGACATAACCTCATTAGCAGAGGTGGAGTTTCTTGTAACCCTCCTTGGA

GATGAAAATGCATCAGAAACATGGATTGGTTTGAGCAGCAATAAAATTCCAGTTTC

CTTTGAATGGTCTAATGACTCTTCAGTCATCTTTACTAATTGGCACACACTTGAGCC

CCACATTTTTCCAAATAGAAGCCAGCTGTGTGTCTCAGCAGAGCAGTCTGAGGGA

CACTGGAAAGTCAAAAATTGTGAAGAAAGACTTTTTTACATTTGTAAAAAAGCAG

GCCATGTCCTCTCTGATGGCGGTGGAGGCTCCGGCGGAGGTGGAAGCGATATACA

GATGACGCAGAGCCCTAGCAGTCTAAGTGCAAGCGTTGGAGACCGTGTCACCATT

ACCTGCCGCGCTAGCCAGGGCGTCGGCACCGCAGTCGCCTGGTATCAGCAAAAAC

CGGGTAAGGCTCCAAAGCTGTTGATCTACAGTGCCAGCGGCCTGTACAGCGGTGT

GCCAAGTAGGTTCAGCGGGAGCCGGAGTGGGACAGACTTTACACTCACTATTAGC

AGTCTGCAGCCCGAGGACTTCGCCACCTACTACTGTCAGCAGCATGGCACAACAC

CCCCAACATTCGGGCAGGGAACCAAAGTTGAGATAAAACGAACGGTTGCCGCGCC

AAGCGTATTTATCTTTCCCCCCAGTGATGAGCAACTGAAGAGTGGTACAGCCAGCG

TTGTCTGCCTTTTGAATAACTTCTATCCTCGGGAGGCAAAGGTCCAGTGGAAAGTG

GATAATGCACTCCAAAGCGGTAACAGCCAGGAGAGTGTGACAGAACAGGACAGC

AAAGATAGTACCTACAGCCTCAGTAGTACCCTCACACTGAGCAAAGCGGACTATG

AAAAGCACAAGGTGTACGCTTGTGAAGTGACCCACCAGGGCCTAAGCAGCCCTGT

TACTAAGAGCTTCAACAGAGGCGAATGT (SEQ ID NO: 66).

The amino acid sequence of RC6:

EGVAAALTPERLLEWQDKGIFVIQSESLKKCIQAGKSVLTLENCKQANKHMLWKWVS

NHGLFNIGGSGCLGLNFSAPEQPLSLYECDSTLVSLRWRCNRKMITGPLQYSVQVAHD

NTVVASRKYIHKWISYGSGGGDICEYLHKDLHTIKGNTHGMPCMFPFQYNHQWHHE

CTREGREDDLLWCATTSRYERDEKWGFCPDPTSAEVGCDTIWEKDLNSHICYQFNLL

SSLSWSEAHSSCQMQGGTLLSITDETEENFIREHMSSKTVEVWMGLNQLDEHAGWQ

WSDGTPLNYLNWSPEVNFEPFVEDHCGTFSSFMPSAWRSRDCESTLPYICKKYLNHID

HEIVEKDAWKYYATHCEPGWNPYNRNCYKLQKEEKTWHEALRSCQADNSALIDITS

LAEVEFLVTLLGDENASETWIGLSSNKIPVSFEWSNDSSVIFTNWHTLEPHIFPNRSQL

CVSAEQSEGHWKVKNCEERLFYICKKAGHVLSDGGGGSGGGGSTHTCPPCPAPELLG

GPSVFLFPPKPKDTLMISRTPEVTCVVVDVSHEDPEVKFNWYVDGVEVHNAKTKPRE

EQYNSTYRVVSVLTVLHQDWLNGKEYKCKVSNKALPAPIEKTISKAKGQPREPQVYT

LPPSRDELTKNQVSLTCLVKGFYPSDIAVEWESNGQPENNYKTTPPVLDSDGSFFLYSK

LTVDKSRWQQGNVFSCSVMHEALHNHYTQKSLSLSPGK (SEQ ID NO: 67).

The nucleotide sequence of RC6:

GAGGGTGTGGCGGCGGCGCTTACCCCCGAGCGGCTCCTGGAGTGGCAGGATAAAG

GAATATTTGTTATCCAAAGTGAGAGTCTCAAGAAATGCATTCAAGCAGGTAAATCG

GTTCTGACCCTGGAGAACTGCAAGCAAGCAAACAAGCACATGCTGTGGAAATGG

GTTTCAAACCATGGCCTCTTTAACATAGGAGGCAGTGGTTGCCTGGGCCTGAATTT

CTCCGCCCCAGAGCAGCCATTAAGCTTATATGAATGTGACTCCACCCTCGTTTCCTT

ACGGTGGCGCTGTAACAGGAAGATGATCACAGGCCCGCTGCAGTACTCTGTCCAG

GTGGCGCATGACAACACAGTGGTGGCCTCACGGAAGTATATTCATAAGTGGATTTC

TTATGGGTCAGGTGGTGGAGACATTTGTGAATATCTACACAAAGATTTGCATACAAT

CAAAGGGAACACCCACGGGATGCCGTGTATGTTTCCCTTCCAGTATAACCATCAGT

GGCATCATGAATGTACCCGTGAAGGTCGGGAAGATGACTTACTGTGGTGTGCCACG

ACAAGCCGTTATGAAAGAGATGAAAAGTGGGGATTTTGCCCTGATCCCACCTCTGC

AGAAGTAGGTTGTGATACTATTTGGGAGAAGGACCTCAATTCACACATTTGCTACC

AGTTCAACCTGCTTTCATCTCTCTCTTGGAGTGAGGCACATTCTTCATGCCAGATGC

AAGGAGGTACGCTGTTAAGTATTACAGATGAAACTGAAGAAAATTTCATAAGGGA

GCACATGAGCAGTAAAACAGTGGAGGTGTGGATGGGCCTCAATCAGCTGGATGAA

CACGCTGGCTGGCAGTGGTCTGATGGAACGCCGCTCAACTATCTGAATTGGAGCC

CAGAGGTAAATTTTGAGCCATTTGTTGAAGATCACTGTGGAACATTTAGTTCATTTA

TGCCAAGTGCCTGGAGGAGTCGGGATTGTGAGTCCACCTTGCCATATATATGTAAA

AAATATCTAAACCACATTGATCATGAAATAGTTGAAAAGATGCGTGGAAATATTAT

GCTACCCACTGTGAGCCTGGCTGGAATCCCTACAATCGTAATTGCTACAAACTTCA

GAAAGAAGAAAGACCTGGCATGAGGCTCTGCGTTCTTGTCAGGCTGATAACAGT

GCATTAATAGACATAACCTCATTAGCAGAGGTGGAGTTTCTTGTAACCCTCCTTGGA

GATGAAAATGCATCAGAAACATGGATTGGTTTGAGCAGCAATAAAATTCCAGTTTC

CTTTGAATGGTCTAATGACTCTTCAGTCATCTTTACTAATTGGCACACACTTGAGCC

CCACATTTTTCCAAATAGAAGCCAGCTGTGTGTCTCAGCAGAGCAGTCTGAGGGA

CACTGGAAAGTCAAAAATTGTGAAGAAAGACTTTTTTACATTTGTAAAAAAGCAG

GCCATGTCCTCTCTGATGGCGGTGGAGGCTCCGGCGGAGGTGGAAGCACTCACAC

ATGCCCACCGTGCCCAGCACCTGAACTCCTGGGGGGACCGTCAGTCTTCCTCTTCC

CCCCAAAACCCAAGGACACCCTCATGATCTCCCGGACCCCTGAGGTCACATGCGT

GGTGGTGGACGTGAGCCACGAAGACCCTGAGGTCAAGTTCAACTGGTACGTGGA CGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTACACCCTGCCCC CATCCCGGGATGAGCTGACCAAGAACCAGGTCAGCCTGACCTGCCTGGTCAAAGG CTTCTATCCCAGCGACATCGCCGTGGAGTGGGAGAGCAATGGGCAGCCGGAGAAC AACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCTC CGGGTAAA (SEQ ID NO: 68).

[Table 14] Primers and templates of pcDNA3.4 vector and target genes

| Name | Sequence | Nucleotide sequence | Template |
|---|---|---|---|
| Vector-f | CCTCTAGAGTCCGGAGGCTG | SEQ ID NO: 43 | Synthetic gene pcDNA3.4 vector |
| Vector-r | CCCTACCGGTTAGTAATGAGTTTGAT | SEQ ID NO: 44 | |
| RCH-f1 | GCCTCCGGACTCTAGAGGGCCACCATGCTGCTGTCG | SEQ ID NO: 69 | Synthetic gene RC1H, RC4H |
| RCH-f2 | TCCGGCGGAGGTGGAAGCGAAGTCCAGCTGGTGGA AAGTGG | SEQ ID NO: 70 | |
| RCH-r1 | CCACTTTCCACCAGCTGGACTTCGCTTCCACCTCCG CCGGA | SEQ ID NO: 71 | |
| RCH-r2 | CATTACTAACCGGTAGGGTCATTTACCCGGAGACAG GGAGAG | SEQ ID NO: 72 | |
| RCL-f1 | GCCTCCGGACTCTAGAGGGCCACCATGCTGCTGTCG | SEQ ID NO: 73 | Synthetic gene RC2L, RC5L |
| RCL-f2 | TCCGGCGGAGGTGGAAGCGATATACAGATGACGCAG AGCCCTAG | SEQ ID NO: 74 | |
| RCL-r1 | CTAGGGCTCTGCGTCATCTGTATATCGCTTCCACCTC CGCCGGA | SEQ ID NO: 75 | |
| RCL-r2 | CATTACTAACCGGTAGGGTCAACATTCGCCTCTGTTG AAGCTC | SEQ ID NO: 76 | |
| RC3, 6-f | GCCTCCGGACTCTAGAGGGTACGGGCCAGATATACG CGT | SEQ ID NO: 77 | Synthetic gene RC3, RC6 |
| RC3, 6-r | CATTACTAACCGGTAGGGTCATTTAGCTCTGTGTGGT AAGCAGGT | SEQ ID NO: 78 | |

[Table 15] Information of PCR target fragment

| DNA | Primer | Template | Theoretical length |
|---|---|---|---|
| pcDNA3.4 vector | Vector-f, Vector-r | Synthetic gene pcDNA3.4 vector | 6000bp |
| RC1H-1 | RCH-f1, RCH-r1 | Synthetic gene RC1H | 1200bp |
| RC1H-2 | RCH-f2, RCH-r2 | | 1400bp |
| RC2L-1 | RCL-f1, RCL-r1 | Synthetic gene RC2L | 1200bp |
| RC2L-2 | RCL-f2, RCL-r2 | | 700bp |
| RC3 | RC3,6-f, RC3,6-r | Synthetic gene RC3 | 5300bp |
| RC4H-1 | RCH-f1, RCH-r1 | Synthetic gene RC4H | 1600bp |
| RC4H-2 | RCH-f2, RCH-r2 | | 1400bp |
| RC5L-1 | RCL-f1, RCL-r1 | Synthetic gene RC5L | 1600bp |
| RC5L-2 | RCL-f2, RCL-r2 | | 700bp |
| RC6 | RC3,6-f, RC3,6-r | Synthetic gene RC6 | 5700bp |

[Table 16] Plasmid Table

| Plasmid name | Target fragment | Volume of target fragment | Volume of deionized water |
|---|---|---|---|
| pcDNA3.4-RC1H | RC1H-1, RC1H-2 | 0.5 $\mu$L for each | 6 $\mu$L |
| pcDNA3.4-RC2L | RC2L-1, RC2L-2 | 0.5 $\mu$L for each | 6 $\mu$L |
| pcDNA3.4-RC3 | RC3 | 0.5 $\mu$L | 6.5 $\mu$L |
| pcDNA3.4-RC4H | RC4H-1, RC4H-2 | 0.5 $\mu$L for each | 6 $\mu$L |
| pcDNA3.4-RCSL | RC5L-1, RCSL-2 | 0.5 $\mu$L for each | 6 $\mu$L |
| pcDNA3.4-RC6 | RC6 | 0.5 $\mu$L | 6.5 $\mu$L |

2. Transient expression of RC1 to RC8 proteins

2.1. About 24 hours before transfection, FreeStyle™ CHO-S® cells (cell density was 5 to $6\times10^5$/mL, from FreeStyle™ MAX CHO expression system of Gibco Company, USA, catalog number: K900020) were taken and cultured at 37°C and 5% $CO_2$ at 120 to 135 rpm/min.

2.2. On the day of transfection, the cells were diluted to a cell density of $1\times10^6$/mL, and 10 mL of the cells were added to each shake flask (the cell survival rate was above 95%).

2.3. FreeStyle™ MAX Reagent was gently and carefully several times, without causing swirl.

2.4. 12.5 $\mu$g of the plasmids corresponding to each protein in Table 17 were taken, Opti-Pro™ SFM was added to a total volume of 0.2 mL and mixed gently.

2.5. 25 $\mu$L of FreeStyle™ MAX Reagent was taken, Opti-Pro™ SFM was added to a total volume of 0.4 mL, and mixed gently (and carefully without causing swirl). After mixing evenly, the mixture was evenly added to the plasmid mixture and mixed gently to obtain two 0.4 ml DNA-FreeStyle™ MAX mixtures, and incubated at room temperature for 10 minutes to form a complex (12.5 $\mu$L FreeStyle™ MAX Reagent was taken for RC3 and RC6 proteins, added Opti-Pro™ SFM to a total volume of 0.2 mL, and finally 0.4 mL of one DNA-FreeStyle™ MAX was obtained).

2.6. 0.4 mL of the above two DNA-FreeStyle™ MAX mixtures were slowly added into the cells (only one DNA-FreeStyle™ MAX mixture was for RC3 or RC6 protein), while the flask was rotated slowly.

2.7. The cells were cultured at 37°C, 5% $CO_2$ and 120 to 135 rpm/min, without replacing or supplementing the culture medium, and the cell fluid was collected after 7 days.

[Table 17] Plasmids corresponding to RC1 to RC8 proteins

| Protein name | Plasmid name | |
|---|---|---|
| RC1 | pcDNA3.4-RC1H | pcDNA3.4-dTrsL |
| RC2 | pcDNA3.4-dTrsH | pcDNA3.4-RC2L |

(continued)

| Protein name | Plasmid name | |
|---|---|---|
| RC3 | pcDNA3.4-RC3 | / |
| RC4 | pcDNA3.4-RC4H | pcDNA3.4-dTrsL |
| RC5 | pcDNA3.4-dTrsH | pcDNA3.4-RCSL |
| RC6 | pcDNA3.4-RC6 | / |
| RC7 | pcDNA3.4-RC1H | pcDNA3.4-RC2L |
| RC8 | pcDNA3.4-RC4H | pcDNA3.4-RCSL |

3. Purification and identification of RC1 to RC8 proteins.

The culture supernatants of RC1 to RC8 were centrifuged at 4°C and 7,000 g for 15 min, and then filtered with a 0.22 μm syringe filter for sterilization. 5 column volumes of Buffer A1 was used to balance the Protein A chromatographic medium (flow rate was 2 mL/min), the treated sample was added, rinsed with Buffer A1 until the absorption value reached the baseline, and eluted with Buffer B1. The elution peak sample was collected, and the target protein eluent was adjusted to neutral with 1M Tris-HCl (pH 9.0), and filtered into with Super dex200.

Identification of the target proteins: 10-well of SDS-PAGE precast gel was selected and run for 50 min at 150V. The sample was heated at 90°C for 5 min, and 3 μg was loaded into each well.

The purification effects of RC1 to RC8 proteins detected by SDS-PAGE was shown in FIG. 13. In FIG. 13, lane M was the marker, lane 1 was RC1 elution without reduction, lane 2 was RC1 elution with reduction, lane 3 was RC2 elution with reduction, lane 4 was RC2 elution without reduction, lane 5 was RC3 elution without reduction, lane 6 was RC3 elution with reduction, lane 7 was RC4 elution with reduction, lane 8 was RC4 elution without reduction, lane 9 was RC5 elution without reduction, lane 10 was RC5 elution with reduction, lane 11 was RC6 elution without reduction, lane 12 was RC6 elution with reduction, lane 13 was RC7 elution without reduction, lane 14 was RC7 elution with reduction, lane 15 was RC8 elution without reduction, and lane 16 was RC8 elution with reduction. The expression levels of RC1 to RC8 proteins are shown in Table 18.

[Table 18] Expression levels of RC1 to RC8 proteins

| Sample | Protein concentration in culture medium (mg/mL) |
|---|---|
| RC1 | 0.363 |
| RC2 | 0.333 |
| RC3 | 0.0234 |
| RC4 | 0.19 |
| RC5 | 0.243 |
| RC6 | 0.087 |
| RC7 | 0.123 |
| RC8 | 0.103 |

4. Stability investigation of RC1 to RC8

Protein aggregation is a common phenomenon for many globular proteins, and it may affect the determination of quality standards of protein drugs as well as formulation composition. As candidate molecules for drug, molecules with low aggregation are usually preferred. Therefore, the stability of RC1 to RC8 prepared in step 3 of the present example was investigated, and the specific steps were as follows.

RC2 exhibited obvious degradation when stored at -20°C, indicating poor stability, and thus no subsequent experiments were conducted.

RC1, RC3, RC4, RC5, RC6, RC7 and RC8, and CC1h (the amino acid sequence as set forth in SEQ ID NO: 92, and the nucleotide sequence as set forth in SEQ ID NO: 93, prepared by conventional methods in the art) were diluted to the same concentration using sample buffer (25 mM sodium phosphate +75 mM NaCl+40% glycerol (pH 7.0)).

The molecular weights of RC1, RC3, RC4, RC5, RC6, RC7, RC8 and CC1h are shown in the following table.

| Protein | CC1h | RC1 | RC2 | RC3 | RC4 | RC5 | RC6 | RC7 | RC8 |
|---|---|---|---|---|---|---|---|---|---|
| Molecular weight (KD) | 41 | 223 | 223 | 130 | 257 | 257 | 164 | 304 | 371 |

The results reveal that CC1h was aggregated (about 82 KD) after being placed at 37°C for 24 hours, and the aggregation increased after 48 hours. RC3 and RC6 exhibited almost no aggregation. RC1 and RC4 exhibited a few bands with double molecular weight, which were stronger at 48h than at 24h, but compared with CC1h, the aggregation degree of RC1 and RC4 was lower. Therefore, it is further demonstrated that RC3, RC6, RC1 and RC4 have a milder aggregation tendency than CC1h, and they may have better drugability.

The amino acid sequence and nucleotide sequence of CC1h are as follows:

EGVAAALTPERLLEWQDKGIFVIQSESLKKCIQAGKSVLTLENCKQANKHMLWKWVS

NHGLFNIGGSGCLGLNFSAPEQPLSLYECDSTLVSLRWRCNRKMITGPLQYSVQVAHD

NTVVASRKYIHKWISYGSGGGDICEYLHKDLHTIKGNTHGMPCMFPFQYNHQWHHE

CTREGREDDLLWCATTSRYERDEKWGFCPDPTSAEVGCDTIWEKDLNSHICYQFNLL

SSLSWSEAHSSCQMQGGTLLSITDETEENFIREHMSSKTVEVWMGLNQLDEHAGWQ

WSDGTPLNYLNWSPEVNFEPFVEDHCGTFSSFMPSAWRSRDCESTLPYICKKYLNHID

HEIVEGGHHHHHH (SEQ ID NO: 92);

and

gagggtgtggcggcggcgcttaccccgagcggctcctggagtggcaggataaaggaatatttgttatccaaagtgagagtctcaag

aaatgcattcaagcaggtaaatcggttctgaccctggagaactgcaagcaagcaaacaagcacatgctgtggaaatgggtttcaaacc

atggcctctttaacataggaggcagtggttgcctgggcctgaatttctccgccccagagcagccattaagcttatatgaatgtgactccac

cctcgtttccttacggtggcgctgtaacaggaagatgatcacaggcccgctgcagtactctgtccaggtggcgcatgacaacacagtg

gtggcctcacggaagtatattcataagtggatttcttatgggtcaggtggtggagacatttgtgaatatctacacaaagatttgcatacaatc

aaagggaacacccacgggatgccgtgtatgtttcccttccagtataaccatcagtggcatcatgaatgtacccgtgaaggtcgggaaga

tgacttactgtggtgtgccacgacaagccgttatgaaagagatgaaaagtggggattttgccctgatcccacctctgcagaagtaggttg

tgatactatttgggagaaggacctcaattcacacatttgctaccagttcaacctgctttcatctctctcttggagtgaggcacattcttcatgc

cagatgcaaggaggtacgctgttaagtattacagatgaaactgaagaaaatttcataagggagcacatgagcagtaaaacagtggagg

tgtggatgggcctcaatcagctggatgaacacgctggctggcagtggtctgatggaacgccgctcaactatctgaattggagcccaga

ggtaaattttgagccatttgttgaagatcactgtggaacatttagttcatttatgccaagtgcctggaggagtcgggattgtgagtccacctt

gccatatatatgtaaaaaatatctaaaccacattgatcatgaaatagttgaaggaggtcatcaccatcaccatcac (SEQ ID NO: 93).

5. Neutralization of PLA2R-Ab in plasma samples by RC1 to RC8

The only FDA-approved test kit for the diagnosis of PLA2R antibody-positive membranous nephropathy (Omon, Germany, quantitative detection of PLA2R-AB concentration in blood samples by ELISA method) in the United States was used for detection, and all steps were carried out according to the instructions. The plasma samples from three patients with PLA2R antibody-positive membranous nephropathy were denoted with pMN-2, pMN-4 and pMN-7, respectively.

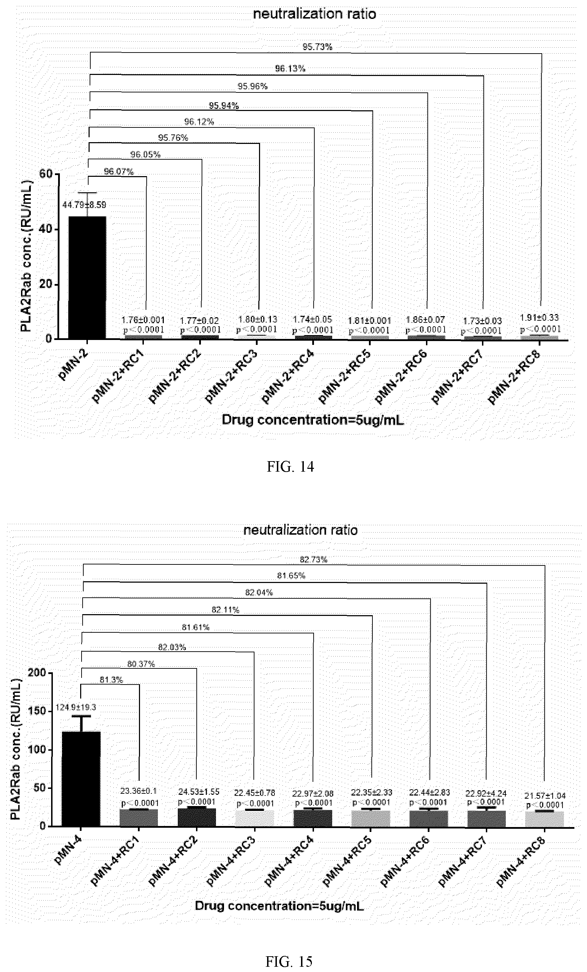

The neutralization effects of PLA2R-Ab in the plasma samples of these three membranous nephropathy-positive patients by RC1 to RC8 were tested. The neutralization rates were obtained, as shown in FIG. 14 to 16 and Table 19. The neutralization ratio in FIG. 14 to FIG. 16 was calculated based on neutralization ratio = (1-(PLA2R-Ab detection value with inhibitor/ PLA2R-Ab detection value without inhibitor))* 100%. The larger the value, the fewer autoimmune antibodies can be detected, and the better the corresponding inhibition effect. (Assuming intravenous administration, the bioavailability is 100%, and the distribution volume of the inhibitor in human body is 8 L, the concentration of the inhibitor in this study is equivalent to a dose of 5 $\mu$g/mL*8L=40 mg/person).

The neutralization rate is calculated as follows.

The capture molecule in the diagnostic kit is PLA2R.

When no drug molecules are added:

$$PLA2R + Ab \overset{K}{\Longleftrightarrow} PLA2R \cdot Ab,$$

$$K = \frac{[PLA2R \cdot Ab]_1}{[PLA2R]_1 \times [Ab]_1}.$$

where $[PLA2R \cdot Ab]_1$ corresponds to the detection concentration of antibody.

After adding drug molecules:

$$K = \frac{[PLA2R \cdot Ab]_2}{[PLA2R]_2 \times [Ab]_2}.$$

where $[PLA2R \cdot Ab]_2$ corresponds to the detection concentration of antibody at this time.

Neutralization ratio (NR):

$$NR = 1 - \frac{[PLA2R \cdot Ab]_2}{[PLA2R \cdot Ab]_1} = 1 - \frac{K \times [PLA2R]_2 \times [Ab]_2}{K \times [PLA2R]_1 \times [Ab]_1}.$$

In the ELISA test, PLA2R as a capture antigen is greatly excessive, and thus $[PLA2R]_2 \approx [PLA2R]_1$. Therefore,

$$NR \approx 1 - \frac{[Ab]_2}{[Ab]_1},$$

that is, the reduction ratio of PLA2R-Ab in the plasma caused by adding drug molecules can represent the neutralization effect of drug molecules.

[Table 19] Neutralization rate of PLA2R-Ab in the plasma of three membranous nephropathy-positive patient by RC1 to RC8.

| Neutralization rate | pMN-2 | pMN-4 | pMN-7 |
|---|---|---|---|
| RC1 | 96.07% | 81.30% | 90.4% |
| RC2 | 96.05% | 80.37% | 90% |
| RC3 | 95.76% | 82.03% | 89.26% |
| RC4 | 96.12% | 81.61% | 87.51% |

(continued)

| Neutralization rate | pMN-2 | pMN-4 | pMN-7 |
|---|---|---|---|
| RC5 | 95.94% | 82.11% | 77.63% |
| RC6 | 95.96% | 82.04% | 81.02% |
| RC7 | 96.13% | 81.65% | 80.22% |
| RC8 | 95.73% | 82.73% | 80.74% |

The dosage of RC1 to RC8 proteins in the three groups of experiments was 5 μg/mL. The pMN-2 was a positive sample, and all the drugs in each group had a neutralization effect of more than 95% for the autoimmune antibody with a concentration of about 44.79 RU/mL in the plasma. The pMN-4 was a positive sample, and all the drugs in each group showed a neutralization effect of more than 80% against the autoimmune antibody with a plasma concentration of about 124.905 RU/mL. The pMN-7 was a positive sample, except for RC5 with a neutralization rate of 77.63%, all the drugs in other groups showed a neutralization effect of more than 80% against the autoimmune antibody with a plasma concentration of about 53.72 RU/mL.

6. Immobilization of RC3 and RC6

A certain volume of NHS-Bestarose (AA0031, Boglon (Shanghai) Biotechnology Co., Ltd.) medium stored in isopropanol was placed in a gravity column, and washed with precooled coupling solution A3 (1 mM hydrochloric acid) (0 to 4°C) for at least 30 min, 1 mL of medium was washed with 20 mL of coupling solution A3 solution. The buffer environment of RC3 and RC6 were replaced with coupling solution B3 (0.1 M sodium bicarbonate, 0.5 M sodium chloride, pH 8.3) (ligand coupling concentration 10 mg/mL filler) by ultrafiltration. The washed medium was diluted with coupling solution A3 (about 0.5 mL of coupling solution A3 for 1 mL of medium), mixed with an equal volume of ligand solution, and shaken at 4°C overnight. The coupling supernatant was removed, the blocking solution (0.1 M Tris-HCl, pH8.5) was added, and the blocking treatment was carried out at room temperature for 4 h. The blocking solution and 0.2 M acetic acid were alternately used to wash for 5 times under the condition of pH 3.5, each time with 3 times the volume of the filler. After washing with PBS, the mixture was used or stored in 0.1 M phosphate buffer to obtain RC3-NHS Bestarose and RC6-NHS Bestarose.

7. Adsorption of PLA2R-Ab in plasma samples by immobilized RC3 and RC6.

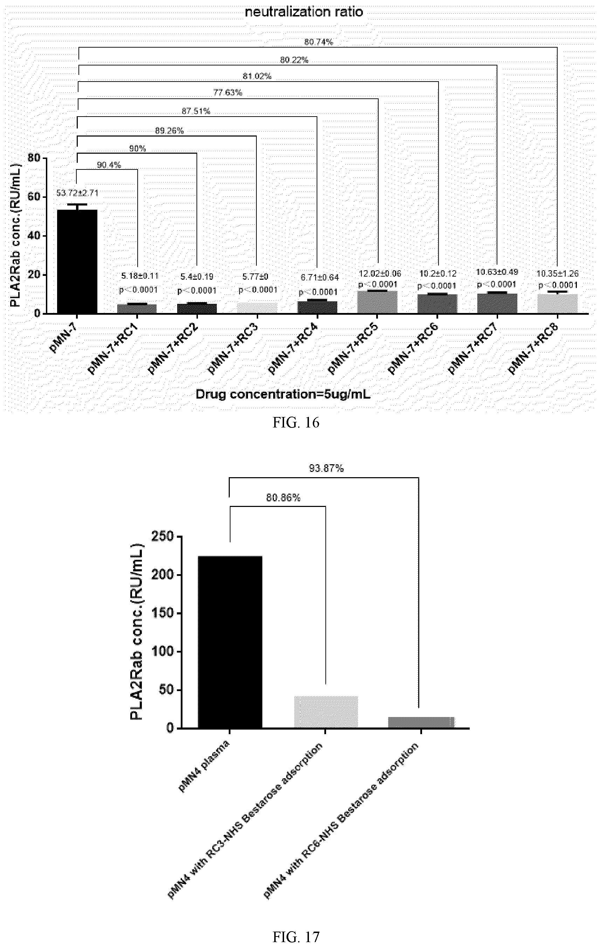

RC3-NHS Bestarose and RC6-NHS Bestarose were fully resuspended, 100 μL of which was added into the corresponding EP tubes, centrifuged at 1,000 g for 5 min, and then the supernatant was sucked off carefully. 500 μL of PBS was added, resuspended, centrifuged and the supernatant was sucked off twice to wash the filler. 100 μL of patient plasma was added into EP tube, resuspended, slightly shaken for 30 min, then centrifuged at 1,000 g for 5 min. The supernatant was pipetted carefully into three EP tubes, which were labeled as RC3-pMN and RC6-pMN, respectively. The adsorption of PLA2R-Ab in PMN-4 and PMN-7 plasma samples by immobilized RC3 and RC6 was detected by ELISA method, which was the same as that in the Section "4. Neutralization of PLA2R-Ab in plasma samples by RC1 to RC8" of the present example. Results were shown in FIG. 17 to FIG. 18. The results indicate that the clearance rates of PLA2R-Ab in PMN-4 and PMN-7 plasma samples by immobilized RC3 and RC6 were both above 80%.

The clearance rates of PLA2R-Ab in PMN-4 plasma samples by immobilized RC3 and RC6 in FIG. 17 were as follows:

$$\text{RC3-NHS Bestarose} = (225.55-43.16)/225.55*100\% = 80.86\%,$$

$$\text{RC6-NHS Bestarose} = (225.55-13.82)/225.55*100\% = 93.87\%.$$

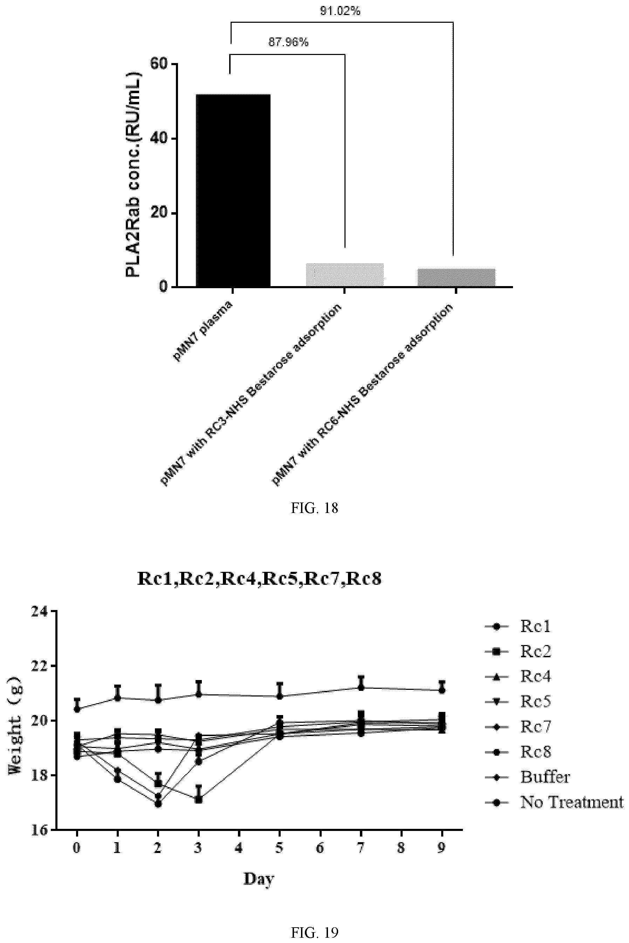

The clearance rates of PLA2R-Ab in PMN-7 plasma samples by immobilized RC3 and RC6 in FIG. 18 were as follows:

$$\text{RC3-NHS Bestarose} = (52.03-6.26)/52.03*100\% = 87.96\%,$$

$$\text{RC6-NHS Bestarose} = (52.03-4.67)/52.03*100\% = 91.02\%.$$

8. Acute toxicity test of RC1 to RC8 mice

The purchased BALB/c female mice were fed for adaption for one week, and randomly divided into eight groups, i.e., RC1 group, RC2 group, RC4 group, RC5 group, RC7 group, RC8 group, placebo group (also called "Buffer") and blank group (also called "No treatment"), with 5 mice in each group, and weighed and marked. The mice were administered with corresponding medicament at 10 times of 40 mg/person. The mice were weighed before administration, and then the medicament was administered. Specifically, the RC1 group was subcutaneously injected with 0.1 mL of RC1 protein with a concentration of 1 mg/mL, the RC2 group was subcutaneously injected with 0.1 mL of RC2 protein with a concentration of 1 mg/mL, the RC4 group was subcutaneously injected with 0.1 mL of RC4 protein with a concentration of 1 mg/mL, the RC5 group was subcutaneously injected with 0.1 mL of RC5 protein with a concentration of 1 mg/mL, the RC7 group was subcutaneously injected with 0.1 mL of RC7 protein with a concentration of 1 mg/mL, the RC8 group was subcutaneously injected with 0.1 mL of RC8 protein with a concentration of 1 mg/mL, the placebo group was injected with 0.1 mL of PBS, and the blank group was not injected. The day of administration was recorded as the 0-th day. After administration, the mice were observed for 1 hour, and no obvious adverse reactions such as dyspnea, decreased exercise and tremor were observed in the mice. The mice were weighed at 24 hours, 48 hours and 72 hours, and then weighed every 48 hours until the tenth day of administration.

FIG. 19 is a graph of weight trend of mice subcutaneously injected with RC1, RC2, RC4, RC5, RC7, and RC8 proteins. The results indicate that there was no significant difference in the weight of mice in the experimental groups and the control group.

The purchased BALB/c female mice were fed for adaption for one week, and randomly divided into four groups: RC3 group, RC6 group, placebo group (also called "Buffer") and blank group (also called "No treatment"), with 5 mice in each group, which were weighed and marked. The mice were administered with corresponding medicament at 10 times of 40 mg/person. The mice were weighed before administration, and then the medicament was administered. Specifically, the RC3 group was subcutaneously injected with 0.1 mL of RC3 protein with a concentration of 1 mg/mL, the RC6 group was subcutaneously injected with 0.1 mL of RC6 protein with a concentration of 1 mg/mL, the placebo group was injected with 0.1 mL of PBS, and the blank group was not injected. The day of administration was recorded as the 0-th day. After administration, the mice were observed for 1 hour, and no obvious adverse reactions such as dyspnea, decreased exercise and tremor were observed in the mice. The mice were weighed at 24 hours, 48 hours and 72 hours, and then weighed every 48 hours until the thirtieth day of administration.

FIG. 20 is a graph of weight trend of mice subcutaneously injected with RC3 and RC6 proteins. The results indicate that there was no significant difference in the weight of mice in the experimental group and the control group.

In the specification, the descriptions referring to the term "one embodiment", "some embodiments", "examples", "specific examples", or "some examples" means that specific features, structures, materials or characteristics described in connection with this embodiment or example are included in at least one embodiment or example of the present disclosure. In this specification, the exemplary expressions of the above terms are not necessarily aimed at the same embodiment or example. Moreover, the specific features, structures, materials or characteristics described may be combined in a suitable manner in any one or more embodiments or examples. In addition, those skilled in the art may combine different embodiments or examples and features of different embodiments or examples described in this specification, unless they contradict each other.

Although the embodiments of the present disclosure have been shown and described above, it can be understood that the above embodiments are illustrative and cannot be understood as limitations of the present disclosure, and those skilled in the art can make changes, modifications, substitutions and variations to the above embodiments within the scope of the present disclosure.

**Claims**

1. An antibody, comprising a light chain variable region CDR and a heavy chain variable region CDR, wherein:

a heavy chain variable region CDR1 sequence is as set forth in $GX_1X_2X_3X_4X_5X_6X_7X_8X_9$;
a heavy chain variable region CDR2 sequence is as set forth in $X_{10}X_{11}X_{12}X_{13}X_{14}X_{15}GX_{16}X_{17}X_{18}X_{19}X_{20}X_{21}X_{22}X_{23}$;
a heavy chain variable region CDR3 sequence is as set forth in $X_{24}GGX_{25}X_{26}X_{27}X_{28}X_{29}X_{30}X_{31}Y$, wherein: $X_1$ is G or F, $X_2$ is G or N, $X_3$ is G or I, $X_4$ is G, K or S, $X_5$ is G or D, $X_6$ is G or T, $X_7$ is G or Y, $X_8$ is G or I, $X_9$ is H or S, $X_{10}$ is R or G, $X_{11}$ is G or I, $X_{12}$ is G or Y, $X_{13}$ is G or P, $X_{14}$ is T or S, $X_{15}$ is G or N, $X_{16}$ is G or Y, $X_{17}$ is G or T, $X_{18}$ is G, S or R, $X_{19}$ is G or Y, $X_{20}$ is G or A, $X_{21}$ is G or D, $X_{22}$ is G or S, $X_{23}$ is S or V, $X_{24}$ is G or W, $X_{25}$ is G or D, $X_{26}$ is G or S, $X_{27}$ is G or F, $X_{28}$ is G or Y, $X_{29}$ is G or A, $X_{30}$ is G or M, and $X_{31}$ is S, G or D, with a provision that the followings do not occur at the same time: $X_1$ is F, $X_2$ is N, $X_3$ is I, $X_4$ is K, $X_5$ is D, $X_6$ is T, $X_7$ is Y, $X_8$ is I, $X_9$ is H, $X_{10}$ is R, $X_{11}$ is I, $X_{12}$ is Y, $X_{13}$ is P,

$X_{14}$ is T, $X_{15}$ is N, $X_{16}$ is Y, $X_{17}$ is T, $X_{18}$ is R, $X_{19}$ is Y, $X_{20}$ is A, $X_{21}$ is D, $X_{22}$ is S, $X_{23}$ is V, $X_{24}$ is W, $X_{25}$ is D, $X_{26}$ is G, $X_{27}$ is F, $X_{28}$ is Y, $X_{29}$ is A, $X_{30}$ is M, and $X_{31}$ is D;

a light chain variable region CDR1 sequence is as set forth in $X_{32}X_{33}X_{34}X_{35}X_{36}X_{37}X_{38}X_{39}X_{40}X_{41}$A;

a light chain variable region CDR2 sequence is as set forth in $X_{42}X_{43}X_{44}X_{45}X_{46}X_{47}$S; and

a light chain variable region CDR3 sequence is as set forth in $X_{48}X_{49}X_{50}X_{51}X_{52}X_{53}X_{54}X_{55}X_{56}$, wherein: $X_{32}$ is G or R, $X_{33}$ is G or A, $X_{34}$ is G or S, $X_{35}$ is G or Q, $X_{36}$ is G, D or S, $X_{37}$ is G or V, $X_{38}$ is G or N, $X_{39}$ is G or T, $X_{40}$ is G or A, $X_{41}$ is S or V, $X_{42}$ is G or S, $X_{43}$ is G or A, $X_{44}$ is G or S, $X_{45}$ is G or F, $X_{46}$ is L or S, $X_{47}$ is G or Y, $X_{48}$ is G or Q, $X_{49}$ is G or Q, $X_{50}$ is G or H, $X_{51}$ is G or Y, $X_{52}$ is S or T, $X_{53}$ is G or T, $X_{54}$ is G or P, $X_{55}$ is G or P, and $X_{56}$ is G or T, with a provision that the followings do not occur at the same time: $X_{32}$ is R, $X_{33}$ is A, $X_{34}$ is S, $X_{35}$ is Q, $X_{36}$ is D, $X_{37}$ is V, $X_{38}$ is N, $X_{39}$ is T, $X_{40}$ is A, $X_{41}$ is V, $X_{42}$ is S, $X_{43}$ is A, $X_{44}$ is S, $X_{45}$ is F, $X_{46}$ is L, $X_{47}$ is Y, $X_{48}$ is Q, $X_{49}$ is Q, $X_{50}$ is H, $X_{51}$ is Y, $X_{52}$ is T, $X_{53}$ is T, $X_{54}$ is P, $X_{55}$ is P, and $X_{56}$ is T.

2. The antibody according to claim 1, wherein the heavy chain variable region CDR and the light chain variable region CDR satisfy at least one of the following conditions:

   $X_4$ is G or S, $X_5$ is G, $X_{21}$ is G, $X_{31}$ is S or G, and $X_{36}$ is G or S.

3. The antibody according to claim 2, wherein:

   in the heavy chain variable region CDR, $X_1$ is G or F, $X_2$ is G or N, $X_3$ is G or I, $X_4$ is G or S, $X_5$ is G, $X_6$ is G or T, $X_7$ is G or Y, $X_8$ is G or I, $X_9$ is H or S, $X_{10}$ is G, $X_{11}$ is G or I, $X_{12}$ is G or Y, $X_{13}$ is G or P, $X_{14}$ is T or S, $X_{15}$ is G or N, $X_{16}$ is G, $X_{17}$ is G or T, $X_{18}$ is G or S, $X_{19}$ is G or Y, $X_{20}$ is G or A, $X_{21}$ is G, $X_{22}$ is G or S, $X_{23}$ is S or V, $X_{24}$ is G, $X_{25}$ is G or D, $X_{26}$ is G or S, $X_{27}$ is G or F, $X_{28}$ is G, $X_{29}$ is G or A, $X_{30}$ is G or M, and $X_{31}$ is S or G; and

   in the light chain variable region CDR, $X_{32}$ is G or R, $X_{33}$ is G or A, $X_{34}$ is G or S, $X_{35}$ is G or Q, $X_{36}$ is G or S, $X_{37}$ is G or V, $X_{38}$ is G, $X_{39}$ is G or T, $X_{40}$ is G or A, $X_{41}$ is S or V, $X_{42}$ is G or S, $X_{43}$ is G or A, $X_{44}$ is G or S, $X_{45}$ is G, $X_{46}$ is L or S, $X_{47}$ is G or Y, $X_{48}$ is G or Q, $X_{49}$ is G or Q, $X_{50}$ is G or H, $X_{51}$ is G, $X_{52}$ is S or T, $X_{53}$ is G or T, $X_{54}$ is G or P, $X_{55}$ is G or P, and $X_{56}$ is G or T.

4. The antibody according to claim 1, comprising a CDR sequence selected from at least one of:

   light chain variable region CDR sequences as set forth in SEQ ID NO: 1, SEQ ID NO: 2, SEQ ID NO: 7, SEQ ID NO: 8, SEQ ID NO: 9, SEQ ID NO: 83, SEQ ID NO: 84, and SEQ ID NO: 85; and

   heavy chain variable region CDR sequences as set forth in SEQ ID NO: 4, SEQ ID NO: 5, SEQ ID NO: 6, SEQ ID NO: 10, SEQ ID NO: 11, SEQ ID NO: 12, SEQ ID NO: 86, SEQ ID NO: 87, SEQ ID NO: 88, SEQ ID NO: 89, SEQ ID NO: 90, and SEQ ID NO: 91.

5. The antibody according to claim 4, wherein:

   the light chain variable region CDR sequences comprise at least one of amino acid sequences having at least 85% similarity to SEQ ID NO: 1 to SEQ ID NO: 3, SEQ ID NO: 7 to SEQ ID NO: 9, and SEQ ID NO: 83 to SEQ ID NO: 85; and

   the heavy chain variable region CDR sequences comprise at least one of amino acid sequences having at least 85% similarity to SEQ ID NO: 4 to SEQ ID NO: 6, SEQ ID NO: 10 to SEQ ID NO: 12, SEQ ID NO: 86 to SEQ ID NO: 88, and SEQ ID NO: 89 to SEQ ID NO: 91.

6. The antibody according to claim 4, comprising:

   the light chain variable region CDR1 sequence, the light chain variable region CDR2 sequence, and the light chain variable region CDR3 sequence as set forth in SEQ ID NO: 1, SEQ ID NO: 2, and SEQ ID NO: 3, respectively, or as set forth in amino acid sequences having at least 85% similarity to SEQ ID NO: 1, SEQ ID NO: 2, and SEQ ID NO: 3, respectively; or

   the light chain variable region CDR1 sequence, the light chain variable region CDR2 sequence, and the light chain variable region CDR3 sequence as set forth in SEQ ID NO: 7, SEQ ID NO: 8, and SEQ ID NO: 9, respectively, or as set forth in amino acid sequences having at least 85% similarity to SEQ ID NO: 7, SEQ ID NO: 8, and SEQ ID NO: 9, respectively; or

   the light chain variable region CDR1 sequence, the light chain variable region CDR2 sequence, and the light chain variable region CDR3 sequence as set forth in SEQ ID NO: 83, SEQ ID NO: 84, and SEQ ID NO: 85, respectively, or as set forth in amino acid sequences having at least 85% similarity to SEQ ID NO: 83, SEQ ID NO: 84, and SEQ ID NO: 85, respectively.

7. The antibody according to claim 4, comprising:

the heavy chain variable region CDR1 sequence, the heavy chain variable region CDR2 sequence, and the heavy chain variable region CDR3 sequence as set forth in SEQ ID NO: 4, SEQ ID NO: 5, and SEQ ID NO: 6, respectively, or as set forth in amino acid sequences having at least 85% similarity to SEQ ID NO: 4, SEQ ID NO: 5, and SEQ ID NO: 6, respectively; or
the heavy chain variable region CDR1 sequence, the heavy chain variable region CDR2 sequence, and the heavy chain variable region CDR3 sequence as set forth in SEQ ID NO: 10, SEQ ID NO: 11, and SEQ ID NO: 12, respectively, or as set forth in amino acid sequences having at least 85% similarity to SEQ ID NO: 10, SEQ ID NO: 11, and SEQ ID NO: 12, respectively; or
the heavy chain variable region CDR1 sequence, the heavy chain variable region CDR2 sequence, and the heavy chain variable region CDR3 sequence as set forth in SEQ ID NO: 86, SEQ ID NO: 87, and SEQ ID NO: 88, respectively, or as set forth in amino acid sequences having at least 85% similarity to SEQ ID NO: 86, SEQ ID NO: 87, and SEQ ID NO: 88, respectively; or
the heavy chain variable region CDR1 sequence, the heavy chain variable region CDR2 sequence, and the heavy chain variable region CDR3 sequence as set forth in SEQ ID NO: 89, SEQ ID NO: 90, and SEQ ID NO: 91, respectively, or as set forth in amino acid sequences having at least 85% similarity to SEQ ID NO: 89, SEQ ID NO: 90, and SEQ ID NO: 91, respectively.

8. The antibody according to claim 4, comprising:

the light chain variable region CDR1 sequence, the light chain variable region CDR2 sequence, and the light chain variable region CDR3 sequence as set forth in SEQ ID NO: 1, SEQ ID NO: 2, and SEQ ID NO: 3, respectively, or as set forth in amino acid sequences having at least 85% similarity to SEQ ID NO: 1, SEQ ID NO: 2, and SEQ ID NO: 3, respectively; and the heavy chain variable region CDR1 sequence, the heavy chain variable region CDR2 sequence, and the heavy chain variable region CDR3 sequence as set forth in SEQ ID NO: 4, SEQ ID NO: 5, and SEQ ID NO: 6, respectively, or as set forth in amino acid sequences having at least 85% similarity to SEQ ID NO: 4, SEQ ID NO: 5, and SEQ ID NO: 6, respectively; or
the light chain variable region CDR1 sequence, the light chain variable region CDR2 sequence, and the light chain variable region CDR3 sequence as set forth in SEQ ID NO: 7, SEQ ID NO: 8, and SEQ ID NO: 9, respectively, or as set forth in amino acid sequences having at least 85% similarity to SEQ ID NO: 7, SEQ ID NO: 8, and SEQ ID NO: 9, respectively; and the heavy chain variable region CDR1 sequence, the heavy chain variable region CDR2 sequence, and the heavy chain variable region CDR3 sequence as set forth in SEQ ID NO: 10, SEQ ID NO: 11, and SEQ ID NO: 12, respectively, or as set forth in amino acid sequences having at least 85% similarity to SEQ ID NO: 10, SEQ ID NO: 11, and SEQ ID NO: 12, respectively; or
the light chain variable region CDR1 sequence, the light chain variable region CDR2 sequence, and the light chain variable region CDR3 sequence as set forth in SEQ ID NO: 83, SEQ ID NO: 84, and SEQ ID NO: 85, respectively, or as set forth in amino acid sequences having at least 85% similarity to SEQ ID NO: 83, SEQ ID NO: 84, and SEQ ID NO: 85, respectively; and the heavy chain variable region CDR1 sequence, the heavy chain variable region CDR2 sequence, and the heavy chain variable region CDR3 sequence as set forth in SEQ ID NO: 86, SEQ ID NO: 87, and SEQ ID NO: 88, respectively, or as set forth in amino acid sequences having at least 85% similarity to SEQ ID NO: 86, SEQ ID NO: 87, and SEQ ID NO: 88, respectively; or
the light chain variable region CDR1 sequence, the light chain variable region CDR2 sequence, and the light chain variable region CDR3 sequence as set forth in SEQ ID NO: 83, SEQ ID NO: 84, and SEQ ID NO: 85, respectively, or as set forth in amino acid sequences having at least 85% similarity to SEQ ID NO: 83, SEQ ID NO: 84, and SEQ ID NO: 85, respectively; and the heavy chain variable region CDR1 sequence, the heavy chain variable region CDR2 sequence, and the heavy chain variable region CDR3 sequence as set forth in SEQ ID NO: 89, SEQ ID NO: 90, and SEQ ID NO: 91, respectively, or as set forth in amino acid sequences having at least 85% similarity to SEQ ID NO: 89, SEQ ID NO: 90, and SEQ ID NO: 91, respectively.

9. The antibody according to any one of claims 1 to 8, comprising:
at least one of a heavy chain frame region sequence and a light chain frame region sequence, wherein:
at least a part of the at least one of the heavy chain frame region sequence and the light chain frame region sequence is derived from at least one of a murine antibody, a humanized antibody, a primate-derived antibody, or a mutant thereof, and preferably, a humanized antibody.

10. The antibody according to any one of claims 1 to 8, comprising:

a light chain variable region, an amino acid sequence of the light chain variable region being as set forth in SEQ ID NO: 13, SEQ ID NO: 14, or SEQ ID NO: 95; and/or
a heavy chain variable region, an amino acid sequence of the heavy chain variable region being as set forth in SEQ ID NO: 15, SEQ ID NO: 16, SEQ ID NO: 96, or SEQ ID NO: 97.

11. The antibody according to claim 10, wherein:

the amino acid sequence of the light chain variable region of the antibody is as set forth in SEQ ID NO: 13, and the amino acid sequence of the heavy chain variable region of the antibody is as set forth in SEQ ID NO: 15;
the amino acid sequence of the light chain variable region of the antibody is as set forth in SEQ ID NO: 14, and the amino acid sequence of the heavy chain variable region of the antibody is as set forth in SEQ ID NO: 16;
the amino acid sequence of the light chain variable region of the antibody is as set forth in SEQ ID NO: 95, and the amino acid sequence of the heavy chain variable region of the antibody is as set forth in SEQ ID NO: 96; or
the amino acid sequence of the light chain variable region of the antibody is as set forth in SEQ ID NO: 95, and the amino acid sequence of the heavy chain variable region of the antibody is as set forth in SEQ ID NO: 97.

12. The antibody according to any one of claims 1 to 8, comprising at least one of a heavy chain constant region and a light chain constant region, wherein:
at least a part of the at least one of the heavy chain constant region and the light chain constant region is derived from at least one of a murine antibody, a humanized antibody, a primate-derived antibody, or a mutant thereof.

13. The antibody according to claim 12, wherein the at least a part of the at least one of the heavy chain constant region and the light chain constant region is derived from a humanized antibody.

14. The antibody according to any one of claims 1 to 8, comprising:

a heavy chain, an amino acid sequence of the heavy chain being as set forth in SEQ ID NO: 17, SEQ ID NO: 18, SEQ ID NO: 33, or SEQ ID NO: 35; and
a light chain, an amino acid sequence of the light chain being as set forth in SEQ ID NO: 19, SEQ ID NO: 20, or SEQ ID NO: 34.

15. The antibody according to claim 14, wherein:

the amino acid sequence of the heavy chain of the antibody is as set forth in SEQ ID NO: 17, and the amino acid sequence of the light chain of the antibody is as set forth in SEQ ID NO: 19;
the amino acid sequence of the heavy chain of the antibody is as set forth in SEQ ID NO: 18, and the amino acid sequence of the light chain of the antibody is as set forth in SEQ ID NO: 20;
the amino acid sequence of the heavy chain of the antibody is as set forth in SEQ ID NO: 33, and the amino acid sequence of the light chain of the antibody is as set forth in SEQ ID NO: 34; or
the amino acid sequence of the heavy chain of the antibody is as set forth in SEQ ID NO: 35, and the amino acid sequence of the light chain of the antibody is as set forth in SEQ ID NO: 34.

16. A nucleic acid molecule, encoding the antibody according to any one of claims 1 to 15.

17. The nucleic acid molecule according to claim 16, wherein the nucleic acid molecule is DNA.

18. An expression vector, carrying the nucleic acid molecule according to claim 16 or 17.

19. The expression vector according to claim 18, wherein the expression vector is a eukaryotic expression vector or a prokaryotic expression vector.

20. The expression vector according to claim 19, wherein the expression vector is a lentiviral vector or a plasmid expression vector.

21. A recombinant cell, carrying the nucleic acid molecule according to claim 16 or 17, or expressing the antibody according to any one of claims 1 to 15.

22. The recombinant cell according to claim 21, wherein the recombinant cell is obtained by introducing the expression vector according to any one of claims 18 to 20 into a host cell.

23. The recombinant cell according to claim 21 or 22, wherein the recombinant cell is a eukaryotic cell.

24. The recombinant cell according to claim 23, wherein the recombinant cell is a mammalian cell.

25. Use of the antibody according to any one of claims 1 to 15 in the preparation of a medicament.

26. The use according to claim 25, wherein the medicament comprises a fusion protein or an antibody-drug conjugate (ADC) drug.

27. Use of the antibody according to any one of claims 1 to 15 as a drug carrier or a negative antibody.

28. The use according to claim 27, wherein the drug carrier or the negative antibody has no antigen-binding ability.

29. A fusion protein, comprising:

   a neutral antibody, comprising an antibody FC segment or the antibody according to any one of claims 1 to 15;
   one or more PLA2R antigen epitope peptides, wherein the one or more PLA2R antigen epitope peptides are linked to the neutral antibody.

30. The fusion protein according to claim 29, wherein a C-terminus of the PLA2R antigen epitope peptide is linked to an N-terminus of the neutral antibody.

31. The fusion protein according to claim 30, wherein the PLA2R antigen epitope peptide comprises CysR, FnII, PLA2R binding region 1, and/or PLA2R binding region 2.

32. The fusion protein according to claim 31, wherein:

   a C-terminus of the CysR is linked to an N-terminus of the FnII; and
   a C-terminus of the FnII is linked to an N-terminus of the PLA2R binding region 1.

33. The fusion protein according to claim 32, wherein:

   the C-terminus of CysR is linked to the N-terminus of FnII;
   the C-terminus of the FnII is linked to the N-terminus of PLA2R binding region 1; and
   a C-terminus of the PLA2R binding region 1 is linked to an N-terminus of PLA2R binding region 2.

34. The fusion protein according to claim 30, wherein the PLA2R antigen epitope peptide has an amino acid sequence as set forth in SEQ ID NO: 21 or SEQ ID NO: 22, or an amino acid sequence having at least 95% similarity to SEQ ID NO: 21 or SEQ ID NO: 22.

35. The fusion protein according to any one of claims 29 to 34, wherein:

   the neutral antibody is an antibody FC segment; and
   a C-terminus of the PLA2R antigen epitope peptide is linked to an N-terminus of at least one antibody FC segment.

36. The fusion protein according to claim 35, wherein:

   the neutral antibody is the antibody according to any one of claims 1 to 15; and
   the C-terminus of the PLA2R antigen epitope peptide is linked to the N-terminus of at least one light chain or heavy chain of the antibody.

37. The fusion protein according to any one of claims 29 to 34, wherein:

   the neutral antibody is a double-stranded antibody; and
   an N-terminus of each chain of the antibody FC segment is linked to a C-terminus of one PLA2R antigen epitope peptide.

38. The fusion protein according to claim 37, wherein an N-terminus of each light chain of the antibody according to any one of claims 1 to 15 is linked to a C-terminus of one PLA2R antigen epitope peptide.

39. The fusion protein according to claim 38, wherein an N-terminus of each heavy chain of the antibody according to any one of claims 1 to 15 is linked to a C-terminus of one PLA2R antigen epitope peptide.

40. The fusion protein according to claim 39, wherein each of an N-terminus of each heavy chain and an N-terminus of each light chain of the antibody according to any one of claims 1 to 15 is linked to a C-terminus of one PLA2R antigen epitope peptide.

41. The fusion protein according to any one of claims 29 to 34, further comprising a connecting peptide, wherein the connecting peptide is disposed between the PLA2R epitope peptide and the antibody.

42. The fusion protein according to claim 41, wherein:

   an N-terminus of the connecting peptide is linked to a C-terminus of the PLA2R antigen epitope peptide; and
   a C-terminus of the connecting peptide is linked to an N-terminus of the neutral antibody.

43. The fusion protein according to claim 42, wherein the connecting peptide has an amino acid sequence of $(GGGGS)_n$, where n is an integer greater than or equal to 1.

44. The fusion protein according to claim 43, wherein n is 1, 2, 3, 4, 5, 6, 7, 8, 9, or 10.

45. The fusion protein according to claim 41, wherein the connecting peptide comprises an amino acid sequence as set forth in SEQ ID NO: 23.

46. The fusion protein according to any one of claims 29 to 34, wherein the antibody FC segment has an amino acid sequence as set forth in SEQ ID NO: 24 or an amino acid sequence having at least 90% similarity to SEQ ID NO: 24.

47. The fusion protein according to any one of claims 29 to 34, wherein:

   the fusion protein has a first peptide segment with an amino acid sequence as set forth in SEQ ID NO: 57 and a second peptide segment with an amino acid sequence as set forth in SEQ ID NO: 19; or
   the fusion protein has a first peptide segment with an amino acid sequence as set forth in SEQ ID NO: 17 and a second peptide segment with an amino acid sequence as set forth in SEQ ID NO: 59; or
   an amino acid sequence of the fusion protein is as set forth in SEQ ID NO: 61; or
   the fusion protein has a first peptide segment with an amino acid sequence as set forth in SEQ ID NO: 63 and a second peptide segment with an amino acid sequence as set forth in SEQ ID NO: 19;
   the fusion protein has a first peptide segment with an amino acid sequence as set forth in SEQ ID NO: 17 and a second peptide segment with an amino acid sequence as set forth in SEQ ID NO: 65;
   an amino acid sequence of the fusion protein is as set forth in SEQ ID NO: 67;
   the fusion protein has a first peptide segment with an amino acid sequence as set forth in SEQ ID NO: 57 and a second peptide segment with an amino acid sequence as set forth in SEQ ID NO: 59; or
   the fusion protein has a first peptide segment with an amino acid sequence as set forth in SEQ ID NO: 63 and a second peptide segment with an amino acid sequence as set forth in SEQ ID NO: 65.

48. A nucleic acid molecule, encoding the fusion protein according to any one of claims 29 to 47.

49. The nucleic acid molecule according to claim 48, wherein the nucleic acid molecule is DNA.

50. An expression vector, carrying the nucleic acid molecule according to claim 48 or 49.

51. The expression vector according to claim 50, wherein the expression vector is a eukaryotic expression vector or a prokaryotic expression vector.

52. The expression vector according to claim 51, wherein the expression vector is a lentiviral vector or a plasmid expression vector.

53. A recombinant cell, carrying the nucleic acid molecule according to claim 48 or 49; or expressing the fusion protein according to any one of claims 29 to 47.

54. The recombinant cell according to claim 53, wherein the recombinant cell is obtained by introducing the expression vector according to any one of claims 50 to 52 into a host cell.

55. The recombinant cell according to claim 53 or 54, wherein the recombinant cell is a eukaryotic cell.

56. The recombinant cell according to claim 55, wherein the recombinant cell is a mammalian cell.

57. A pharmaceutical composition, comprising the fusion protein according to any one of claims 29 to 47.

58. The pharmaceutical composition according to claim 57, further comprising a pharmaceutically acceptable excipient.

59. An immunosorbent, comprising the fusion protein according to any one of claims 29 to 47.

60. A kit, comprising:

the fusion protein according to any one of claims 29 to 47; or
the immunosorbent according to claim 59.

61. An adsorption product, comprising:

the immunosorbent according to claim 59; and
a solid phase carrier, the immunosorbent being linked to the solid phase carrier.

62. The adsorption product according to claim 61, being selected from an immunosorbent column or a blood purifier.

63. Use of the immunosorbent according to claim 59 in the preparation of an adsorption product for adsorbing a PLA2R antibody.

64. The use according to claim 63, wherein the adsorption product is selected from an immunosorbent column or a blood purifier.

65. The use according to claim 64, wherein the adsorption product is an immunosorbent column, and wherein the adsorption product is used to prevent or treat a PLA2R antibody-related disease.

66. The use according to claim 65, wherein the PLA2R antibody-related disease is selected from PLA2R antibody-positive membranous nephropathy.

67. The use according to claim 64, wherein the adsorption product is a blood purifier, and wherein the adsorption product is used to adsorb the PLA2R antibody in blood or plasma.

68. Use of the fusion protein according to any one of claims 29 to 47, the nucleic acid molecule according to any one of claims 48 to 49, the expression vector according to any one of claims 50 to 52, the recombinant cell according to any one of claims 53 to 56, the pharmaceutical composition according to any one of claims 57 and 58, or the immunosorbent according to claim 59 in the preparation of a medicament for preventing or treating a PLA2R antibody-related disease.

69. The use according to claim 68, wherein the PLA2R antibody-related disease is selected from PLA2R antibody-positive membranous nephropathy.

70. Use of the fusion protein according to any one of claims 29 to 47, the nucleic acid molecule according to claim 48 or 49, the expression vector according to any one of claims 50 to 52, the recombinant cell according to any one of claims 53 to 56, the pharmaceutical composition according to any one of claims 57 and 58 in the preparation of an immunosorbent for adsorbing a PLA2R antibody.

71. Use of the fusion protein according to any one of claims 29 to 47, the nucleic acid molecule according to claim 48 or 49, the expression vector according to any one of claims 50 to 52, the recombinant cell according to any one of claims 53 to 56, the pharmaceutical composition according to any one of claims 57 and 58, or the immunosorbent according to claim 59 in preparation of a kit for detecting an PLA2R antibody.

72. A method for preventing or treating a PLA2R antibody-related disease, the method comprising:
administering a pharmaceutically acceptable amount of the fusion protein according to any one of claims 29 to 47 or the pharmaceutical composition according to any one of claims 57 and 58 to a subject.

73. The method according to claim 72, wherein an administration route is subcutaneous injection or intravenous injection.

74. The method according to claim 73, wherein the PLA2R antibody-related disease is selected from PLA2R antibody-positive membranous nephropathy.

75. An immunoadsorption therapy method for preventing or treating a PLA2R antibody-related disease, the method comprising:
treating a subject with the immunosorbent according to claim 59.

76. The method according to claim 75, wherein the subject is treated with extracorporeal blood purification.

77. The method according to claim 75, wherein the PLA2R antibody-related disease is selected from PLA2R antibody-positive membranous nephropathy.

78. A method for diagnosing a PLA2R antibody-related disease, the method comprising:

detecting the PLA2R antibody in a sample to be tested by using the fusion protein according to any one of claims 29 to 47, the nucleic acid molecule according to claim 48 or 49, the expression vector according to any one of claims 50 to 52, the recombinant cell according to any one of claims 53 to 56, the pharmaceutical composition according to any one of claims 57 and 58, the immunosorbent according to claim 59, or the kit according to claim 60; and
determining a content of PLA2R antibody in the sample to be tested based on a detection result.

79. The method according to claim 78, wherein the content of PLA2R antibody in the sample to be tested being not less than 14 RU/mL is an indication that the sample to be tested is derived from a patient suffering from the PLA2R antibody-related disease.

80. The method according to claim 79, wherein the content of PLA2R antibody in the sample to be tested being not less than 20 RU/mL is the indication that the sample to be tested is derived from a patient suffering from the PLA2R antibody-related disease.

81. The method according to any one of claims 78 to 80, wherein the sample to be tested is blood.

82. The method according to any one of claims 78 to 80, wherein the PLA2R antibody-related disease is selected from PLA2R antibody-positive membranous nephropathy.

83. A method for evaluating a stage of a PLA2R antibody-related disease, the method comprising:

detecting the PLA2R antibody in a sample to be tested by using the fusion protein according to any one of claims 29 to 47, the nucleic acid molecule according to claim 48 or 49, the expression vector according to any one of claims 50 to 52, the recombinant cell according to any one of claims 53 to 56, the pharmaceutical composition according to any one of claims 57 and 58, the immunosorbent according to claim 59, or the kit according to claim 60, wherein the sample to be tested is derived from a patient to be tested; and
determining a content of PLA2R antibody in the sample to be tested based on a detection result.

84. The method according to claim 83, wherein the stage of the PLA2R antibody-related disease in the patient to be tested is determined based on the content of PLA2R antibody in the sample to be tested.

85. The method according to claim 83 or 84, wherein the PLA2R antibody-related disease is selected from PLA2R antibody-positive membranous nephropathy.

86. The method according to claim 83 or 84, wherein the sample to be tested is blood.

87. A method for evaluating a prognosis of a PLA2R antibody-related disease, comprising:

detecting the PLA2R antibody in a sample to be tested by using the fusion protein according to any one of claims 29 to 47, the nucleic acid molecule according to claim 48 or 49, the expression vector according to any one of claims 50 to 52, the recombinant cell according to any one of claims 53 to 56, the pharmaceutical composition according to any one of claims 57 and 58, the immunosorbent according to claim 59, or the kit according to claim 60; and

determining a content of PLA2R antibody in the sample to be tested based on a detection result.

88. The method according to claim 87, wherein the sample to be tested is derived from a patient suffering from the PLA2R antibody-related disease before or after treatment.

89. The method according to claim 87, wherein a prognostic effect on the PLA2R antibody-related disease is determined based on the content of PLA2R antibody in the sample to be tested of the patient suffering from the PLA2R antibody-related disease before or after treatment.

90. The method according to claim 87, wherein a decrease in the content of PLA2R antibody in a sample to be tested of a patient suffering from the PLA2R antibody-related disease after treatment is an indication of a good prognosis for the patient.

91. The method according to any one of claims 87 to 90, wherein the sample to be tested is blood.

92. The method according to any one of claims 87 to 90, wherein the PLA2R antibody-related disease is selected from PLA2R antibody-positive membranous nephropathy.

FIG. 1

FIG. 2

FIG. 3

FIG. 4

FIG. 5

FIG. 6

FIG. 7

FIG. 8

FIG. 9

FIG. 10

FIG. 11

77

FIG. 12

FIG. 13

FIG. 14

FIG. 15

FIG. 16

FIG. 17

FIG. 18

FIG. 19

FIG. 20

FIG. 21

FIG. 22

FIG. 23

1 : CC1h   -17 ℃
2 : CC1h   37℃ 24h
3 : CC1h   37℃ 48h

1 : Rc1   -17 ℃
2 : Rc1   37℃ 24h
3 : Rc1   37℃ 48h

1 : Rc3   -17 ℃
2 : Rc3   37℃ 24h
3 : Rc3   37℃ 48h

1 : Rc4   -17 ℃
2 : Rc4   37℃ 24h
3 : Rc4   37℃ 48h

1 : Rc5   -17 ℃
2 : Rc5   37℃ 24h
3 : Rc5   37℃ 48h

1 : Rc6   -17 ℃
2 : Rc6   37℃ 24h
3 : Rc6   37℃ 48h

1 : Rc7   -17 ℃
2 : Rc7   37℃ 24h
3 : Rc7   37℃ 48h

1 : Rc8   -17 ℃
2 : Rc8   37℃ 24h
3 : Rc8   37℃ 48h

FIG. 24

| | INTERNATIONAL SEARCH REPORT | International application No. |
|---|---|---|
| | | **PCT/CN2023/084857** |

**A. CLASSIFICATION OF SUBJECT MATTER**

C07K16/28(2006.01)i; C07K19/00(2006.01)i; C07K14/705(2006.01)i; G01N33/68(2006.01)i; G01N33/564(2006.01)i; A61K39/395(2006.01)i

According to International Patent Classification (IPC) or to both national classification and IPC

**B. FIELDS SEARCHED**

Minimum documentation searched (classification system followed by classification symbols)

IPC: C07K, G01N, A61K

Documentation searched other than minimum documentation to the extent that such documents are included in the fields searched

Electronic data base consulted during the international search (name of data base and, where practicable, search terms used)

VEN, CNABS, PubMed, DWPI, CNTXT, WOTXT, USTXT, EPTXT, JPTXT, CNKI, Web of Science, 百度学术, BAIDU SCHOLAR, Patentics, 中国生物序列检索系统, China Patent Biological Sequence Search System, NCBI Genbank, EBI, STNext: 申请人, 发明人, 序列1-6, 13, 15, CDR, antibody, PLA2R, epitope peptide, fusion protein, vector, carrier, nucleic acid, 表位肽, 融合蛋白, 载体, 核酸, 抗体

**C. DOCUMENTS CONSIDERED TO BE RELEVANT**

| Category* | Citation of document, with indication, where appropriate, of the relevant passages | Relevant to claim No. |
|---|---|---|
| A | CN 102030827 A (SHANGHAI ANTIBODIES NATIONAL ENGINEERING RESEARCHCENTER CO., LTD.) 27 April 2011 (2011-04-27) entire document | 1-92 |
| A | CN 106432508 A (SHENZHEN BLOT BIOTECH CO., LTD.) 22 February 2017 (2017-02-22) entire document | 1-92 |
| A | CN 107663235 A (VAZYME MEDICAL CO., LTD.) 06 February 2018 (2018-02-06) entire document | 1-92 |
| A | CN 113527499 A (XUANZHU BIOLOGICAL TECHNOLOGY CO., LTD. et al.) 22 October 2021 (2021-10-22) entire document | 1-92 |
| A | EP 3373008 A1 (PAD 4 DI EMANUELE GHIGGERI S.A.S.) 12 September 2018 (2018-09-12) entire document | 1-92 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 May 2023** | **08 June 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

| | International application No. |
|---|---|
| | **PCT/CN2023/084857** |

**C.    DOCUMENTS CONSIDERED TO BE RELEVANT**

| Category* | Citation of document, with indication, where appropriate, of the relevant passages | Relevant to claim No. |
|---|---|---|
| A | WO 2020161478 A1 (NOTTINGHAM TRENT UNIVERSITY) 13 August 2020 (2020-08-13) entire document | 1-92 |
| A | 盛茜等 (SHENG, Qian et al.). "PLA2R及其抗体与特发性膜性肾病关系的研究进展 (Non-official translation: Research Progress on the Relationship between PLA2R and its Antibodies and Idiopathic Membranous Nephropathy)" 右江民族医学院学报 (*Journal of Youjiang Medical University for Nationalities*), Vol. 42, No. (3), 30 June 2020 (2020-06-30), pages 372-375 | 1-92 |
| A | JATEM-ESCALANTE, E. et al. "Monitoring anti-PLA2R Antibody Titres to Predict the Likelihood of Spontaneous Remission of Membranous Nephropathy" *Clinical Kidney Journal*, Vol. 14, No. (12), 06 July 2021 (2021-07-06), pages 2556-2562 | 1-92 |

Form PCT/ISA/210 (second sheet) (July 2022)

**INTERNATIONAL SEARCH REPORT**

International application No.

**PCT/CN2023/084857**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

| **INTERNATIONAL SEARCH REPORT** | International application No. |
|---|---|
| | **PCT/CN2023/084857** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **72-77, 83-92**
because they relate to subject matter not required to be searched by this Authority, namely:

Claims 72-77 and 83-92 relate to a method for treating diseases/evaluating staging and prognosis of diseases, which falls within the scope defined in PCT Rule 39.1(iv) for which no search is required. However, a search is still carried out on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

The international application has four inventions: an antibody in the claims comprises several groups of inventions by means of definition methods of different amino acids in a CDR, which relates to four antibody structures (claim 8) recited in the description, the CDR structures thereof being different, that is, the claims relate to at least four groups of inventions. The same technical feature of the inventions is: performing mutation on a CDR of Trastuzumab. However, Trastuzumab is known in the art, and mutation of a CDR is a conventional technique in the art. It can be seen that the inventions do not have a corresponding special technical feature, and therefore do not comply with PCT Rule 13.2. Therefore, the four inventions contained in claims 1-92 do not meet the requirement of unity of invention (PCT Rule 13.1).

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **The technical solutions of claims 1-92 that relate to antibody DTRS4.**

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/CN2023/084857**

| Patent document cited in search report | | | Publication date (day/month/year) | Patent family member(s) | | | Publication date (day/month/year) |
|---|---|---|---|---|---|---|---|
| CN | 102030827 | A | 27 April 2011 | None | | | |
| CN | 106432508 | A | 22 February 2017 | None | | | |
| CN | 107663235 | A | 06 February 2018 | None | | | |
| CN | 113527499 | A | 22 October 2021 | US | 2020140568 | A1 | 07 May 2020 |
| | | | | US | 11325982 | B2 | 10 May 2022 |
| | | | | WO | 2018191188 | A1 | 18 October 2018 |
| | | | | EP | 3609580 | A1 | 19 February 2020 |
| | | | | EP | 3609580 | A4 | 24 March 2021 |
| EP | 3373008 | A1 | 12 September 2018 | IT | 201700025190 | A1 | 07 September 2018 |
| WO | 2020161478 | A1 | 13 August 2020 | US | 2022127374 | A1 | 28 April 2022 |
| | | | | GB | 201901640 | D0 | 27 March 2019 |
| | | | | GB | 2581174 | A | 12 August 2020 |
| | | | | EP | 3921037 | A1 | 15 December 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MC GROGAN A** ; **FRANSSEN CF** ; **DE VRIES CS**. *Nephrol Dial Transplant.*, 2011, vol. 26 (2), 414-430
- **LWEZAULA BF** ; **AMEH OI** ; **EKRIKPO UE et al.** *BMC Nephrol.*, 2021, vol. 22 (1), 15
- **BECK LH JR** ; **BONEGIO RG** ; **LAMBEAU G et al.** *N Engl J Med.*, 2009, vol. 361 (1), 11-21
- **BOBART SA** ; **DE VRIESE AS** ; **PAWAR AS et al.** *Kidney Int.*, 2019, vol. 95 (2), 429-438
- **LIAN MINGJIAN** ; **LU RONG** ; **ZHANG JIAQIN et al.** *Journal of Clinical Laboratory*, 2017, vol. 35 (7), 545-549
- **XU X** ; **NIE S** ; **DING H et al.** *Nat Rev Nephrol.*, 2018, vol. 14 (5), 313-324
- **PASSERINI P** ; **MALVICA S** ; **TRIPODI F et al.** *Front Immunol.*, 2019, vol. 10, 1326
- **ZHENG NA** ; **WANG MEIHUA** ; **AN ZHI**. *Journal of Clinical Military Medicine*, 2021, vol. 49 (08), 942-944
- **LIYO KAO** ; **VINSON LAM**. *J Am Soc Nephrol.*, February 2015, vol. 26 (2), 291-301
- Current Protocols in Molecular Biology. Greene Publishing and Wiley-Interscience, 1995
- **DAYHOFF**. Atlas of Protein Sequence and Structure. National Biomedical Research Foundation, 1978, vol. 5
- **NEEDLEMAN et al.** *J.Mol.Biol.*, 1970, vol. 48, 443
- **SMITH et al.** *Adv. Appl. Math.*, 1981, vol. 2, 482
- **PEARSON et al.** *Proc.Natl.Acad.Sci.*, 1988, vol. 85, 2444
- *Meth.Mol.Biol.*, 1997, vol. 70, 173-187
- **ALTSCHUL et al.** *J.Mol.Biol.*, 1990, vol. 215, 403-410
- **ALTSCHUL et al.** *Meth. Enzym.*, 1996, vol. 266, 460-480
- Handbook of Pharmaceutical Excipients. American Pharmaceutical Association,Pharmaceutical Press